# EUROPEAN PATENT APPLICATION

(11) **EP 3 808 741 A1**
(43) Date of publication of application: **21.04.2021**
(21) Application number: 19203700.0
(22) Date of filing: 16.10.2019
(51) Int. Cl.: C07D 401/14, A61K 31/4545, A61P 35/00

(54) **COMPOUNDS FOR TARGETED DEGRADATION OF CARRIER PROTEINS AND USES THEREOF**

(71) Applicant: CeMM - Forschungszentrum für Molekulare Medizin GmbH, 1090 Wien (AT)
(72) Inventor: BENSIMON, Ariel, 1090 Wien (AT); WINTER, Georg, 1140 Wien (AT); SUPERTI-FURGA, Giulio, 1070 Wien (AT)
(74) Representative: Vossius & Partner Patentanwälte Rechtsanwälte mbB

(57) **Abstract**

The present invention relates to compounds of formula compounds of formulae (I) by which transmembrane proteins such as SLC transporters can be unlocked to chemically-induced degradation, whereby a multitude of diseases can be addressed for the first time. Particularly, the present invention relates to compounds with the ability to induce degradation of SLC transporters via the ubiquitin-proteasome pathway. The invention also relates to the compounds and composition for use as medicaments as well as pharmaceutical compositions comprising these compounds, particularly for use as degraders of SLC transporters which are, e.g, associated with cancer and for use in the treatment of various diseasese such as cancer. Furthermore, the present invention relates to a method for treating a disease or condition, such as cancer, comprising administering the compound of the present invention.

SEQUENCE LISTING

<110> CeMM - Forschungszentrum für Molekulare Medizin GmbH

<120> COMPOUNDS FOR TARGETED DEGRADATION OF CARRIER PROTEINS AND USES THEREOF

<130> AC3083 EP S3

<160> 13

<170> BiSSAP 1.3.6

<210> 1
<211> 2445
<212> DNA
<213> Artificial Sequence

<220>
<223> SLC9A1, also denoted as P19634

<400> 1
atggtgctgc gctctggaat ctgcggactg agcccacacc ggatcttccc ctccctgctg 60

gtggtggtgg ccctggtggg actgctgcct gtgctgagat ctcacggact gcagctgagc 120

ccaaccgcat ccacaatcag gagctccgag ccacctaggg agcggagcat cggcgacgtg 180

accacagcac caccagaggt gacacctgag tccaggccag tgaaccactc tgtgaccgat 240

cacggcatga agcctcggaa ggccttccca gtgctgggca tcgactacac ccacgtgaga 300

acaccctttg agatcagcct gtggatcctg ctggcctgcc tgatgaagat cggcttccac 360

gtgatcccca caatctctag catcgtgcct gagtcctgtc tgctgatcgt ggtgggactg 420

ctggtgggag gactgatcaa gggagtggga gagacccctc cattcctgca gtctgacgtg 480

ttcttcctgt tcctgctgcc ccctatcatc ctggacgccg gctacttcct gccactgagg 540

cagtttaccg agaacctggg cacaatcctg atcttcgccg tggtgggcac actgtggaat 600

gccttctttc tgggcggcct gatgtatgcc gtgtgcctgg tgggaggaga gcagatcaac 660

aatatcggcc tgctggataa cctgctgttt ggcagcatca tctccgccgt ggacccagtg 720

gccgtgctgg ccgtgttcga ggagatccac atcaacgagc tgctgcacat cctggtgttt 780

ggcgagtctc tgctgaatga tgccgtgacc gtggtgctgt accacctgtt cgaggagttt 840

gccaattatg agcacgtggg catcgtggac atcttcctgg gctttctgag cttctttgtg 900

gtggccctgg gaggcgtgct ggtgggagtg gtgtacggcg tgatcgcagc cttcacctct 960

cggttcacca gccacatcag agtgatcgag cccctgttcg tgtttctgta cagctatatg 1020

gcctatctgt ccgccgagct gttccacctg tctggcatca tggcactgat cgcaagcgga 1080

gtggtcatgc ggccttacgt ggaggccaat atctctcaca agagccacac cacaatcaag 1140

tattttctga agatgtggtc ctctgtgagc gagacactga tcttcatctt tctgggcgtg 1200

agcaccgtgg caggatccca ccactggaac tggaccttcg tgatctccac actgctgttt 1260

tgcctgatcg caagggtgct gggcgtgctg ggactgacat ggttcatcaa taagtttcgc 1320

atcgtgaagc tgacccccaa ggatcagttc atcatcgcat acggaggact gagaggagca 1380

atcgcctttt ccctgggcta tctgctggat aagaagcact tccctatgtg cgacctgttt 1440

ctgaccgcca tcatcacagt gatcttcttt acagtgttcg tgcagggcat gaccatcagg 1500

ccactggtgg atctgctggc cgtgaagaag aagcaggaga ccaagcgcag catcaacgag 1560

gagatccaca cacagtttct ggatcacctg ctgaccggca tcgaggacat ctgcggccac 1620

tacggccacc accactggaa ggacaagctg aaccggttca ataagaagta tgtgaagaag 1680

tgtctgatcg ccggcgagag atctaaggag cctcagctga tcgcctttta ccacaagatg 1740

gagatgaagc aggcaatcga gctggtggag agcggaggaa tgggcaagat cccatccgcc 1800

gtgtctaccg tgagcatgca gaatatccac cccaagtccc tgccttctga gaggatcctg 1860

cccgccctgt ccaaggataa ggaggaggag atccgcaaga tcctgcggaa caatctgcag 1920

aagacacggc agagactgag gtcttacaac aggcacaccc tggtggccga cccatatgag 1980

gaggcctgga atcagatgct gctgcggaga cagaaggccc gccagctgga gcagaagatc 2040

aacaattacc tgacagtgcc agcccacaag ctggattccc caaccatgtc tagggcaagg 2100

atcggatccg acccactggc atatgagcct aaggaggatc tgccagtgat caccatcgac 2160

ccagcaagcc cacagtcccc tgagtctgtg gacctggtga acgaggagct gaagggcaag 2220

gtgctgggac tgagcaggga tcctgcaaag gtggcagagg aggacgagga cgatgacgga 2280

ggcatcatga tgagatccaa ggagaccagc tcccccggca cagatgacgt gttcacccca 2340

gccccctccg attctccttc tagccagaga atccagaggt gcctgagcga ccctggacca 2400

cacccagagc ctggagaggg agagccattc tttcccaagg gccag 2445

<210> 2
<211> 2436
<212> DNA
<213> Artificial Sequence

<220>
<223> SLC9A2, also denoted as Q9UBY0

<400> 2
atggagcctc tgggcaactg gcgctccctg agggcaccac tgccacctat gttattactg 60

ctgctgctgc aagtggcagg accagtgggc gccctggccg agacactgct gaatgcacct 120

agggcaatgg gcaccagctc ctctccacca agcccagcat ccgtggtggc accaggaacc 180

acactgttcg aggagagccg gctgcccgtg tttacactgg attaccctca cgtgcagatc 240

ccattcgaga tcaccctgtg gatcctgctg gcctctctgg ccaagatcgg ctttcacctg 300

tatcacaagc tgccaacaat cgtgcccgag agctgcctgc tgatcatggt gggcctgctg 360

ctgggcggca tcatcttcgg cgtggatgag aagtcccctc cagccatgaa gaccgacgtg 420

ttctttctgt acctgctgcc acctatcgtg ctggacgcag gctatttcat gcccacccgc 480

cctttctttg agaacatcgg cacaatcttt tggtacgccg tggtgggcac cctgtggaat 540

tctatcggca tcggcgtgag cctgttcggc atttgtcaaa tagaggcctt tggcctgagc 600

gatatcaccc tgctgcagaa cctgctgttc ggctctctga tcagcgccgt ggaccctgtg 660

gccgtgctgg ccgtgttcga gaacatccac gtgaatgagc agctgtacat cctggtgttt 720

ggcgagagcc tgctgaacga tgccgtgacc gtggtgctgt ataatctgtt caagtccttt 780

tgtcagatga agaccatcga gacaatcgac gtgtttgccg gcatcgccaa cttctttgtg 840

gtgggaatcg gaggcgtgct gatcggaatc ttcctgggct ttatcgccgc cttcaccaca 900

cgctttacac acaatatcag agtgatcgag cccctgttcg tgtttctgta ctcctatctg 960

tcttacatca ccgccgagat gttccacctg tccggcatca tggccatcac cgcctgcgcc 1020

atgacaatga acaagtatgt ggaggagaat gtgagccaga agtcctacac cacaatcaag 1080

tacttcatga agatgctgag ctccgtgtct gagacactga tcttcatctt tatgggcgtg 1140

agcaccgtgg gcaagaacca cgagtggaat tgggccttcg tgtgctttac actggccttc 1200

tgtctgatgt ggagggccct gggcgtgttc gtgctgaccc aggtcatcaa ccgctttcgg 1260

accatcccac tgacattcaa ggaccagttt atcatcgcat acggaggact gaggggagca 1320

atctgtttcg cactggtgtt tctgctgcca gcagccgtgt tccctcggaa gaagctgttt 1380

atcaccgccg ccatcgtggt catcttcttc accgtgttca tcctgggcat cacaatcaga 1440

cccctggtgg agtttctgga tgtgaagagg tctaataaga agcagcaggc cgtgagcgag 1500

gagatctatt gcagactgtt cgatcacgtg aagaccggaa tcgaggacgt gtgcggacac 1560

tggggccaca acttttggag ggacaagttc aagaagtttg acgataagta cctgagaaag 1620

ctgctgatca gggagaatca gcctaagtct agcatcgtgt ccctgtataa gaagctggag 1680

atcaagcacg ccatcgagat ggccgagaca ggcatgatca gcaccgtgcc aacattcgcc 1740

tccctgaacg attgtcgcga ggagaagatc cggaaggtga cctcctctga gacagacgag 1800

atcagagagc tgctgtccag gaacctgtac cagatcagac agaggaccct gagctataat 1860

cgccactccc tgaccgccga tacatctgag cggcaggcca aggagatcct gatccggaga 1920

aggcactctc tgagagagag catcaggaag gacagctccc tgaacagaga gcacagggcc 1980

tccacctcta caagcagata cctgagcctg cctaagaata caaagctgcc agagaagctg 2040

cagaagcgcc ggaccatcag catcgccgat ggcaactcta gcgactccga tgcagacgca 2100

ggaaccacag tgctgaatct gcagccaagg gccagaaggt tcctgcccga gcagttttct 2160

aagaagagcc cccagtccta taagatggag tggaagaatg aggtggatgt ggactccggc 2220

agagacatgc cctctacccc acccacacct cactccaggg agaagggcac ccagacatct 2280

ggcctgctgc agcagcctct gctgtccaag gatcagtccg gctctgagag agaggactct 2340

ctgaccgagg gcatccctcc aaagccacct ccaaggctgg tgtggagggc atccgagcca 2400

ggatctagga aggcaaggtt cggaagcgag aagccc 2436

<210> 3
<211> 2502
<212> DNA
<213> Artificial Sequence

<220>
<223> SLC9A3, also denoted as P48764

<400> 3
atgtggggcc tgggcgccag aggcccagac aggggcctgc tgctggccct ggccctggga 60

ggactggcaa gggcaggagg cgtggaggtg gagcccggcg gcgcccacgg cgagtctggc 120

ggctttcagg tggtgacctt cgagtgggcc cacgtgcagg atccttatgt gatcgccctg 180

tggatcctgg tggcaagcct ggccaagatc ggcttccacc tgtctcacaa ggtgaccagc 240

gtcgtgccag agtccgccct gctgatcgtg ctgggactgg tgctgggagg aatcgtgtgg 300

gccgccgacc acatcgccag ctttaccctg acacccaccg tgttcttttt ctacctgctg 360

ccccctatcg tgctggatgc cggctatttc atgcctaacc ggctgttttt cggcaatctg 420

ggcacaatcc tgctgtatgc agtggtggga accgtgtgga acgcagcaac cacaggactg 480

agcctgtacg gcgtgtttct gtccggactg atgggcgacc tgcagatcgg actgctggat 540

tttctgctgt tcggaagcct gatggcagca gtggaccctg tggccgtgct ggccgtgttc 600

gaggaggtgc acgtgaacga ggtgctgttt atcatcgtgt tcggcgagtc cctgctgaac 660

gatgccgtga cagtggtgct gtacaacgtg ttcgagagtt ttgtcgcact tggtggggac 720

aatgtgaccg gcgtggattg cgtgaagggc atcgtgagct tcttcgtggt gagcctggga 780

ggcacactgg tgggcgtggt gtttgccttc ctgctgtccc tggtgacacg ctttaccaag 840

cacgtgcgga tcatcgagcc cggcttcgtg ttcatcatct cctacctgtc ttatctgaca 900

tctgagatgc tgtctctgag cgccatcctg gccatcacct tctgtggcat ctgctgtcag 960

aagtacgtga aggccaacat cagcgagcag tccgccacca cagtgagata tacaatgaag 1020

atgctggcca gctccgccga gaccatcatc tttatgttcc tgggcatcag cgccgtgaac 1080

cccttcatct ggacatggaa taccgccttt gtgctgctga cactggtgtt catctccgtg 1140

tacagggcaa tcggagtggt gctgcagacc tggctgctga acagatatag gatggtgcag 1200

ctggagccca tcgaccaggt ggtgctgtcc tacggaggac tgaggggagc agtggccttc 1260

gccctggtgg tgctgctgga cggcgataag gtgaaggaga agaatctgtt tgtgagcacc 1320

acaatcatcg tggtgttttt cacagtgatc ttccagggcc tgaccatcaa gccactggtg 1380

cagtggctga aggtgaagag atccgagcac agggagccac ggctgaacga gaagctgcac 1440

ggcagggcct ttgaccacat cctgtccgcc atcgaggata tctctggcca gatcggccac 1500

aattacctga gggacaagtg gtcccacttt gatcgcaagt tcctgtctcg ggtgctgatg 1560

cggagaagcg cccagaagtc cagggaccgg atcctgaacg tgttccacga gctgaatctg 1620

aaggatgcca tcagctatgt ggcagaggga gagaggaggg gatccctggc ctttatcaga 1680

tccccctcta ccgacaacgt ggtgaatgtg gatttcacac ctcgctctag caccgtggag 1740

gccagcgtgt cctacctgct gcgggagaac gtgagcgccg tgtgcctgga catgcagtct 1800

ctggagcagc ggagaaggag catcagggac gccgaggata tggtgacaca ccacaccctg 1860

cagcagtacc tgtataagcc tagacaggag tacaagcacc tgtattccag gcacgagctg 1920

acaccaaccg aggacgagaa gcaggataga gagatctttc acaggacaat gagaaagagg 1980

ctggagtctt tcaagagcac caagctgggc ctgaaccaga ataagaaggc cgccaagctg 2040

tataagaggg agagggcaca gaagaggagg aactcctcta tccctaatgg caagctgcct 2100

atggagtctc cagcccagaa cttcacaatc aaggagaagg acctggagct gagcgatacc 2160

gaggagccac ccaattacga cgaggagatg agcggcggca tcgagtttct ggcctccgtg 2220

acaaaggaca ccgcctctga tagcccagcc ggcatcgata atcccgtgtt ctctcctgac 2280

gaggccctgg atcgcagcct gctggcaagg ctgcctccat ggctgtcccc aggagagacc 2340

gtggtgccat ctcagagagc caggacacag atcccataca gccccggcac cttttgtaga 2400

ctgatgccct tcaggctgag ctccaagtcc gtggactctt tcctgcaggc cgatggacct 2460

gaggagaggc cacctgccgc cctgccagag tccacccaca tg 2502

<210> 4
<211> 2394
<212> DNA
<213> Artificial Sequence

<220>
<223> SLC9A4, also denoted as Q6AI14

<400> 4
atggccctgc agatgttcgt gacctactct ccctggaact gcctgctgct gctggtggcc 60

ctggagtgta gcgaggccag ctccgatctg aacgagtctg ccaatagcac agcccagtat 120

gccagcaatg catggttcgc agcagcatct agcgagccag aggagggcat ctccgtgttt 180

gagctggatt acgactatgt gcagatcccc tacgaggtga ccctgtggat cctgctggcc 240

tctctggcca agatcggctt ccacctgtat cacaggctgc ctggcctgat gccagagagc 300

tgcctgctga tcctggtggg cgccctggtg ggaggcatca tctttggcac cgatcacaag 360

tccccccctg tgatggactc ctctatctac ttcctgtatc tgctgccacc aatcgtgctg 420

gagggaggat actttatgcc cacacgccct ttctttgaga acatcggctc catcctgtgg 480

tgggccgtgc tgggcgccct gatcaatgca ctgggcatcg gcctgtctct gtatctgatc 540

tgtcaggtga aggccttcgg cctgggcgat gtgaacctgc tgcagaatct gctgtttggc 600

tccctgatct ctgccgtgga ccctgtggcc gtgctggccg tgttcgagga ggcacgggtg 660

aacgagcagc tgtacatgat gatctttggc gaggccctgc tgaacgacgg catcaccgtg 720

gtgctgtata atatgctgat cgccttcacc aagatgcaca agtttgagga tatcgagaca 780

gtggacatcc tggcaggatg cgcaaggttc atcgtggtgg gactgggagg cgtgctgttt 840

ggcatcgtgt tcggctttat ctccgccttc atcacccgct ttacacagaa catctctgcc 900

atcgagcccc tgatcgtgtt catgtttagc tacctgtcct atctggcagc agagaccctg 960

tacctgtccg gaatcctggc aatcaccgca tgtgcagtga caatgaagaa gtatgtggag 1020

gagaacgtga gccagacaag ctacaccaca atcaagtatt tcatgaagat gctgagctcc 1080

gtgagcgaga ccctgatctt catctttatg ggcgtgtcca cagtgggcaa gaaccacgag 1140

tggaattggg ccttcatctg ctttaccctg gccttttgtc agatctggcg ggccatcagc 1200

gtgttcgccc tgttttacat cagcaaccag ttcagaacat tccctttttc tatcaaggat 1260

cagtgcatca tcttttatag cggcgtgagg ggagcaggat ctttcagcct ggcctttctg 1320

ctgcctctga gcctgttccc acgcaagaag atgtttgtga ccgccacact ggtggtcatc 1380

tacttcaccg tgttcatcca gggcatcaca gtgggcccac tggtgcggta tctggacgtg 1440

aagaagacca acaagaagga gtccatcaat gaggagctgc acatcagact gatggatcac 1500

ctgaaggcag gaatcgagga cgtgtgcgga cactggagcc actaccaggt gagggataag 1560

ttcaagaagt ttgaccacag gtatctgcgc aagatcctga tccgcaagaa tctgcccaag 1620

tctagcatcg tgtccctgta caagaagctg gagatgaagc aggccatcga gatggtggag 1680

accggcatcc tgtcctctac agccttcagc atcccccacc aggcccagag gatccagggc 1740

atcaagcgcc tgtctcctga ggatgtggag agcatcagag acatcctgac ctctaacatg 1800

taccaggtgc ggcagagaac actgagctac aacaagtata atctgaagcc tcagacctcc 1860

gagaagcagg ccaaggagat cctgatccgg agacagaata cactgaggga gtccatgcgc 1920

aagggacact ctctgccatg gggcaagcct gcaggaacca agaacatccg gtacctgtcc 1980

tacccatatg gcaatcccca gtctgccgga agggatacca gagcagcagg cttcagcgac 2040

gatgacagct ccgacccagg ctccccctct atcacatttt ccgcctgctc tagaatcggc 2100

tccctgcaga agcaggaggc ccaggagatc atcccaatga agtctctgca caggggccgc 2160

aaggccttca gctttggcta ccagcggaac acctcccagg aggagtatct gggaggcgtg 2220

aggagggtgg ccctgagacc taagccactg ttccacgcag tggatgagga gggagagagc 2280

ggaggagagt ccgagggcaa ggcaagcctg gtggaggtgc ggtccagatg gacagcagat 2340

cacggacacg gaagggacca ccacagaagc cactcccccc tgctgcagaa gaag 2394

<210> 5
<211> 2688
<212> DNA
<213> Artificial Sequence

<220>
<223> SLCA5, also denoted as Q14940

<400> 5
atgctgagag ccgccctgtc tctgctggcc ctgccactgg caggagcagc agaggagcct 60

acccagaagc cagagagccc aggagagcca cctccaggac tggagctgtt cagatggcag 120

tggcacgagg tggaggcacc atacctggtg gccctgtgga tcctggtggc aagcctggcc 180

aagatcgtgt ttcacctgtc taggaaggtg accagcctgg tgcctgagtc ctgcctgctg 240

atcctgctgg gactggtgct gggaggaatc gtgctggccg tggccaagaa ggccgagtat 300

cagctggagc caggcacatt ctttctgttc ctgctgcccc ctatcgtgct ggactccggc 360

tactttatgc cctctcgcct gttctttgat aacctgggcg ccatcctgac ctacgcagtg 420

gttgggactt tgtggaatgc cttcaccaca ggagccgccc tgtggggact gcagcaggca 480

ggcctggtgg cccctagggt gcaggcagga ctgctggact tcctgctgtt tggcagcctg 540

atctccgccg tggatccagt ggccgtgctg gccgtgtttg aggaggtgca cgtgaacgag 600

accctgttca tcatcgtgtt tggcgagagc ctgctgaatg acgccgtgac agtggtgctg 660

tacaaggtgt gcaacagctt cgtggagatg ggctccgcca atgtgcaggc caccgattat 720

ctgaagggcg tggcctctct gtttgtggtg agcctgggag gagcagcagt gggactggtg 780

ttcgcctttc tgctggccct gaccacaaga ttcacaaaga gagtgaggat catcgagcct 840

ctgctggtgt ttctgctggc ctacgccgcc tatctgaccg ccgagatggc ctctctgagc 900

gccatcctgg ccgtgacaat gtgcggcctg ggctgtaaga agtacgtgga ggccaacatc 960

tctcacaaga gcaggaccac agtgaagtat accatgaaga cactggcctc ctgtgccgag 1020

accgtgatct tcatgctgct gggcatctcc gccgtggaca gctccaagtg ggcatgggat 1080

tctggcctgg tgctgggcac cctgatcttc atcctgttct ttcgcgccct gggagtggtg 1140

ctgcagacat gggtgctgaa tcagtttcgg ctggtgccac tggacaagat cgatcaggtg 1200

gtcatgagct acggaggact gaggggagca gtggccttcg ccctggtcat cctgctggac 1260

cggaccaagg tgcccgccaa ggattatttt gtggccacca caatcgtggt ggtgttcttt 1320

accgtgatcg tgcagggcct gacaatcaag cccctggtga agtggctgaa ggtgaagcgg 1380

tccgagcacc acaagcctac cctgaaccag gagctgcacg agcacacatt cgaccacatc 1440

ctggcagcag tggaggatgt ggtgggacac cacggctacc actattggcg cgaccggtgg 1500

gagcagtttg ataagaagta cctgtcccag ctgctgatgc ggagatctgc ctatagaatc 1560

agggaccaga tctgggacgt gtactatcgc ctgaatatcc gggacgccat cagcttcgtg 1620

gatcagggag gacacgtgct gtctagcacc ggactgacac tgccttccat gccatctaga 1680

aacagcgtgg ccgagacctc cgtgacaaat ctgctgaggg agtccggatc tggagcatgc 1740

ctggacctgc aggtcatcga taccgtgcgc tctggaaggg acagggagga tgccgtgatg 1800

caccacctgc tgtgcggagg actgtacaag ccaaggcgcc ggtataaggc cagctgttcc 1860

agacacttca tctctgagga cgcccaggag aggcaggata aggaggtgtt tcagcagaac 1920

atgaagagaa ggctggagtc cttcaagtct accaagcaca atatctgttt tacaaagtcc 1980

aagcctagac caaggaagac cggccgccgg aagaaggacg gagtggcaaa cgcagaggca 2040

acaaatggca agcacagggg actgggattc caggacaccg cagccgtgat cctgacagtg 2100

gagagcgagg aggaggagga ggagtccgat tcctctgaga ccgagaagga ggacgatgag 2160

ggcatcatct ttgtggcaag ggccacaagc gaggtgctgc aggagggcaa ggtgtctgga 2220

agcctggagg tgtgcccatc tcctaggatc atcccaccca gcccaacctg tgccgagaag 2280

gagctgccat ggaagagcgg acagggcgac ctggccgtgt acgtgagctc cgagaccaca 2340

aagatcgtgc ctgtggatat gcagacaggc tggaaccagt ccatctctag cctggagagc 2400

ctggcctccc ctccatgcaa tcaggcccct atcctgacct gtctgccccc tcacccaaga 2460

ggcacagagg agccccaggt gcctctgcac ctgccaagcg accccaggtc ctctttcgcc 2520

tttccaccat ccctggcaaa ggcaggccgc tcccggtctg agagctccgc cgacctgcct 2580

cagcagcagg agctgcagcc actgatgggc cacaaggatc acacccacct gtctccaggc 2640

accgccacaa gccactggtg tatccagttc aacagaggct ccaggctg 2688

<210> 6
<211> 2007
<212> DNA
<213> Artificial Sequence

<220>
<223> SLCA6, also denoted as Q92581

<400> 6
atggctcgga gaggctggag gcgcgcccct ctgcggagag gagtgggaag ctcccctagg 60

gcaaggcgcc tgatgagacc actgtggctg ctgctggcag tgggcgtgtt cgattgggca 120

ggagcatctg acggaggagg aggagaggcc cgggccatgg atgaggagat cgtgagcgag 180

aagcaggccg aggagtccca cagacaggac tctgccaatc tgctgatctt catcctgctg 240

ctgacactga ccatcctgac catctggctg tttaagcacc ggagagccag gttcctgcac 300

gagacaggcc tggccatgat ctacggactg ctggtgggac tggtgctgcg ctatggcatc 360

cacgtgccat ccgatgtgaa caatgtgacc ctgtcttgcg aggtgcagtc tagccccacc 420

acactgctgg tgacattcga ccctgaggtg ttctttaata tcctgctgcc ccctatcatc 480

ttttacgccg gctattccct gaagaggcgc cacttctttc ggaacctggg ctctatcctg 540

gcctacgcct ttctgggcac cgccatcagc tgcttcgtga tcggctccat catgtatggc 600

tgcgtgaccc tgatgaaggt gaccggccag ctggccggcg atttctactt taccgactgt 660

ctgctgttcg gcgccatcgt gtccgccaca gatccagtga ccgtgctggc catcttccac 720

gagctgcagg tggacgtgga gctgtatgcc ctgctgtttg gcgagagcgt gctgaatgac 780

gccgtggcca tcgtgctgtc ctctagcatc gtggcatacc agccagcagg cgataactct 840

cacacatttg acgtgaccgc catgttcaag agcatcggca tcttcctggg catcttttct 900

ggcagcttcg ccatgggagc agcaacagga gtggtgaccg ccctggtgac aaagtttacc 960

aagctgaggg agttccagct gctggagaca ggcctgttct ttctgatgtc ctggtctacc 1020

tttctgctgg cagaggcatg gggcttcaca ggagtggtgg ccgtgctgtt ttgcggcatc 1080

acacaggccc actacaccta taacaatctg agcacagagt cccagcaccg caccaagcag 1140

ctgtttgagc tgctgaactt cctggccgag aatttcatct tttcctatat gggcctgaca 1200

ctgttcacct ttcagaacca cgtgtttaat cctaccttcg tggtgggcgc ctttgtggcc 1260

atcttcctgg gcagggccgc caacatctac ccactgagcc tgctgctgaa tctgggccgg 1320

agatctaaga tcggcagcaa ttttcagcac atgatgatgt tcgcaggact gaggggagca 1380

atggccttcg ccctggccat cagggataca gccacctacg cccgccagat gatgttctcc 1440

accacactgc tgatcgtgtt ctttaccgtg tgggtgtttg gaggaggaac cacagcaatg 1500

ctgagctgcc tgcacatccg ggtgggcgtg gattccgacc aggagcacct gggagtgcca 1560

gagaacgaga ggaggaccac aaaggcagag tccgcctggc tgtttagaat gtggtacaac 1620

ttcgaccaca attatctgaa gccactgctg acccactctg gaccaccact gaccacaacc 1680

ctgccagcat gctgtggacc tatcgcaaga tgtctgacca gccctcaggc ctatgagaac 1740

caggagcagc tgaaggacga tgactccgat ctgatcctga atgatggcga catctctctg 1800

acctacggcg acagcacagt gaacaccgag ccagccacat cctctgcccc acggagattc 1860

atgggcaaca gctccgagga tgcactggac agggagctgg cctttggcga tcacgagctg 1920

gtcatcaggg gaaccaggct ggtgctgcca atggatgaca gcgagcctcc actgaacctg 1980

ctggacaata caagacacgg ccccgcc 2007

<210> 7
<211> 1743
<212> DNA

<213> Artificial Sequence

<220>
<223> SLCA8, also denoted as Q9Y2E8

<400> 7
atgggcgaga agatggccga ggaggagcgg ttcccaaaca ccacacacga gggctttaat 60

gtgacactgc acaccacact ggtggtgacc acaaagctgg tgctgccaac acccggcaag 120

cctatcctgc cagtgcagac cggagagcag gcacagcagg aggagcagag ctccggcatg 180

accatcttct ttagcctgct ggtgctggcc atctgcatca tcctggtgca cctgctgatc 240

aggtacaggc tgcacttcct gccagagtcc gtggcagtgg tgtctctggg catcctgatg 300

ggcgccgtga tcaagatcat cgagttcaag aagcttgcca actggaaaga ggaggagatg 360

tttagaccta acatgttctt tctgctgctg ctgcccccta tcatcttcga gagcggctac 420

tccctgcaca agggcaactt ctttcagaat atcggctcca tcacactgtt cgccgtgttt 480

ggcaccgcaa tctctgcctt cgtggtggga ggaggcatct atttctgggg ccaggccgac 540

gtgatctcta agctgaatat gacagatagc ttcgcctttg gctctctgat cagcgccgtg 600

gacccagtgg caaccatcgc catcttcaac gccctgcacg tggatcccgt gctgaatatg 660

ctggtgtttg gcgagtccat cctgaacgac gccgtgtcta tcgtgctgac aaataccgcc 720

gagggcctga caaggaagaa catgtccgac gtgtctggct ggcagacctt cctgcaggcc 780

ctggattact ttctgaagat gttctttgga agcgccgccc tgggcacact gaccggactg 840

atctccgccc tggtgctgaa gcacatcgat ctgcgcaaga cccctagcct ggagttcggc 900

atgatgatca tctttgccta cctgccatat ggactggcag agggaatctc tctgagcgga 960

atcatggcca tcctgttcag cggcatcgtg atgtcccact atacacacca caacctgtcc 1020

ccagtgaccc agatcctgat gcagcagaca ctgaggaccg tggcctttct gtgcgagacc 1080

tgcgtgttcg cctttctggg cctgagcatc ttctcctttc cccacaagtt cgagatcagc 1140

tttgtgatct ggtgtatcgt gctggtgctg ttcggccggg ccgtgaatat ctttcccctg 1200

tcctacctgc tgaacttctt tagagaccac aagatcaccc ctaagatgat gttcatcatg 1260

tggttttctg gactgagggg agcaatccca tatgcactga gcctgcacct ggatctggag 1320

cctatggaga agagacagct gatcggcacc acaaccatcg tgatcgtgct gttcacaatc 1380

ctgctgctgg gcggctctac catgcctctg atcagactga tggacatcga ggatgccaag 1440

gcccaccgga gaaacaagaa ggacgtgaat ctgtccaaga cagagaagat gggcaatacc 1500

gtggagtctg agcacctgag cgagctgacc gaggaggagt acgaggccca ctatatcagg 1560

cgccaggacc tgaagggctt cgtgtggctg gatgccaagt acctgaatcc tttctttaca 1620

cggagactga cccaggagga tctgcaccac ggcaggatcc agatgaagac actgaccaac 1680

aagtggtatg aggaggtgcg ccagggacca tccggatctg aggacgatga gcaggagctg 1740

ctg 1743

<210> 8
<211> 1935
<212> DNA
<213> Artificial Sequence

<220>
<223> SLCA9, also denoted as Q8IVB4

<400> 8
atggagaggc agtctcgcgt gatgagcgag aaggacgagt accagtttca gcaccaggga 60

gcagtggagc tgctggtgtt caactttctg ctgatcctga ccatcctgac aatctggctg 120

ttcaagaatc accggttcag atttctgcac gagaccggcg gcgccatggt gtatggcctg 180

atcatgggac tgatcctgag atacgcaacc gcaccaacag acatcgagag cggaaccgtg 240

tacgattgcg tgaagctgac cttcagccct tccacactgc tggtgaacat cacagaccag 300

gtgtacgagt ataagtacaa gcgcgagatc tcccagcaca acatcaatcc ccaccagggc 360

aacgccatcc tggagaagat gacctttgat cctgagatct tctttaatgt gctgctgccc 420

cctatcatct tccacgccgg ctattccctg aagaagcggc acttctttca gaacctgggc 480

tctatcctga cctacgcctt tctgggcaca gccatcagct gcatcgtgat cggcctgatc 540

atgtatggct tcgtgaaggc catgatccac gccggccagc tgaagaatgg cgacttccac 600

tttaccgatt gtctgttctt tggcagcctg atgtccgcca ccgacccagt gacagtgctg 660

gccatctttc acgagctgca cgtggacccc gatctgtaca ccctgctgtt cggagagtcc 720

gtgctgaacg acgcagtggc catcgtgctg acatattcta tcagcatcta cagcccaaag 780

gagaacccca atgccttcga tgccgccgcc ttctttcagt ccgtgggcaa tttcctgggc 840

atctttgccg gctccttcgc catgggctct gcctatgcca tcatcaccgc cctgctgacc 900

aagtttacaa agctgtgcga gttccccatg ctggagacag gcctgttctt tctgctgtcc 960

tggtctgcct ttctgtccgc cgaggcagca ggactgaccg gaatcgtggc cgtgctgttc 1020

tgtggcgtga cccaggccca ctatacatac aacaatctga gctccgattc taagatcagg 1080

accaagcagc tgttcgagtt tatgaacttt ctggccgaga atgtgatctt ctgctacatg 1140

ggcctggccc tgttcacatt tcagaaccac atctttaatg ccctgttcat cctgggcgcc 1200

tttctggcca tcttcgtggc ccgggcctgt aacatctatc ctctgagctt tctgctgaat 1260

ctgggcagaa agcagaagat cccatggaac tttcagcaca tgatgatgtt ctctggactg 1320

aggggagcaa tcgccttcgc cctggccatc cgcaataccg agagccagcc caagcagatg 1380

atgtttacca caaccctgct gctggtgttc tttaccgtgt gggtgttcgg aggaggaaca 1440

accccaatgc tgacatggct gcagatcagg gtgggagtgg acctggatga gaacctgaag 1500

gaggacccct ctagccagca ccaggaggcc aacaatctgg ataagaatat gacaaaggcc 1560

gagtctgccc ggctgtttag aatgtggtat agcttcgacc acaagtacct gaagcctatc 1620

ctgacccact ctggcccacc cctgacaacc acactgcctg agtggtgtgg cccaatcagc 1680

cggctgctga catccccaca ggcctacggc gagcagctga aggaggacga tgtggagtgc 1740

atcgtgaacc aggatgagct ggccatcaat tatcaggagc aggcctcctc tccttgttcc 1800

cctccagcca gactgggcct ggaccagaag gcctctcccc agacccctgg caaggagaac 1860

atctatgagg gcgatctggg actgggagga tacgagctga agctggagca gacactgggc 1920

cagagccagc tgaat 1935

<210> 9
<211> 1611
<212> DNA
<213> Artificial Sequence

<220>
<223> SLCB2, also denoted as Q86UD5

<400> 9
atgggcgacg aggataagcg gatcacctac gaggactccg agccatctac cggcatgaac 60

tatacaccct ctatgcacca ggaggcccag gaggagaccg tgatgaagct gaagggcatc 120

gatgccaatg agccaacaga gggcagcatc ctgctgaaga gctccgagaa gaagctgcag 180

gagaccccaa cagaggcaaa ccacgtgcag cggctgagac agatgctggc atgcccacct 240

cacggactgc tggacagagt gatcaccaat gtgacaatca tcgtgctgct gtgggccgtg 300

gtgtggtcca tcaccggatc tgagtgcctg ccaggaggaa acctgttcgg catcatcatc 360

ctgttttact gtgccatcat cggcggcaag ctgctgggcc tgatcaagct gcccacactg 420

ccacccctgc cttccctgct gggaatgctg ctggcaggct tcctgatcag gaatatcccc 480

gtgatcaacg acaatgtgca gatcaagcac aagtggtcta gctccctgcg gagcatcgcc 540

ctgtccatca tcctggtgag agccggcctg ggcctggatt ctaaggccct gaagaagctg 600

aagggcgtgt gcgtgcgcct gtctatggga ccttgcatcg tggaggcatg taccagcgcc 660

ctgctggcac actacctgct gggactgcca tggcagtggg gcttcatcct gggctttgtg 720

ctgggagccg tgtctcctgc agtggtggtg ccaagcatgc tgctgctgca gggaggagga 780

tatggagtgg agaagggagt gcctacactg ctgatggcag caggctcctt cgacgatatc 840

ctggccatca ccggctttaa cacatgtctg ggcatcgcct tcagcaccgg ctccacagtg 900

tttaatgtgc tgaggggcgt gctggaggtg gtcatcggag tggcaaccgg atccgtgctg 960

ggcttcttta tccagtactt cccctctagg gaccaggata agctggtgtg caagcgcaca 1020

ttcctggtgc tgggactgag cgtgctggcc gtgttttcta gcgtgcactt cggatttcct 1080

ggatccggag gactgtgcac cctggtcatg gcctttctgg ccggcatggg ctggacatcc 1140

gagaaggccg aggtggagaa gatcatcgcc gtggcctggg atatcttcca gcccctgctg 1200

tttggcctga tcggcgccga ggtgtctatc gcaagcctga ggcctgagac cgtgggactg 1260

tgcgtggcaa cagtgggaat cgccgtgctg atccgcatcc tgaccacatt cctgatggtg 1320

tgcttcgccg gctttaacct gaaggagaag atcttcatca gctttgcctg gctgcccaag 1380

gccaccgtgc aggcagcaat cggatccgtg gccctggaca cagcacggtc tcacggcgag 1440

aagcagctgg aggactatgg catggatgtg ctgaccgtgg cctttctgag catcctgatc 1500

acagccccta tcggatccct gctgatcgga ctgctgggac caagactgct gcagaaggtg 1560

gagcaccaga ataaggatga ggaggtgcag ggcgagacca gcgtgcaggt g 1611

<210> 10
<211> 86
<212> DNA
<213> Artificial Sequence

<220>
<223> SLC38A2_5-PITCH

<400> 10
cgcgttacat agcatcgtac gcgtacgtgt ttggtagctt gaagaaggcc gaaaccatgg 60

ccaagccttt gtctcaagaa gaatcc 86

<210> 11
<211> 95
<212> DNA
<213> Artificial Sequence

<220>
<223> SLC38A2_3-PITCH

<400> 11
catcagcatt ctagagcatc gtacgcgtac gtgtttgggg ggaaatactg aatcgtccca 60

tctctgcttt cttcatagat ccgccgccac ccgac 95

<210> 12
<211> 25
<212> DNA
<213> Artificial Sequence

<220>
<223> SLC38A2_gRNA_S

<400> 12
caccgaatac tgaatcgtcc cattt 25

<210> 13
<211> 25
<212> DNA
<213> Artificial Sequence

<220>
<223> SLC38A2_gRNA_AS

<400> 13
aaacaaatgg gacgattcag tattc                                          25

## Description

### FIELD OF THE INVENTION

The present invention relates to compounds of formula (I) with the ability to ubiquitinate a transmembrane protein/transmembrane proteins, such as SLCs/solute carrier proteins. Particularly, the present invention relates to compounds with the ability to induce degradation of SLCs/solute carrier proteins via the ubiquitin-proteasome pathway. The invention also relates to the compounds and composition for use as medicaments as well as pharmaceutical compositions comprising these compounds, particularly for use as degraders of transmembrane proteins, such as SLCs/solute carrier proteins. Furthermore, the present invention relates to a method for treating a disease or condition, such as cancer, comprising administering the compound of the present invention.

### BACKGROUND OF THE INVENTION

Several families of transmembrane transporters including solute carriers (SLCs), ion channels, water channels and ATP-driven pumps, enable the exchange of nutrients, ions and metabolic products across cellular membranes (Hediger, M. A., Clémençon, B., Burrier, R. E. & Bruford, E. A. The ABCs of membrane transporters in health and disease (SLC series): Introduction. Molecular Aspects of Medicine 34, 95-107 (2013)). The more than 450 members of the Solute carrier group of proteins (SLC) represent the largest class of transmembrane transporters encoded in the human genome (Lin, L. Nature Publishing Group 14, 543-560 (2015)). Their several-pass transmembrane domain structure and hydrophobicity contributed to the orphan status of many SLCs, devoid of both natural cargos and functional inhibitors. 65 families are identified on the basis of sequence similarities. For example, amino acid accumulation is mediated by members of the SLC1, SLC3/7, SLC6, SLC15, SLC16, SLC17, SLC32, SLC36, SLC38 and SLC43. Further members of the SLC superfamily, such as the SLC9 family of sodium/hydrogen exchangers, regulate ion fluxes at the plasma membrane, or solute transport into and out of cellular organelles. In particular, sodium/hydrogen exchangers or sodium/proton antiports are a family of transporters that maintain cellular pH by utilising the sodium gradient across the plasma membrane to extrude protons produced by metabolism, in a stoichiometry of 1 Na⁺ (in): 1 H⁺ (out). For example, NHE1 is (also known as SLC9A1) is an electroneutral and reversible ion transporter that exchanges one Na⁺ ion for one H⁺ ion, contributing to both cytoplasmic alkalinization, and acidification of the microenvironment (Parks et al. Nature Reviews Cancer 13, 611-623 (2013)).

NHE3, which is also known as SLC9A3, is highly expressed in the intestine and kidneys and regulate sodium movements in those tissues. NHE1, also denoted SLC9A1 is considered to be a ubiquitously-expressed transporter. NHE3 is highly expressed in the intestine and kidneys and regulate sodium movements in those tissues.

In recent years, evidence of direct roles for some SLCs in key tumorigenic processes has been accumulating, demonstrating that SLCs may be therapeutically attractive for targeted drug development in cancer (El-gebali, S., Bentz, S., Hediger, M. A. & Anderle, P. Molecular Aspects of Medicine Solute carriers (SLCs) in cancer. Molecular Aspects of Medicine 34, 719-734 (2013); Qing and Shu, Mol Cell Ther. 2, 15 (2014)). Moreover, SLC9A1 was a promising target in cardiovascular disease but clinical development of specific inhibitors was abandoned due to poor response and adverse side effects. Further, SLC9A1 has been investigated in a variety of cancer models, but most prominently in glioma and breast cancer (Amith SR, Fong S, Baksh S, Fliegel L. Na (+)/H (+)exchange in the tumour microenvironment: does NHE1 drive breast cancer carcinogenesis?, Int J Dev Biol. 2015;59(7-9):367-77; Cong D, Zhu W, Kuo JS, Hu S, Sun D, Ion transporters in brain tumors, Curr Med Chem. 2015;22(10):1171-81).

Most of SLC-targeting small molecule development has been oriented towards inhibition (Lin, L., Yee, S. W., Kim, R. B. & Giacomini, K. M. SLC Transporters as Therapeutic Targets: Emerging Opportunities. Nat Rev Drug Discov 14, 543-560 (2015)). However, this approach does not allow degradation of the transmembrane proteins such as SLCs. Thus, novel paradigms in drug design are highly needed.

Several techniques developed in recent years to control the targeted degradation of specific proteins by a new generation of small-molecule degraders or "PROTACs" (proteolysis targeting chimeras) now enables the rational design of small-molecules that induce the selective and immediate degradation of target protein (Winter, G. E. et al. DRUG DEVELOPMENT. Phthalimide conjugation as a strategy for in vivo target protein degradation. Science 348, 1376-1381 (2015), Bondeson, D. P. et al. Catalytic in vivo protein knockdown by small-molecule PROTACs. Nat. Chem. Biol. 11, 611-617 (2015)). PROTACs operate by inducing molecular proximity between the protein of interest (POI) and a cellular E3 ligase substrate receptor by binding simultaneously to both proteins. This induced proximity leads to ubiquitination and proteasomal degradation of the POI. Of note, the modular design consisting of a warhead binding to the POI, a flexible linker, and a defined E3 ligase ligand renders PROTAC development very flexible. The list of proteins permissive to targeted degradation now contains a large number of protein kinases, including one instance of a single-pass transmembrane receptor tyrosine kinase. Some proteins with one (1) transmebrane region, like EGFR, HER2, c-Met, ALK and FLT-3 (Cell Chem Biol. 2018 Jan 18;25(1):67-77. The Advantages of Targeted Protein Degradation Over Inhibition: An RTK Case Study. Burslem GM, Smith BE, Lai AC, Jaime-Figueroa S, McQuaid DC, Bondeson DP, Toure M, Dong H, Qian Y, Wang J, Crew AP, Hines J, Crews CM. / Eur J Med Chem. 2018 May 10;151:304-314. Proteolysis Targeting Chimeras (PROTACs) of Anaplastic Lymphoma Kinase (ALK). Zhang C, Han XR, Yang X, Jiang B, Liu J, Xiong Y, Jin J. J Am Chem Soc. 2018 Dec 5;140(48):16428-16432/ Enhancing Antiproliferative Activity and Selectivity of a FLT-3 Inhibitor by Proteolysis Targeting Chimera Conversion.Burslem GM, Song J, Chen X, Hines J, Crews CM) have been shown to be degradable by "PROTAC" induced degradation. However, many proteins, such as SLCs/solute carrier transporters, may have more than ten (10) transmembrane regions which is challenging for the potential degradation of these proteins by PROTAC. Moreover, several of the main drug targets and disease-associated genes have a multi-pass transmembrane domain topology and are located at different subcellular locations. Prominent examples are G-protein-coupled receptors (GPCRs), ABC-transporters, such as the cystic fibrosis transmembrane conductance regulator (CFTR), and solute carrier transporter proteins (SLCs). Consequently, compounds that are able to effectively target such disease associated transmembrane proteins are not known. Hence, in view of the above, the technical problem underlying the present invention is the provision of chemical compounds which can degrade transmembrane proteins, such as multi-pass transmembrane domain proteins.

The solution to this technical problem is provided by the embodiments as defined herein below and as characterized in the claims. In the context of the present invention novel compounds are provided that alter protein function or protein abundance (for instance via induced targeted protein degradation) by altering the function of or binding to ubiquitin E3 ligases, including ligases of the cullin RING E3 ligase family.

In this context, transmembrane proteins, particularly solute carriers, are increasingly attracting the interest of chemical biologists and drug discoverers as they are involved in a variety of diseases and there are some prominent drug targets. Thus, invention described herein thus represents a breakthrough in terms of chemical strategy, in particular because it has been shown that the degradation machinery required for ligand-induced proteolysis is accessible to transmembrane proteins such as solute carriers.

The invention also relates to the following items:
1. A compound having the following formula (I): 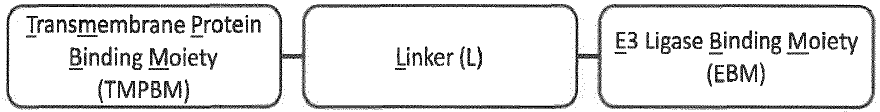 wherein
   - TMPBM: is a moiety binding to a transmembrane protein,
   - L: is a linker moiety; and
   - EBM: is a moiety modifying the function of the E3 ligase and/or binding to at least one member of the E3 ligase complex.
2. The compound according to item 1, wherein TMPBM is a moiety having the following partial formula (II) or a stereoisomer, tautomer, pharmaceutically acceptable salt, solvate or prodrug: 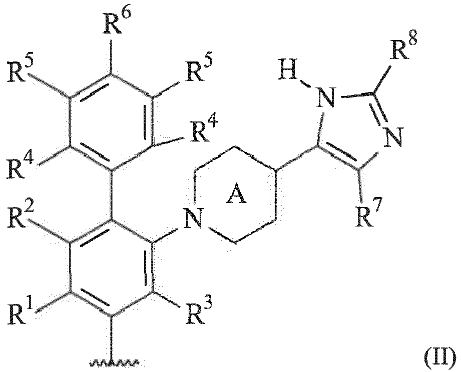 wherein
   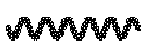 indicates the position of attachment of the partial formula (II) to the linker (L), R¹, R², R³, R⁴, R⁵, R⁶, R⁷ and R⁸ are each independently selected from -H, -halogen, - NO₂, -CN, -C₁₋₃ alkyl, -OH, -O-C₁₋₂ alkyl, NH₂, -NH(C₁₋₂ alkyl), -N(C₁₋₂ alkyl)₂, - CHO, -CO(C₁₋₂ alkyl), COOH, COO(C₁₋₂ alkyl), CONH₂, CONH(C₁₋₂ alkyl), and CON(-C₁₋₂ alkyl)₂, wherein each C₁₋₂ alkyl is optionally substituted with one or more F, and Ring A is optionally substituted with one or more F.
3. The compound according to item 2, wherein in the moiety of partial formula (II) the following definitions apply:
   R¹, R², R³ and each R⁴ are each hydrogen,
   R⁷ is methyl,
   R⁸ is H or NH₂,
   and Ring A is not substituted with any F.
4. The compound according to item 2 or 3, wherein in the moiety of partial formula (II) the following definitions apply:
   one R⁵ is selected from H, F, Cl, Me, CF₃, CF₂H and CH₂F,
   the other R⁵ is H, and
   R⁶ is F or Cl.
5. The compound according to any one of items 2 to 4, wherein the moiety of partial formula (II) has the following formula (IIa): 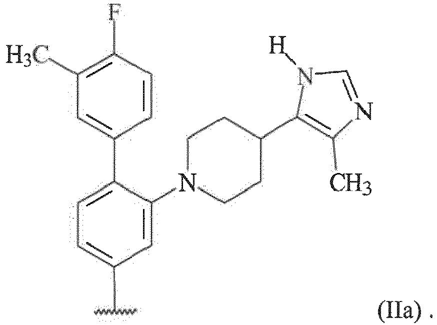
6. The compound according to any one of items 1 to 5, wherein L is selected from C₁₄₋₂₄ alkylene, C₁₄₋₂₄ alkenylene, and C₁₄₋₂₄ alkynylene, wherein said alkylene, said alkenylene and said alkynylene are each optionally substituted with one or more groups independently selected from halogen, C₁₋₅ haloalkyl, -O(C₁₋₅ haloalkyl), -CN, -OR²¹, -NR²¹R²¹, -NR²¹OR²¹, -COR²¹, -COOR²¹, -OCOR²¹, -CONR²¹R²¹, -NR²¹COR²¹, -NR²¹COOR²¹, -OCONR²¹R²¹, -SR²¹, -SOR²¹, -SO₂R²¹, -SO₂NR²¹R²¹, -NR²¹SO₂R²¹, -SO₃R²¹, and -NO₂, and further wherein one or more -CH₂- units comprised in said alkylene, said alkenylene or said alkynylene are each optionally replaced by a group independently selected from -O-, -NR²¹-, -CO-, -S-, -SO-, and -SO₂-;
   each R²¹ is independently selected from hydrogen, C₁₋₅ alkyl, C₂₋₅ alkenyl, C₂₋₅ alkynyl, carbocyclyl, and heterocyclyl, wherein said alkyl, said alkenyl and said alkynyl are each optionally substituted with one or more groups R^{Alk}, and further wherein said carbocyclyl and said heterocyclyl are each optionally substituted with one or more groups R^{Cyc};
   any two R²¹ are optionally linked to form a ring;
   each R^{Alk} is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl);
   each R^{Cyc} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl).
7. The compound according to item 6, wherein the C₁₄₋₂₄ alkylene, C₁₄₋₂₄ alkenylene, and C₁₄₋₂₄ alkynylene are C₁₅₋₂₂ alkylene, C₁₅₋₂₂ alkenylene, and C₁₅₋₂₂ alkynylene, respectively, preferably C₁₆₋₂₀ alkylene, C₁₆₋₂₀ alkenylene, and C₁₆₋₂₀ alkynylene respectively wherein each of the alkylenes, alkenylenes and alkynylenes may be modified as set out in item 6.
8. The compound according to item 6, wherein L is selected from C₁₄₋₂₄ alkylene and C₁₄₋₂₄ alkenylene, more preferably C₁₅₋₂₂ alkylene, C₁₅₋₂₂ alkenylene, even more preferably C₁₆₋₂₀ alkylene, still more preferably C₁₇₋₁₉ alkylene, most preferably C₁₇ alkylene, wherein each of the alkylenes and alkenylenes may be modified as set out in item 6.
9. The compound according to item 6, wherein L has one of the following structures:

   -O-L¹-NH-C(=O)- and -NH-L¹-NH-C(=O)-

   wherein L¹ is selected from C₁₃₋₁₇ alkylene and C₁₃₋₁₇ alkenylene, wherein said alkylene and said alkenylene are each optionally substituted with one or more groups independently selected from halogen, C₁₋₃ alkyl and C₁₋₃ haloalkyl, and further wherein one or more -CH₂- units comprised in said alkylene or said alkenylene is/are replaced by a group independently selected from -O-, -NR²¹-, -CO-, -S-, -SO-, and -SO₂-.
10. The compound according to item 6, wherein L has one of the following structures:

   -O-L¹-NH-C(=O)- and -NH-L¹-NH-C(=O)-

   -wherein L¹ is selected from C₁₃₋₁₇ alkylene, wherein said alkylene is optionally substituted with one or more groups independently selected from halogen, C₁₋₃ haloalkyl and C₁₋₃ haloalkyl, and further wherein one or more -CH₂- units comprised in said alkylene or said alkenylene is/are replaced by a group independently selected from -O-, -NR²¹ and -CO-.
11. The compound according to item 9 or 10, wherein the -O- or -NH- on the left hand side binds to EBM as defined in any of the preceding items and the NH-C(=O)- on the right hand side is bound to the TMPBM as defined in any of the preceding items.
12. The compound of formula (I) according to any one of the preceding items, wherein EBM is a moiety binding to at least one member of the E3 ligase complex with a Kd of at least 10 µM or less.
13. The compound of formula (I) according to any one of the preceding items, wherein EBM is a moiety of partial formula (III): 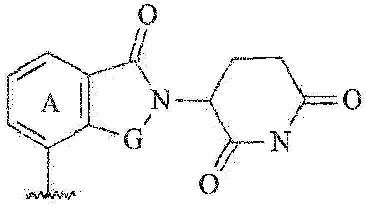 wherein
   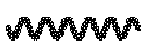 indicates the position of attachment of the partial formula (III) to the linker (L),
   G is selected from -C(=O)- and CH₂,
   and Ring A is optionally substituted with one or more F,
   or any stereoisomer thereof.
14. The compound according to item 13, wherein in partial formula (III), G is -C(=O)- and Ring a is not substituted with any F.
15. The compound of any of items 1 to 14, wherein the TMPBM is selected from a moiety binding to membrane transport proteins, which are preferably carriers and/or channels, more preferably carriers.
16. The compound of any of items 1 to 15, wherein the TMPBM is a moiety binding to a solute carrier (SLC) protein,
   preferably to a SLC selected from a SLC family consisting of SLC1 (high affinity glutamate and neutral amino acid transporter), SLC2 (facilitative GLUT transporter), SLC3 (heavy subunits of the heteromeric amino acid transporters), SLC4 (bicarbonate transporter), SLC5 (sodium glucose cotransporter), SLC6 (sodium- and chloride-dependent neurotransmitter transporter), SLC7 (cationic amino acid transporter/glycoprotein-associated amino-acid transporter), SLC8 (Na+/Ca2+ exchanger), SLC9 (Na+/ H+ exchanger), SLC10 (sodium bile salt cotransport), SLC11 (proton coupled metal ion transporter), SLC12 (electroneutral cation-Cl cotransporter), SLC13 (human Na+-sulfate/carboxylate cotransporter), SLC14 (urea transporter), SLC15 (proton oligopeptide cotransporter), SLC16 (monocarboxylate transporter), SLC17 (vesicular glutamate transporter), SLC18 (vesicular amine transporter), SLC19 (folate/thiamine transporter), SLC20 (type III Na+-phosphate cotransporter), SLC21/SLCO (organic anion transporting), SLC22 (organic cation/anion/zwitterion transporter), SLC23 (Na+-dependent ascorbic acid transporter), SLC24 (Na+/(Ca2+-K+) exchanger), SLC25 (mitochondrial carrier), SLC26 (multifunctional anion exchanger), SLC27 (fatty acid transport protein), SLC28 (Na+-coupled nucleoside transport), SLC29 (facilitative nucleoside transporter), SLC30 (zinc efflux), SLC31 (copper transporter), SLC32 (vesicular inhibitory amino acid transporter), SLC33 (Acetyl-CoA transporter), SLC34 (type II Na+-phosphate cotransporter), SLC35 (nucleoside-sugar transporter), SLC36 (proton-coupled amino acid transporter), SLC37 (sugar-phosphate/phosphate exchanger), SLC38 (System A & N, sodium-coupled neutral amino acid transporter), SLC39 (metal ion transporter), SLC40 (basolateral iron transporter), SLC41 (MgtE-like magnesium transporter), SLC42 (Rh ammonium transporter family (pending)), SLC43 (Na+-independent, system-L like amino acid transporter), SLC44 (Choline-like transporter), SLC45 (Putative sugar transporter), SLC46 (Folate transporter), SLC47 (Multidrug and Toxin Extrusion (MATE)), SLC48 (Heme transporter), SLC49 (FLVCR-related transporter), SLC50 (Sugar efflux transporters), SLC51 (Transporters of steroid-derived molecules), and SLC52 (Riboflavin transporter), SLC53 (Phosphate carriers), SLC54 (Mitochondrial pyruvate carriers), SLC 55 (Mitochondrial cation/proton exchangers), SLC56 (Sideroflexins), SLC57 (NiPA-like magnesium transporter family), SLC58 (MagT-like magnesium transporter family), SLC59 (Sodium-dependent lysophosphatidylcholine symporter family), SLC60 (Glucose transporters), SLC61 (Molybdate transporter family), SLC 62 (Pyrophosphate transporters), SLC63 (Sphingosine-phosphate transporters), SLC64 (Golgi Ca2+/H+ exchangers), SLC65 (NPC-type cholesterol transporters) proteins, more preferably selected from SLC9 (Na+/ H+ exchanger) family proteins,
   even more preferably selected from SLC9A subfamily proteins.
17. The compound of any of items 1 to 16, wherein the TMPBM is a moiety binding to a solute carrier (SLC) protein;
   (i) wherein the SLC (i) is a SLC located on the cell surface, the endoplasmatic reticulum (ER), the Golgi apparatus, the mitochondria, the intracellular vesicles and/or the lysosomes;
      preferably wherein the SLC located on the cell surface is selected from the group consisting of SLC38A1, SLC38A2, SLC2A3, SLC1A5, SLC16A1, SLC4A4, SLC7A3, SLC1A5 and SLC2A1, wherein the SLC located at the ER is selected from the group consisting SLC30A9 and SLC39A7, wherein the SLC on the Golgi apparatus is selected from the groups consisting of SLC35B2 and SLC33A1, wherein the SLC on the mitochondria is selected from the groups consisting of SLC25A50 (also denoted as Mitochondrial carrier homolog 2(MTCH2)) and wherein the SLC located in the lysosomes is selected from the group consisting of SLC38A9.
      more preferably, wherein the SLC located on the cell surface is selected from the group consisting of SLC38A1, SLC38A2, SLC2A3, SLC1A5, SLC16A1, SLC4A4 and SLC7A3;
      and/or
   (ii) wherein the SLC is a member of the SLC9 family:
      preferably wherein the SLC is selected from the group consisting of SLC9A1, SLC9A2, SLC9A3, SLC9A4, SLC9A5, SLC9A6, SLC9A7, SLC9A8, SLC9A9, SLC9B1, SLC9B2, SLC9C1 and SLC9C2,
      more preferably wherein the SLC is selected from the group consisting of SLC9A1, SLC9A2, SLC9A3, SLC9A4, SLC9A5, SLC9A6, SLC9A8, SLC9A9 and SLC9B2,
      even more preferably the SLC is selected from the group consisting of SLC9A1, SLC9A2 and SLC9A4, more preferably the SLC is SLC9A1.
18. The compound of any of items 1 to 17, wherein the TMPBM is a moiety binding to SLC9A1.
19. The compound of any of items 1 to 18, wherein the TMPBM is a moiety binding to a SLC having between 3 and 17 transmembrane domains, preferably between 3 and 14 transmembrane domains, preferably between 3 and 13 transmembrane domains, preferably between 6 and 14 transmembrane domains, preferably between 8 and 14 transmembrane domains, and even more preferably between 8 and 12 transmembrane domains.
20. The compound of any of items 1 to 19, wherein the TMPBM is a moiety binding to an inhibitory site or an allosteric binding site of the SLC.
21. A composition comprising a compound of any of items 1 to 19.
22. The compound of any of items 1 to 20 or the composition of item 21 further comprising a pharmaceutically acceptable diluent, excipient or carrier.
23. The compound of any of items 1 to 20 or 22 or the composition of item 21 or 22 for use as a medicament.
24. A method for use in treating a disease comprising administering a therapeutically effective amount of the compound of any of items 1 to 20, 22 or 23 or a pharmaceutical composition of any of items 21 to 23 to a subject in need of such a treatment.
25. The compound of any of items 1 to 20, 22 to 24, the composition of any of items 21 to 23 or the method of items 24 for use in treating or preventing cancer, particularly leukemia such as chronic myeloid leukemia.

### DESCRIPTION OF THE INVENTION

The present invention relates to a compound having the following formula (I): 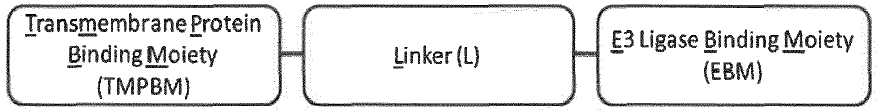 In formula (I), TMPBM is a moiety binding to a transmembrane protein, L is a linker moiety; and EBM is a moiety modifying the function of the E3 ligase and/or binding to at least one member of the E3 ligase complex.

The compounds of formula (I) and other compounds disclosed herein are highly potent PROTACs for the degradation of transmembrane proteins, such as SLCs. By the use of these compounds in particular multi-pass transmembrane proteins of different topology and structural folds are amenable to the proximity-induced proteolysis. This is an important breakthrough with manifold opportunities for the treatment of a wide variety of diseases.

In the following, examples of compounds of formula (I) are presented. It is to be understood that these also encompass any stereoisomers, tautomers, pharmaceutically acceptable salts, solvates and prodrugs (in particular stereoisomers, pharmaceutically acceptable salts and solvates) of the compounds presented as Markush formulae or specific formulae. It is to be understood, that this also applies to any partial structures of compound of formula (1), such as partial formula (II), and partial formula (III).

TMPBM preferably represents a moiety having the following partial formula (II): 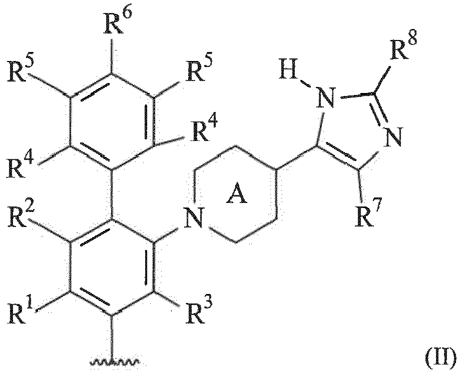 including any stereoisomers, tautomers, pharmaceutically acceptable salts, solvates and prodrugs thereof.

In Formula (II), 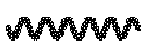 indicates the position of attachment of the partial formula (II) to the linker (L).

R¹, R², R³, R⁴, R⁵, R⁶, R⁷ and R⁸ in Formula (II) are each independently selected from -H, - halogen, -NO₂, -CN, -C₁₋₃ alkyl, -OH, -O-C₁₋₂ alkyl, NH₂, -NH(C₁₋₂ alkyl), -N(C₁₋₂ alkyl)₂, -CHO, -CO(C₁₋₂ alkyl), COOH, COO(C₁₋₂ alkyl), CONH₂, CONH(C₁₋₂ alkyl), and CON(-C₁₋₂ alkyl)₂, wherein each alkyl is optionally substituted with one or more F.

In addition, Ring A in formula (II) is optionally substituted with one or more F.

It is more preferred that in the moiety of partial formula (II) R¹, R², R³ and each R⁴ are each hydrogen, R⁷ is methyl, R⁸ is H or NH₂, and Ring A is not substituted with any F.

In addition, it is preferred that in the moiety of partial formula (II) one R⁵ is selected from H, F, Cl, Me, CF₃, CF₂H and CH₂F; while the other R⁵ is H, and R⁶ is F or Cl.

A particularly preferred example of the moiety of partial formula (II) has the following formula (IIa):

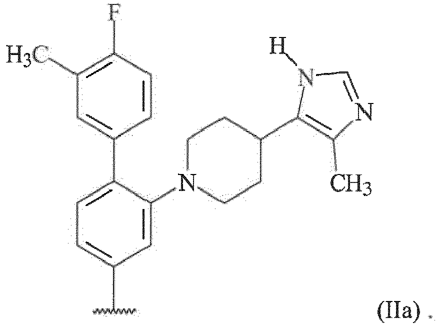

The present inventors have found that in particular compounds of formula (I) in which the linker has a certain length, such as about 14 to about 24 atoms in length, show highly surprisingly good suitability as PROTACs for the degradation of transmembrane proteins, such as SLCs.

In the compounds according to the present invention, L is preferably selected from C₁₄₋₂₄ alkylene, C₁₄₋₂₄ alkenylene, and C₁₄₋₂₄ alkynylene, wherein said alkylene, said alkenylene and said alkynylene are each optionally substituted with one or more groups independently selected from halogen, C₁₋₅ haloalkyl, -O(C₁₋₅ haloalkyl), -CN, -OR²¹, -NR²¹R²¹, -NR²¹OR²¹, -COR²¹, -COOR²¹, -OCOR²¹, -CONR²¹R²¹, -NR²¹COR²¹, -NR²¹COOR²¹, -OCONR²¹R²¹, -SR²¹, -SOR²¹, -SO₂R²¹, -SO₂NR²¹R²¹, -NR²¹SO₂R²¹, -SO₃R²¹, and -NO₂, and further wherein one or more -CH₂- units comprised in said alkylene, said alkenylene or said alkynylene are each optionally replaced by a group independently selected from -O-, -NR²¹-, -CO-, -S-, -SO-, and -SO₂-;
each R²¹ is independently selected from hydrogen, C₁₋₅ alkyl, C₂₋₅ alkenyl, C₂₋₅ alkynyl, carbocyclyl, and heterocyclyl, wherein said alkyl, said alkenyl and said alkynyl are each optionally substituted with one or more groups R^{Alk}, and further wherein said carbocyclyl and said heterocyclyl are each optionally substituted with one or more groups R^{Cyc};
any two R²¹ are optionally linked to form a ring;
each R^{Alk} is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl);
each R^{Cyc} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl).

In L, the C₁₄₋₂₄ alkylene, C₁₄₋₂₄ alkenylene, and C₁₄₋₂₄ alkynylene are preferably C₁₅₋₂₂ alkylene, C₁₅₋₂₂ alkenylene, and C₁₅₋₂₂ alkynylene, respectively, preferably C₁₆₋₂₀ alkylene, C₁₆₋₂₀ alkenylene, and C₁₆₋₂₀ alkynylene respectively wherein each of the alkylenes, alkenylenes and alkynylenes may be modified as set out above. It is further preferred that L is selected from C₁₄₋₂₄ alkylene and C₁₄₋₂₄ alkenylene, more preferably C₁₅₋₂₂ alkylene, C₁₅₋₂₂ alkenylene, even more preferably C₁₆₋₂₀ alkylene, still more preferably C₁₇₋₁₉ alkylene, most preferably C₁₇ alkylene, wherein each of the alkylenes and alkenylenes may be modified as set out above.

Particularly preferred examples of L include one of the following structures:

-O-L¹-NH-C(=O)-

and

-NH-L¹-NH-C(=O)-,

preferably

-NH-L¹-NH-C(=O)-

wherein L¹ is selected from C₁₃₋₁₇ alkylene and C₁₃₋₁₇ alkenylene, wherein said alkylene and said alkenylene are each optionally substituted with one or more groups independently selected from halogen, C₁₋₃ alkyl and C₁₋₃ haloalkyl, and further wherein one or more -CH₂-units comprised in said alkylene or said alkenylene is/are replaced by a group independently selected from -O-, -NR²¹-, -CO-, -S-, -SO-, and -SO₂-.

It is preferred that L¹ is selected from C₁₃₋₁₇ alkylene, wherein said alkylene is optionally substituted with one or more groups independently selected from halogen, C₁₋₃ haloalkyl and C₁₋₃ haloalkyl, and further wherein one or more -CH₂- units comprised in said alkylene or said alkenylene is/are replaced by a group independently selected from -O-, -NR²¹- and - CO-.

Specific examples of L¹ include:

-CH₂-CH₂-O-CH₂-CH₂-O-CH₂-CH₂-O-CH₂-CH₂-O-CH₂-CH₂-,

and

-CH₂-C(=O)-NH-CH₂-CH₂-O-CH₂-CH₂-CH₂-O-CH₂7-CH₂-CH₂-O-CH₂-CH₂-.

Specific examples of L include:

-NH-CH₂-CH₂-O-CH₂-CH₂-O-CH₂-CH₂-O-CH₂-CH₂-O-CH₂-CH₂-NH-C(=O)-,

and

-O-CH₂-C(=O)-NH-CH₂-CH₂-O-CH₂-CH₂-CH₂-O-CH₂-CH₂-CH₂-O-CH₂-CH₂-NH-C(=O)-.

More preferred is that the -O- or -NH- on the left hand side of the above examples of L is bound to EBM as defined herein and the NH-C(=O)- on the right hand side is bound to the TMPBM as defined herein.

EBM is preferably a moiety binding to at least one member of the E3 ligase complex. EBM in the compound of formula (I) is more preferably a moiety binding to at least one member of the E3 ligase complex with a Kd of 10 µM or less, as determined by the assay set out in the present application.

In particular, the binding of the EBM as disclosed herein and used in context of the invention to at least one member of the E3 ligase complex, such as the substrate receptor CRBN, can be measured by using assays based on the NanoBRET™ technology, according to the manufacturer's protocol and recommendations (Promega; NanoBRET™ assay).

Likewise, the interaction between a transmembrane protein, such as SLC9A1, as disclosed herein and used in context of the invention and at least one member of the E3 ligase complex, such as the substrate receptor CRBN, can be measured by using assays based on the NanoBRET™ technology, according to the manufacturer's protocol and recommendations (Promega; NanoBRET™ assay).

Likewise, the interaction between a compound, such as a PROTAC as disclosed herein and used in context of the invention, and at least one member of the E3 ligase complex, such as the substrate receptor CRBN, can be measured by using assays based on the NanoBRET™ technology, according to the manufacturer's protocol and recommendations (Promega; NanoBRET™ assay).

Thereby, the NanoBRET™ assay allows measuring proximity of an EBM, a compound, such as a PROTAC or a transmembrane protein, to at least one member of the E3 ligase complex, such as a substrate receptor, like CRBN or VHL, by determining the energy transfer from a luminescent donor to a fluorescent acceptor (see, e.g., Riching et al. ACS Chem. Biol. 2018, 13, 2758-2770). In this context, the ternary complex formation of a compound comprising an EBM, such as a PROTAC, an E3 ligase member, such as a substrate receptor, and a target protein, such as a transmembrane protein/transmembrane proteins, can be measured.

For the expression of a donor in HEK293 cells, a transmembrane protein can be cloned into a plasmid comprising a luciferase, such as NanoLuc® luciferase. For example, the transmembrane protein, such as SLC9A1, can be cloned into the NanoLuc donor plasmid (Promega; NanoLuc® luciferase) at the C-terminus. Alternatively, a transmembrane protein used as a donor on this assay can also be tagged at another position and/or site, for example on the N-terminus or a loop of the transmembrane protein that is directed to the cytoplasm. The person skilled in the art knows which positions and sites can be used for tagging a transmembrane protein and is aware which transmembrane proteins are feasible to be tagged at a particular position or site. The donor may alternatively be tagged, for example with HiBiT using CRISPR-Cas9. As another alternative, the assay can be adapted to become a TR-FRET assay by changing NanoLuc to a tag that enables labeling with fluorophore (for example but not limited to SNAP-tag, followed by Tb3+ cryptate labelling). Therefore, it can be generalized that the nanoBRET assay can also be transferred to TR-FRET. The donor may also comprise a compound comprising an EBM, such as a PROTAC, as disclosed herein and used in context of the invention.

For expression of an acceptor in HEK293 cells, a HaloTag can be employed that is attached to a E3 ligase member, such as the substrate receptor CRBN (Promega; HaloTag®). Alternatively, any other tag can be used that enables labeling with an acceptor fluorophore. Examples of such tags include but are not limited to SNAPtag, CLIPtag, biotin and streptavidin. The donor and/or acceptor can be transiently or stably expressed in these cells.

For example, and according to the manufacturer's protocol and recommendations (Promega; NanoBRET™ assay), HEK293 cells are seeded in 6-well plates, at 800k cells per well. The two plasmids expressing the acceptor and donor are transiently transfected in HEK293 at a ratio 1:1, using FuGene. The next day, the transfected cells are trypsinized, washed, mixed with nanoBRET 618 ligand and re-seeded in a 96-well white plate. The next day, cells are treated with 10uM MG132 for 1 hour and then treated for 6 hours with 7 doses of a compound, such as the compound denoted as PROTAC d9A1-2 (in uM:0.000256, 0.00128, 0.0064, 0.032, 0.16, 0.8, 4, 20). NanoBRET Nano-Glo Substrate are prepared in Opti-MEM, and dispensed (50ul) in each well, followed by 30sec shaking in the plate reader (Molecular Devices). Donor emission (460nm) and acceptor emission (618nm) are measured within 10 minutes of substrate addition on the plate reader (Molecular Devices). The BRET ratio is calculated as the ratio between acceptor emission and donor emission. The raw BRET ratio is normalized to maximum, from which the Kd value are derived.

In view of the above, using assays based on the NanoBRET™ technology, the interaction between transmembrane proteins, such as SLCs, or a compound comprising an EBM, such as a PROTAC, as disclosed herein and in context of the invention and a member of the E3 ligase, such as the substrate receptor of the E3 ligase, such as CRBN or VHL, can be measured. In this context, the Kd values that can be determined by applying this technology comprise Kd values of a transmembrane protein, such as a SLC as provided herein, or a compound comprising an EBM, such as a PROTAC, as disclosed herein and in context of the invention and at least one member of the E3 ligase, such as a substrate receptor. Determination of the Kd by using this assay can also be employed to SLCs from the SLC9 family or other families.

Preferably, EBM is a moiety of partial formula (III): 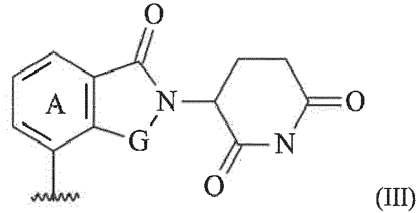 including any stereoisomers, tautomers, pharmaceutically acceptable salts, solvates and prodrugs thereof, in particular stereoisomers, pharmaceutically acceptable salts and solvates thereof, wherein 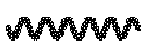 indicates the position of attachment of the partial formula (III) to the linker (L).

G is selected from -C(=O)- and CH₂. Preferably, G is -C(=O)-.

Ring A is optionally substituted with one or more F. More preferably, Ring A is not substituted with one or more F.

It is to be understood that partial compounds of formula (III) may be present in the form of the following two enantiomers:

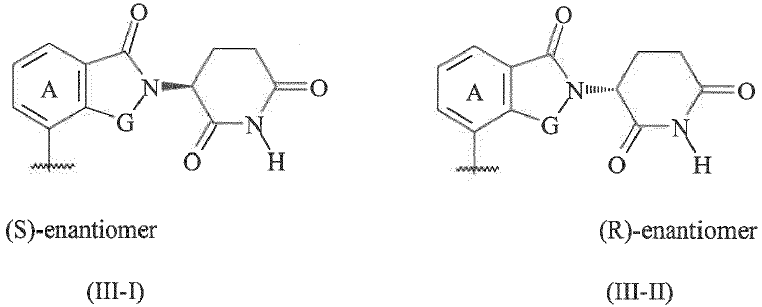

It is preferred that the partial compounds of formula (III) are selected from partial compounds of formula (III-I), in other words, the (S) enantiomer is preferred.

Particularly preferred examples of the compounds of formula (I) include the following: 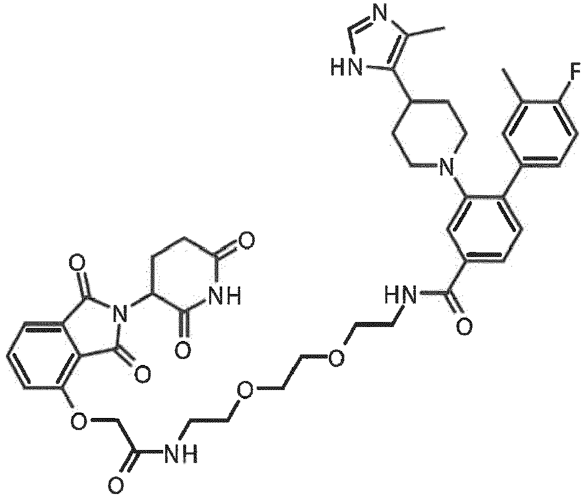 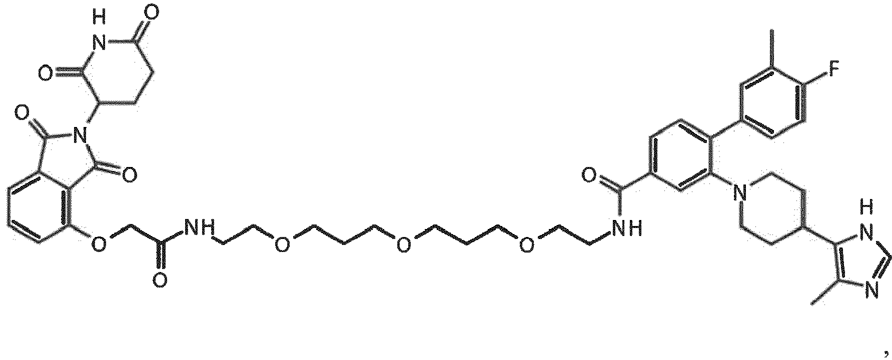 and

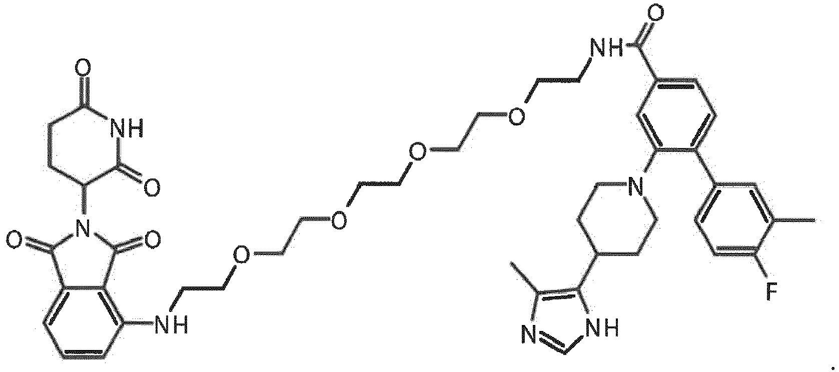

In each of these, the left-hand side part of the molecule preferably has an (S) stereochemistry in the part corresponding to the partial compound of formula (III). It is to be understood that these also encompass any stereoisomers, tautomers, pharmaceutically acceptable salts, solvates and prodrugs of the compounds presented above.

Until now, compounds capable to degrading membrane proteins with several transmembrane regions via degradation by the proteasome system have not been available. The inventors idientified compounds with the ability to degrade membrane proteins with several transmembrane regions by proteasome mediated degradation. Thereby, it was shown in the appended Eaxmples that SLCs, particularly from the SLC9 family such as SLC9A1 which has twelve transmembrane regions, are amenable to proteasome mediated degradation. As shown in the appended Examples, SLCs have been ectopically expressed as proteins tagged with a mutated FKBP domain, which made these SLCs amenable to degradation by specific degrader molecules (e.g. dTAG7/dTAG13) published recently (Nabet, B. et al.. Nat Chem Biol 14, 431-441 (2018). To test targeted degradation of endogenous SLCs, bifunctional molecules, also denoted PROTACs were designed in which we the chemical structure of warhead binding an SLC of interest and the linker connecting this warhead to the thalidomide derivative were varied. It was found that such compounds, for examples those binding SLC9A1, led to efficient degradation of the target. Particularly, it was shown that various human SLCs, localized to different subcellular membrane locations (cell surface, endoplasmic reticulum and lysosome), were amenable degradation by heterobifunctional ligands when fused to the appropriate degradation domain, for example when the chromosomal locus is engineered to express a fusion protein of the SLC with the degradation-mediating tag. It was further shown that the compound as disclosed herein and in context of the invention can lead to the degradation of an SLC protein.. Moreover, it was shown that compounds as described herein and in context of the invention were able to engage and degrade endogenous members of the SLC9 family, a family of sodium/proton exchangers.

Transmembrane proteins with several transmembrane domains, such as SLCs, are attracting the attention of the scientific community but there are few tools to study their function. Particularly, SLCs are increasingly attracting the interest of chemical biologists and drug discoverers as they are involved in a variety of diseases and there are some prominent drug targets (SSRIs, glifozins).

Hence, the inventors found for the first time compounds of new molecular entity targeting multi-pass transmembrane domain proteins. Since the inventors identified compounds which have the ability to degrade multi-pass transmembrane proteins and since several drug targets are multi-pass transmembrane proteins, such as SLCs, the newly identified compounds of the as disclosed herein represent valuable candidate drugs for such targets such as drugs for treating cancer or cardiological diseases. Such compounds may further be able to bind to more than one transmembrane protein, in particular more than one multi-pass transmembrane domain protein. For example, the compound as disclosed herein and in context of the invention may bind to one or more transmembrane proteins of the SLC9A family. For example, the compound as disclosed herein and in context of the invention may bind to at least 2, preferably to at least 5, more preferably to at least 10 transmembrane proteins such as from the SLC9A family.

Further, the compounds as disclosed herein and in context of the invention may be able to target transmembrane proteins within specific locations of the cells, such as the golgi apparatus or the endoplasmatic reticulum, or within specific tissues.

Accordingly, and disclosed herein, the compounds comprise a E3 ligase binding moiety/ EBM, a linker/L and a transmembrane protein binding moiety/TMPBM and are able of stimulating/inducing ubiquitination of a target protein/target proteins, e.g. via degradation of a transmembrane protein/ transmembrane proteins by the ubiquitination system. The terms "E3 ligase binding moiety" and "EBM" or are used interchangeably and means that the E3 ligase binding moiety/ EBM is moiety modifying the function of the E3 ligase and/or binding to at least one regulator or member of the E3 ligase complex/cullin-RING ubiquitin ligase complex/CRL complex. "Modifying the function of the E3 ligase" as used in context of the invention means that the cullin-RING ubiquitin ligase activity/CRL activity is enhanced by the E3 ligase binding moiety/ EBM, for example by binding of the E3 ligase binding moiety/ EBM to the E3 ligase/cullin-RING ubiquitin ligase/CRL or by modifying the function of the E3 ligase complex/cullin-RING ubiquitin ligase complex/CRL complex. The term "cullin RING ubiquitin E3 ligase" or "CRL" are used interchangeably and refer to an ubiquitin ligase in a complex in which the catalytic core consists of a member of the cullin family and a RING domain protein; the core is associated with one or more additional proteins that confer substrate specificity. The RING domain proteins of the CRL mediate the transfer of ubiquitin from the E2 to the E3-bound substrate. In particular, the cullin RING ubiquitin E3 ligase (CRL) are modular multi-subunit complexes that all contain a common core comprising a cullin subunit and a zinc-binding RING domain subunit. In particular, the cullin subunit folds into an extended structure that forms the backbone of CRLs. The C-terminal region of the cullin subunit forms a globular domain that wraps itself around the RING protein, which in turn recruits the E2 conjugating enzyme to form the enzymatic core. The N-terminal region of the cullin subunit, which resides at the opposite end of the elongated cullin structure, recruits substrate receptors via adapter proteins.

In context of the invention, the compound has the capacity of stimulating/inducing ubiquitination of a transmembrane protein/transmembrane proteins by enhancing the cullin-RING ubiquitin ligase activity/CRL activity. As discosed herein and in context of the invention, the E3 ligase binding moiety/EBM may be pomalidomide, thalidomide and lenalidomide. For examples, the EBM may be pomalidomide or thalidomide. As another example, the E3 ligase binding moiety/EBM may be pomalidomide. Particularly, the E3 ligase binding moiety/EBM may be pomalidomide (see, e.g., Winter, G. E. et al. DRUG DEVELOPMENT.. Science 348, 1376-1381 (2015)).

Said "enhanced cullin-RING ubiquitin ligase activity"/"enhanced CRL activity" means that said cullin-RING ubiquitin ligase activity/CRL activity is enhanced in the presence of the compound of the present invention compared to the cullin-RING ubiquitin ligase activity/CRL activity in the absence of said compound. Accordingly, the present invention relates to a compound with the capacity to induce and/or stimulate the ubiquitination of a transmembrane protein/ transmembrane proteins via enhancing the CRL activity. As disclosed herein and as illustrated in the appended Examples, said enhanced activity of the E3 ligase complex/cullin-RING ubiquitin ligase complex/CRL complex may be determined by the measurement of the level/amount of transmembrane protein/transmembrane proteins in a cell expressing the transmembrane protein/transmembrane proteins in the presence of the compound and compared to the level in the absence of said compound, wherein a decreased level of the transmembrane protein/transmembrane proteins in the presence of said compound compared to the level in the absence of said compound indicates the capacity of said compound to induce and/or stimulate the ubiquitination of a transmembrane protein/ transmembrane proteins, for example via enhancing the CRL activity.

The enhanced CRL activity may be induced by the presence of said compound. Said compound may be able to induce molecular proximity between a component of a E3 ligase complex/cullin-RING ubiquitin ligase complex/CRL complex and a transmembrane protein/transmembrane proteins which may be bound to the compound or which may be part of a ternary complex comprising the E3 ligase complex/cullin-RING ubiquitin ligase complex/CRL complex, the target protein/target proteins and the compound. Means and methods known in the art of how to determine the binding of the E3 ligase binding moiety/ EBM may to the E3 ligase complex/cullin-RING ubiquitin ligase complex/CRL complex comprise, inter alia, immunoassays (like Western blots, ELISA tests and the like) and/or reporter assay (like luciferase assays and the like). Such means and methods to determine the binding of a compound to the E3 ubiquitin ligase can be determined, for example, by immunoassays as for instance but not limited to radioimmunoassays, chemiluminescence- and fluorescence- immunoassays, Enzyme-linked immunoassays (ELISA), Luminex-based bead arrays, protein microarray assays, assays suitable for point-of-care testing and rapid test formats such as for instance immune-chromatographic strip tests. Suitable immunoassays may be selected from the group of immunoprecipitation, enzyme immunoassay (EIA)), enzyme-linked immunosorbenassays (ELISA), radioimmunoassay (RIA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay or Luminex ® Assays. An immunoassay is a biochemical test that measures the presence or concentration of a macromolecule/polypeptide in a solution through the use of an antibody or immunoglobulin as a binding agent. In principle, all labeling techniques which can be applied in assays of said type can be used, such as labeling with radioisotopes, enzymes, fluorescence-, chemoluminescence- or bioluminescence labels and directly optically detectable color labels, such as gold atoms and dye particles.

The enhanced activity of the E3 ligase complex/cullin-RING ubiquitin ligase complex/CRL complex may also be determined by methods known in the art, such as surface plasmon resonance spectroscopy (SPR) or Förster resonance energy transfer (FRET), and methods as described herein and as illustrated in the appended examples. For example, such methods may include, but are not limited to FRET (Forster Resonance Energy Transfer) analysis. The theory of FRET (Forster Resonance Energy Transfer) defines a distance dependent, non-radiative transfer of energy from an excited donor (D) to an acceptor molecule (A). The relationship between easily accessible spectroscopic data and theoretical equations was the achievement of Theodor Förster, thereby enabling the possibility of many FRET applications in all kinds of natural sciences. FRET has been used in biochemical applications within the 1 to 10 nm scale (K. E. Sapsford et al., Angew. Chem. Int. Ed., 45, 4562, 2006) (e.g. protein-protein binding, protein folding, molecular interactions at and in cell membranes, DNA hybridization and sequencing, immunoreactions of antigens and antibodies). Details of the theory of FRET are well known. Further examples include protein complementation assay (PCA). Protein complementation assays (PCA) provide a means to detect the interaction of two biomolecules, e.g., polypeptides. PCA utilizes two fragments of the same protein, e.g., enzyme, that when brought into close proximity with each other can reconstitute into a functional, active protein.

Further, binding of a compound to the E3 ubiquitin ligase may be detected, for example, in a Western Blot. Western blotting involves application of a protein sample (lysate) onto a polyacrylamide gel, subsequent separation of said complex mixture by electrophoresis, and transferal or "electro-blotting" of separated proteins onto a second matrix, generally a nitrocellulose or polyvinylidene fluoride (PVDF) membrane. Following the transfer, the membrane is "blocked" to prevent nonspecific binding of antibodies to the membrane surface. Many antibody labeling or tagging strategies are known to those skilled in the art. In the simplest protocols, the transferred proteins are incubated or complexed with a primary enzyme-labeled antibody that serves as a probe. After blocking non-specific binding sites a suitable substrate is added to complex with the enzyme, and together they react to form chromogenic, chemiluminescent, or fluorogenic detectable products that allow for visual, chemiluminescence, or fluorescence detection, respectively. This procedure is described by Gordon et al., U.S. Patent 4,452,901 issued June 15, 1984.

As is evident from the appended Examples, the compound of the present invention may bind a transmembrane protein/transmembrane proteins via the transmembrane protein binding moiety/TMPBM of the compound and bind or modify the function of the E3 ligase complex/cullin-RING ubiquitin ligase complex/CRL complex, for example by recruiting the transmembrane protein/transmembrane proteins bound to the transmembrane protein binding moiety/TMPBM of the compound to the E3 ligase complex/cullin-RING ubiquitin ligase complex/CRL complex. As another example, the compound in context of the invention may alter the function of a transmembrane protein/transmembrane proteins, for example by modifying posttranslational changes of a transmembrane protein/transmembrane proteins. A posttranslational modification may include but is not limited to the phosphorylation status of a transmembrane protein, e.g. a tyrosine kinase phosphorylating a transmembrane protein. Thus, the compound may induce ubiquitination of a transmembrane protein/transmembrane proteins, e.g., by modifying a transmembrane protein/transmembrane proteins in that the target protein becomes accessible for a E3 ligase complex/cullin-RING ubiquitin ligase complex/CRL complex, thereby the compound may not associate with a transmembrane protein/transmembrane proteins and/or E3 ligase complex/cullin-RING ubiquitin ligase complex/CRL complex.

As described herein and in context of the invention, the TMPBM may be a moiety binding to a transmembrane protein/transmembrane proteins. In one embodiment, the TMPBM may be a moiety binding to a SLC having between 3 and 17 transmembrane domains, such as between 3 and 14 transmembrane domains or 3 and 13 transmembrane domains, particularly between 6 and 14 transmembrane domains, more particularly between 8 and 14 transmembrane domains, even more particularly between 8 and 12 transmembrane domains. As used herein, the term "transmembrane protein" or "transmembrane protein(s)", as used herein, refers to a protein that is attached to or associated with a membrane of a cell or an organelle. They are often subdivided into several categories including integral membrane proteins, peripheral membrane proteins and lipid-anchored proteins. For example, the membrane protein is an integral membrane protein that is permanently bound to the lipid bilayer and which requires a detergent or another apolar solvent to be removed. Integral membrane proteins include transmembrane proteins that are permanently attached to the lipid membrane and span across the membrane one or several times. Examples of suitable membrane proteins include receptors such as GPCRs and growth factor receptors; transmembrane ion channels such as ligand-gated and voltage gated ion channels; transmembrane transporters such as neurotransmitter transporters; enzymes; carrier proteins; ion pumps; viral membrane proteins and supramolecular complexes thereof, amongst others.

Specific non-limiting examples of transmembrane proteins as used herein are provided further in the specification. For example, transmembrane proteins may function as gateways to permit transport of specific substances across the membrane. They may undergo conformational changes to move a substance through the membrane. Typically, the peptide sequence spanning the membrane or the transmembrane domain, is mainly hydrophobic. Membrane proteins are involved in signal transduction, mass transportation, energy production, formation of cytoskeleton etc. at cell membranes. In addition, the membrane protein is very important as a potential drug target. Non-limiting examples of such membrane transport proteins may be carriers and/or channels. Particularly non-limiting examples of such membrane transport proteins may be carriers. As used herein, the term "membrane transport proteins", "carrier" or "transporter" refers to transmembrane proteins which are embedded (singly or in complexes) in the cellular membrane (reviewed by Oh and Amidon (1999) in Membrane Transporters as Drug Targets, ed. Amidon and Sadee, Kluwer Academic/Plenum Publishers, New York, Chapter 1). There are two general classes of carriers/membrane transport proteins: channels or pores, and transporters (also known as carriers or permeases). Channels and transporters differ in their translocation mechanisms. Channels are hydrophilic group-lined protein tunnels whose opening by a regulatory event allow free, rapid passage of their charge-, size-, and geometry-selected small ions down their concentration gradients. Transporters specifically and selectively bind the molecules they move, some with and some against their concentration gradients, across membranes.

Carriers/membrane transporters are critical facilitators of the uptake (e.g. solute carrier family (SLC) transporters) and export (e.g. ABC transporters) of drugs. Such carriers/membrane transporters can alter drug disposition and distribution in several important ways. Many of these carriers/membrane transporters play key roles in pharmacology. As such, these carriers/membrane transporters play critical roles in mediating both the chemo-sensitivity and chemo-resistance of cancer cells to cancer chemotherapeutics. ABC transporters are frequently associated with decreased intracellular concentration of chemotherapeutic agents and acquired multi-drug resistance of tumor cells. SLC transporters, including anion, cation, nucleoside and amino acid transporters, are associated with increased sensitivity of tumor cells to chemotherapeutic agents since these transporters facilitate the cellular uptake of hydrophilic compounds. Carriers/membrane transporters can be classified as either passive or active transporters. The active transporters can be further divided into primary or secondary active transporters based on the process of energy coupling and facilitated transport. The ABC transporters are primary active transporters which export compounds against a chemical gradient driven by ATP and an inherent ATPase activity. The majority of passive transporters, which permit compounds to equilibrate along a concentration gradient, ion pumps, secondary active transporters and exchangers belong to the SLC transporter family. Transporters can move molecules by two types of processes. In one process, "facilitated diffusion" transporters move molecules with their concentration gradients. In the other process, "active transport," transporters move molecules against their concentration gradients. Active transport to move a molecule against its gradient requires energy. Primary active transporters, such as Na⁺/K⁺ ATPases or ABC transporters use energy from ATP hydrolysis or light, and establish ion gradients and membrane potential energy. Secondary active transporters, such as the H⁺/peptide transporter, use the pH or ion gradients established by primary active transporters to transport other molecules. In secondary active transport, the transporter uses two separate binding sites to move the primary ion down its concentration gradient to produce the energy to move the secondary solute against its gradient. The coupled solute either travels in the same direction as the primary solute (symport) or in the opposite direction (antiport).

The term "transport proteins" and "transport molecules" are used interchangeably and refer to transport proteins that are specific for a particular target solute or class of solutes, and are also present in one or more specific membranes. Transport molecules localized to the plasma membrane permit an exchange of solutes with the surrounding environment, while transport molecules localized to intracellular membranes (e.g., golgi apparatus, vesicles, membranes of the mitochondrion, peroxisome, lysosome, endoplasmic reticulum, nucleus, or vacuole) permit import and export of molecules from organelle to organelle or to the cytoplasm. For example, in the case of the mitochondrion, transporters in the inner and outer mitochondrial membranes permit the import of sugar molecules, calcium ions, and water (among other molecules) into the organelle and the export of newly synthesized ATP to the cytosol.

Transporters play important roles in the ability of the cell to regulate homeostasis, to grow and divide, and to communicate with other cells, e.g., to transport signaling molecules, such as hormones, reactive oxygen species, ions, neurotransmitters or vitamins. A wide variety of human diseases and disorders are associated with defects in transporter or other membrane transport molecules, including certain types of liver disorders (e.g., due to defects in transport of long-chain fatty acids (A1 Odaib et al. (1998) New Eng. J. Med. 339:1752-1757), hyperlysinemia (mitochondrial lysine transport defect (Oyanagi et al. (1986) Inherit. Metab. Dis. 9:313-316)), and cataract (Wintour (1997) Clin. Exp. Pharmacol. Physiol. 24(1):1-9).

As described herein and in context of the invention, the TMPBM may be a moiety binding to a solute carrier (SLC) protein, for example but not limited to a TMPBM binding to a SLC selected from a SLC of the SLC family selected from the group consisting of SLC1 (high affinity glutamate and neutral amino acid transporter), SLC2 (facilitative GLUT transporter), SLC3 (heavy subunits of the heteromeric amino acid transporters), SLC4 (bicarbonate transporter), SLC5 (sodium glucose cotransporter), SLC6 (sodium- and chloride-dependent neurotransmitter transporter), SLC7 (cationic amino acid transporter/glycoprotein-associated amino-acid transporter), SLC8 (Na+/Ca2+ exchanger), SLC9 (Na+/ H+ exchanger), SLC10 (sodium bile salt cotransport), SLC11 (proton coupled metal ion transporter), SLC12 (electroneutral cation-Cl cotransporter), SLC13 (human Na+-sulfate/carboxylate cotransporter), SLC14 (urea transporter), SLC15 (proton oligopeptide cotransporter), SLC16 (monocarboxylate transporter), SLC17 (vesicular glutamate transporter), SLC18 (vesicular amine transporter), SLC19 (folate/thiamine transporter), SLC20 (type III Na+-phosphate cotransporter), SLC21/SLCO (organic anion transporting), SLC22 (organic cation/anion/zwitterion transporter), SLC23 (Na+-dependent ascorbic acid transporter), SLC24 (Na+/(Ca2+-K+) exchanger), SLC25 (mitochondrial carrier), SLC26 (multifunctional anion exchanger), SLC27 (fatty acid transport protein), SLC28 (Na+-coupled nucleoside transport), SLC29 (facilitative nucleoside transporter), SLC30 (zinc efflux), SLC31 (copper transporter), SLC32 (vesicular inhibitory amino acid transporter), SLC33 (Acetyl-CoA transporter), SLC34 (type II Na+-phosphate cotransporter), SLC35 (nucleoside-sugar transporter), SLC36 (proton-coupled amino acid transporter), SLC37 (sugar-phosphate/phosphate exchanger), SLC38 (System A & N, sodium-coupled neutral amino acid transporter), SLC39 (metal ion transporter), SLC40 (basolateral iron transporter), SLC41 (MgtE-like magnesium transporter), SLC42 (Rh ammonium transporter family (pending)), SLC43 (Na+-independent, system-L like amino acid transporter), SLC44 (Choline-like transporter), SLC45 (Putative sugar transporter), SLC46 (Folate transporter), SLC47 (Multidrug and Toxin Extrusion (MATE)), SLC48 (Heme transporter), SLC49 (FLVCR-related transporter), SLC50 (Sugar efflux transporters), SLC51 (Transporters of steroid-derived molecules), and SLC52 (Riboflavin transporter), SLC53 (Phosphate carriers), SLC54 (Mitochondrial pyruvate carriers), SLC 55 (Mitochondrial cation/proton exchangers), SLC56 (Sideroflexins), SLC57 (NiPA-like magnesium transporter family), SLC58 (MagT-like magnesium transporter family), SLC59 (Sodium-dependent lysophosphatidylcholine symporter family), SLC60 (Glucose transporters), SLC61 (Molybdate transporter family), SLC 62 (Pyrophosphate transporters), SLC63 (Sphingosine-phosphate transporters), SLC64 (Golgi Ca2+/H+ exchangers), SLC65 (NPC-type cholesterol transporters) proteins. Particularly non-limiting examples the TMPBM may bind to SLC9 (Na+/ H+ exchanger) family proteins, even more preferably selected from SLC9A subfamily proteins.

There are over 30 families of secondary transporters, also known as solute carriers or SLC (reviewed by Berger, et al. (2000) in The Kidney: Physiology and Pathophysiology, eds. Seldin DW and Giebisch ., Lippincott, Williams & Wilkins, Philadelphia 1:107-138; see also the website maintained by the HUGO gene nomenclature committee, University College London, UK; in particular Gene Nomenclature Committee (HGNC); see also (http://slc.bioparadigms.org/; https://www.ncbi.nlm.nih.gov/pmc/articles/PMC3853582; https://salilab.org/pdf/Schlessinger_ClinPharmacolTher_2013.pdf; https://www.tandfonline.com/doi/pdf/10.1080/09687688.2018.1448123).

For example, sequences of the SCL9 family may comprise the sequences as provided by https://www.uniprot.org. Such sequences of the SLC9 family include but are not limited to SLC9A1, also denoted as P19634, and having SEQ ID NO.1; SLC9A2, also denoted as Q9UBYO, and having the SEQ ID NO.2; SLC9A3, also denoted as P48764, and having SEQ ID NO.3; SLC9A4, also denoted as Q6AI14 and having SEQ ID NO.4; SLC9A4, also denoted as Q6AI14 and having SEQ ID NO.4; SLCA5, also denoted as Q14940 and having SEQ ID NO.5; SLCA6, also denoted as Q92581 and having SEQ ID NO.6; SLCA7, also denoted as Q96T83; SLCA8, also denoted as Q9Y2E8 and having SEQ ID NO.7; SLCA9, also denoted as Q8IVB4 and having SEQ ID NO.8; SLCB1, also denoted as Q4ZJI4; SLCB2, also denoted as Q86UD5 and having SEQ ID NO.9; SLC9C1, also denoted as Q4G0N8; and SLC9C2, also denoted as Q5TAH2.

As used herein, the term "SLC", "solute carrier" or "SLC transporter" are used interchangeably and refer to a protein encoded by the corresponding SLC gene, e.g. by the term "SLC9A1 polypeptide" is meant a polypeptide encoded by SLC9A1 gene. The SLCs are also known in the art by other names recognized by the skilled person, e.g. SLC9A1 is also known in the art as NHE-1 .

"SLC", "solute carrier" or "SLC transporter" as used herein may belong to different members such as for example but not limited to amino acid transporter. The following members of the solute carrier families are:
(A) high affinity glutamate and neutral amino acid transporters including SLC1A1, SLC1A2, SLC1A3, SLC1A4, SLC1A5, SLC1A6, SLC1A7;
(B) facilitative GLUT transporter including, SLC2A1, SLC2A2, SLC2A3, SLC2A4, SLC2A5, SLC2A6, SLC2A7, SLC2A8, SLC2A9, SLC2A10, SLC2A11, SLC2A12, SLC2A13, SLC2A14;
(C) heavy subunits of heteromeric amino acid transporters including SLC3A1, SLC3A2;
(D) bicarbonate transporters including SLC4A1, SLC4A1, SLC4A2, SLC4A3, SLC4A4, SLC4A5, SLC4A6, SLC4A7, SLC4A8, SLC4A9, SLC4A10, SLC4A11;
(E) sodium glucose cotransporters SLC5A1, SLC5A2, SLC5A3, SLC5A4, SLC5A5, SLC5A6, SLC5A7, SLC5A8, SLC5A9, SLC5A10, SLC5A11, SLC5A12; (F) sodium- and chloride- dependent neurotransmitter transporters including SLC6A1, SLC6A2, SLC6A3, SLC6A4, SLC6A5, SLC6A6, SLC6A7, SLC6A8, SLC6A9, SLC6A10, SLC6A11, SLC6A12, SLC6A13, SLC6A14, SLC6A15, SLC6A16, SLC6A17, SLC6A18, SLC6A19, SLC6A20;
(G) cationic amino acid transporter/glycoprotein-associated including SLC7A1, SLC7A2, SLC7A3, SLC7A4, SLC7A5, SLC7A6, SLC7A7, SLC7A8, SLC7A9, SLC7A10, SLC7A11, SLC7A13, SLC7A14;
(H) Na+/Ca2+ exchangers including SLC8A1, SLC8A2, SLC8A3;
(I) Na+/H+ exchangers including SLC9A1, SLC9A2, SLC9A3, SLC9A4, SLC9A5, SLC9A6, SLC9A7, SLC9A8, SLC9A9, SLC9A10, SLC9A11;
(J) organic cation/anion/zwitterion transporters including SLC22A1, SLC22A2, SLC22A3, SLC22A4, SLC22A5, SLC22A6, SLC22A7, SLC22A8, SLC22A9, SLC22A10, SLC22A11, SLC22A12, SLC22A13, SLC22A14, SLC22A15, SLC22A16, SLC22A17, SLC22A18, SLC22A19, SLC22A20;
(K) Na+/(Ca2+-K+) exchangers including SLC24A1, SLC24A2, SLC24A3, SLC24A4, SLC24A5, SLC24A6;
(L) multifunctional anion exchangers including SLC26A1, SLC26A2, SLC26A3, SLC26A4, SLC26A5, SLC26A6, SLC26A7, SLC26A8, SLC26A9, SLC26A10, SLC26A11;
(M) H+/amino acid symporters including SLC36A1, SLC36A2, SLC36A3, SLC36A4;
(N) System A & N, sodium-coupled neutral amino acid transporters including SLC38A1, SLC38A2, SLC38A3, SLC38A4, SLC38A5, SLC38A6; and (O) Na+-independent, system-L like amino acid transporters including SLC43A1, SLC43A2, SLC43A3;
(O) Na+-independent, system-L like amino acid transporters including SLC43A1, SLC43A2, SLC43A3.

The transmembrane protein/transmembrane proteins may be ubiquitinated by the E3 ligase complex/cullin-RING ubiquitin ligase complex/CRL complex. Particularly, the inventors found that transmembrane protein/transmembrane proteins can be recognized by the compounds of the present invention. Such transmembrane protein/transmembrane proteins may further include proteins which are not recognized E3 ligase complex/cullin-RING ubiquitin ligase complex/CRL complex in the absence of the compound of the present invention. Thus, it has been surprisingly found that the compounds of the present invention are able to induce ubiquitination of the transmembrane protein/transmembrane proteins, i.e. via degradation of the transmembrane protein/transmembrane proteins by the ubiquitination system.

In one particular embodiment, the TMPBM may be a moiety binding to a solute carrier (SLC) protein;
(i) wherein the SLC (i) may be a SLC located on the cell surface, the endoplasmatic reticulum (ER), the Golgi apparatus, the mitochondria, the intracellular vesicles and/or the lysosomes; for example wherein the SLC located on the cell surface may be selected from the group consisting of SLC38A1, SLC38A2, SLC2A1, SLC2A3, SLC16A1; SLC1A5; SLC4A4 and SLC7A3; wherein the SLC located at the ER may be selected from the group consisting SLC30A9 and SLC39A7, wherein the SLC on the Golgi apparatus may be SLC35B2 and SLC33A1, wherein the SLC on the mitochondria may be SLC25A50 (also denoted as Mitochondrial carrier homolog 2(MTCH2)) and wherein the SLC located in the lysosomes may be SLC38A9. More particularly, the SLC located on the cell surface may be SLC38A1, SLC38A2, SLC2A3, SLC1A5, SLC16A1, SLC4A4, SLC7A3, SLC1A5 and SLC2A1, even more particularly SLC38A1, SLC38A2, SLC2A3, SLC1A5, SLC16A1, SLC4A4 and SLC7A3;
   and/or
(ii) wherein the SLC may be a member of the SLC9 family: for example wherein the SLC maybe SLC9A1, SLC9A2, SLC9A3, SLC9A4, SLC9A5, SLC9A6, SLC9A7, SLC9A8, SLC9A9, SLC9B1, SLC9B2, SLC9C1 and SLC9C2. Particularly, the SLC may be SLC9A1, SLC9A2, SLC9A3, SLC9A4, SLC9A5, SLC9A6, SLC9A8, SLC9A9 and SLC9B2. Even more particularly, the SLC may be SLC9A1, SLC9A2 and SLC9A4 and even more particularly the SLC may be SLC9A1. In one embodiment, the TMPBM may be a moiety binding to SLC9A1.

The intracellular vesicles as disclosed herein and in context of the invention may be for example, synaptic vesicles, insulin granules or phagosomes. The person skilled in the art can clearly identify intracellular vesicles carrying a transmembrane protein, such as an SLC protein and can further identify subtypes of transmembrane proteins such as from the SLC family on such an intracellular vesicle.

In one embodiment, the TMPBM may be a moiety binding to an inhibitory site or an allosteric binding site of the transmembrane protein such as SLC. As used herein, the term "orthosteric binding site" refers to the site within a transmembrane protein that binds an endogenous compound. By contrast, the term "allosteric binding site" refers to a site other than an orthosteric binding site to which compounds bind. Accordingly, allosteric compounds bind to a transmembrane protein through a site that is spatially distinct from the orthosteric binding site. For example, binding of a compound as disclosed herein and as used in context of the invention to an allosteric binding site of the transmembrane protein may maintain the functionality of said transmembrane protein. As another example, binding of a compound as disclosed herein and as used in context of the invention to an allosteric binding site of the transmembrane protein may result in a conformational change of said transmembrane protein which may alter the functionality of said transmembrane protein. The term "inhibitory binding site" refers to the site within a transmembrane protein that binds an inhibitory compound. In other words, a compound as disclosed herein and as used in context of the invention bound to an inhibitory binding site of the transmembrane protein/transmembrane proteins may decrease and/or deplete the function of said transmembrane protein/transmembrane proteins.

In one embodiment, the present invention provides compounds that stimulate/induce ubiquitination of a target protein/target proteins, i.e. via transmembrane protein/transmembrane degradation by the cullin RING E3 ligase, wherein the compound is a compound of compounds of formulae (I) as disclosed herein and illustrated in the appended examples. A corresponding read-out whether the compound to be identified in the methods of this invention is able and/or capable to stimulate/induce ubiquitination of a transmembrane protein/transmembrane proteins may be the measurement of the level of the transmembrane protein/transmembrane proteins. When said "level" of the target transmembrane protein/transmembrane proteins is decreased in the presence of the compound compared to the "level" determined in the absence of the compound, said compound is able to stimulate/induce the activity of the E3 ubiquitin ligase. For example, the prediction whether a compound is able to degrade a transmembrane protein/transmembrane proteins, in particular by inducing ubiquitination of a transmembrane protein/transmembrane proteins can be assessed, e.g., by the determination of a fold change in transmembrane protein/transmembrane proteins level. Hereby, the respective fold change value for identifying a compound to degrade a protein refers to measurements of the level of a transmembrane protein/transmembrane proteins. Fold change values may be determined by previously described methods known in the art. For example, methods are known to a skilled person for using the Coefficient of variation in assessing variability of quantitative assays in order to establish fold change values (Reed et al., Clin Diagn Lab Immunol.2002; 9(6):1235-1239).

In one embodiment, the present invention relates to a compound for use in medicine. The term "medicine" as used herein is intended to be a generic term inclusive of prescription and non-prescription medications. The compound for use in medicine should be understood as being useful in maintaining health or promoting recovery from a disease, preferably cancer. Further, the term "medicine" includes medicine in any form, including, without limitation, e.g., pills, salves, creams, powders, ointments, capsules, injectable medications, drops, vitamins and suppositories. The scope of this invention is not limited by the type, form or dosage of the medicine. In one aspect of the present invention, the chemical compound or agent is for use in the treatment of cancer.

In context of the invention, the compounds are particularly useful as medicaments, for example in the treatment of diseases and/or disorders wherein it is desired to degrade target protein/ target proteins via ubiquitination. Thus, in one embodiment, the invention refers to a composition comprising a compound as disclosed herein and in context of the invention. The term "composition", "pharmaceutical composition" or "pharmaceutical formulation" refers to a preparation which is in such form as to permit the biological activity of an active ingredient contained therein to be effective, and which contains no additional components which are unacceptably toxic to a subject to which the pharmaceutical composition would be administered. As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, decreasing the rate of disease progression, amelioration or palliation of the disease state, and remission or improved prognosis. "Alleviation," "alleviating," or equivalents thereof, refer to both therapeutic treatment and prophylactic or preventative measures, wherein the object is to ameliorate, prevent, slow down (lessen), decrease or inhibit a disease or condition, e.g., the formation of atherosclerotic plaques. Those in need of treatment include those already with the disease or condition as well as those prone to having the disease or condition or those in whom the disease or condition is to be prevented.

The present invention also provides for methods of treating such diseases or disorders, said methods comprising the administration to an individual in need of such a treatment with the compound of the invention, i.e. the compound that can stimulating/induce ubiquitination of a target protein/target proteins. A "disorder," a "disease," or a "condition," as used interchangeably herein, is any condition that would benefit from treatment with a composition (e.g., a pharmaceutical composition) described herein. This includes chronic and acute disorders or diseases including those pathological conditions which predispose the mammal to the disorder in question.

A large number of diseases may be treated or prevented by the compounds of the present invention. These include 2p21 microdeletion syndrome, abnormal coordination, abnormality of the cornea, absence seizures, ache, type ib (disorder) achondrogenesis, acidosis, acrodermatitis, acrodermatitis enteropathica, acute cerebrovascular accidents, acute confusional senile dementia, acute kidney injury, acute kidney insufficiency, acute lung injury, adenocarcinoma of lung (disorder), adenoid cystic carcinoma, adenoma, basal cell adenoma, microcystic adenoma, monomorphic adenoma, trabecular adenoma, adult fanconi syndrome, adult learning disorders, adult neuronal ceroid lipofuscinosis, adult-onset citrullinemia type 2, adverse reaction to drug, psychotic affective disorders, age related macular degeneration, age-related memory disorders, akinetic-rigid variant of Huntington disease, ocular albinism, alcohol abuse, alcohol sensitivity, alcohol use disorder, alcohol withdrawal seizures, alcohol withdrawal-induced major motor seizure, alcoholic intoxication, chronic alcoholic intoxication, Allan-Herndon-Dudley syndrome (ahds), allodynia, alloxan diabetes, alternating hemiplegia of childhood, early onset Alzheimer disease, late onset Alzheimer disease, Alzheimer's disease, focal onset Alzheimer's disease, amelogenesis imperfecta, amelogenesis imperfecta hypomaturation type, hypomaturation type iia5 amelogenesis imperfecta, type iii amelogenesis imperfecta, inborn errors amino acid metabolism, inherited disorders amino acid metabolism, amphetamine abuse, amphetamine addiction, amphetamine-related disorders, amyotrophic lateral sclerosis, amyotrophic lateral sclerosis with dementia, guam form amyotrophic lateral sclerosis, anaplastic carcinoma, anasarca, anemia, hemolytic anemia, acquired hemolytic anemia, idiopathic acquired hemolytic anemia, hereditary spherocytic hemolytic anemia, hypochromic microcytic anemia with iron overload, hypochromic microcytic anemia with iron overload 1, microangiopathic anemia, pyridoxine-refractory sideroblastic anemia 2, pyridoxine-refractory autosomal recessive sideroblastic anemia, anhedonia, animal mammary neoplasms, anxiety disorders, anxiety neurosis (finding), neurotic anxiety states, thoracic aortic aneurysm, aortic valve calcification, apnea, aprosodia, arterial tortuosity, arterial tortuosity syndrome, arthrogryposis, arthrogryposis, mental retardation and seizures, as if personality, asperger syndrome, ataxia, appendicular ataxia, motor ataxia, sensory ataxia, truncal ataxia, hereditary ataxias, atelosteogenesis type 2, atherogenesis, atherosclerosis, atonic absence seizures, atrophic, attention deficit disorder, attention deficit hyperactivity disorder, attention deficit hyperactivity disorder (ADHD), atypical ductal breast hyperplasia, aura, autism spectrum disorders, autistic disorder, autoimmune hemolytic anemia, autosomal dominant juvenile parkinson disease, autosomal dominant parkinsonism, autosomal recessive parkinsonism, autosomal recessive polycystic kidney disease, autosomal recessive primary microcephaly, autosomal recessive sideroblastic anemia, avoidant personality disorder, awakening epilepsy, nonobstructive azoospermia, Barrett epithelium, Barrett esophagus, Bartter disease, antenatal type 1 Bartter syndrome, basal cell carcinoma, idiopathic basal ganglia calcification 6, biotin-responsive basal ganglia disease, basal ganglia diseases, benign neoplasm, bilateral deafness, bile acid coa ligase deficiency and defective amidation, primary bile acid malabsorption, biliary cirrhosis, secondary biliary cirrhosis, bipolar disorder, bipolar i disorder, Birk-Landau-Perez syndrome, bladder neoplasm, blastocyst disintegration, body mass index quantitative trait locus 11, body mass index quantitative trait locus 4 (disorder), bone diseases, developmental bone diseases, brain diseases, brain dopamine-serotonin vesicular transport disease, brain hemorrhage, brain injuries, focal brain injuries, brain ischemia, brain lacerations, breast cancer, familial breast cancer, breast carcinoma, brittle diabetes, bronchioloalveolar adenocarcinoma, brown oculocutaneous albinism, Brown-Vialetto-van Laere syndrome, Brown-Vialetto-van Laere syndrome 2, brucellosis, pulmonary brucellosis, bulbar palsy, burn induced multiple organ injury, buruli ulcer, calcinosis, calcium pyrophosphate arthropathy, calcium pyrophosphate deposition disease, embryonal cancer, embryonal and mixed cancer, cannabis use, carbohydrate deficient glycoprotein syndrome type 2k, carcinoma, carcinoma of lung, spindle-cell carcinoma, carcinomatosis, cardiac arrhythmia, cardiac hypertrophy, cardiac ischaemic reperfusion, cardiomegaly, dilated cardiomyopathy, familial idiopathic cardiomyopathy, cardiovascular diseases, carditis, carnitine-acylcarnitine translocase deficiency, cataract, cataract 47, cataract and cardiomyopathy, juvenile cataract with microcornea and glucosuria, celiac disease, central hypothyroidism, cerebral injury, cerebral ischemia, cerebrovascular accident, cerebrovascular disorders, neuronal ceroid lipofuscinosis 7, parry type neuronal ceroid lipofuscinosis, ceruloplasmin deficiency, cervical cancer, Charcot-Marie-Tooth disease, type ia (disorder) Charcot-Marie-Tooth disease, type ib Charcot-Marie-Tooth disease, chemical and drug induced liver injury, chemically-induced liver toxicity, child development deviations, specific child development disorders, childhood acute lymphoblastic leukemia, childhood progressive bulbar palsy, childhood tic disorders, cholangiocarcinoma, choledochal cyst, type i choledochal cyst, type ii choledochal cyst, type iii choledochal cyst, type iv choledochal cyst, type v choledochal cyst, cholestasis, cholestasis in newborn, episodic choreoathetosis/spasticity, chromophobe renal cell carcinoma, chronic depression, chronic motor or vocal tic disorder, cirrhosis, type ii neonatal-onset citrullinemia, cleft palate, isolated cleft palate, cleft upper lip, cluttering, cocaine abuse, cocaine dependence, cocaine-induced mood disorder, cocaine-related disorders, cognition disorders, cognitive impairment, cold hemagglutinin disease, collecting duct carcinoma of the kidney, collodion fetus, colon cancer, colonic neoplasms, colorectal cancer, colorectal carcinoma, colorectal neoplasms, combined d-2- and 1-2-hydroxyglutaric aciduria, combined oxidative phosphorylation deficiency 18, combined oxidative phosphorylation deficiency 28, complete hearing loss, complex partial seizures, complex partial status epilepticus, preneoplastic condition, conduction disorder of the heart, congenital nonprogressive cone-rod synaptic disorder, congenital abnormality, congenital anemia, congenital aneurysm of ascending aorta, congenital cataracts, hearing loss and neurodegeneration, congenital chloride diarrhea, type 2c congenital disorder of glycosylation, type iif congenital disorder of glycosylation, type iim congenital disorder of glycosylation, type iin congenital disorder of glycosylation, congenital disorders of glycosylation, congenital glucose-galactose malabsorption, congenital heart defects, congenital hypothyroidism, congenital ichthyosis, postsynaptic congenital myasthenic syndromes, presynaptic congenital myasthenic syndromes, congenital myopathy (disorder), congenital nonbullous ichthyosiform erythroderma, sodium type (disorder) congenital secretory diarrhea, congestive heart failure, connective tissue diseases, constipation, constipation-predominant irritable bowel syndrome, contact dermatitis, contact hypersensitivity, conventional (clear cell) renal cell carcinoma, convulsions, convulsive seizures, corneal dystrophy and perceptive deafness, fuchs endothelial corneal dystrophy 4, corneal endothelial dystrophy 2, corneal opacity, corpus callosum agenesis neuronopathy, cortical dysplasia, coxsackievirus infections, craniofacial abnormalities, autosomal dominant craniometaphyseal dysplasia, cranioosteoarthropathy, craniosynostosis, x-linked creatine deficiency, crohn disease, Crohn's disease of large bowel, Crohn's disease of the ileum, stomatin-deficient cryohydrocytosis with mental retardation, seizures, cataracts, and massive hepatosplenomegaly, cystic fibrosis, cystic fibrosis modifier 1, cystinuria, type a cystinuria, type a-b cystinuria, type b cystinuria, cytopenia, de la chapelle dysplasia, de Toni-Debre-Fanconi syndrome, deaf mutism, deafness, acquired deafness, autosomal dominant 25 (disorder) deafness, autosomal dominant 72 deafness, autosomal recessive 4 deafness with enlarged vestibular aqueduct, autosomal recessive 61 deafness, deficiency of glutamate decarboxylase, degenerative polyarthritis, delirium, dementia, depressed mood, depression, bipolar depression, neurotic depression, postpartum depression, depressive disorder, treatment-resistant depressive disorder, depressive symptoms, depressive syndrome, allergic contact dermatitis, atopic dermatitis, developmental academic disorder, developmental disabilities, diabetes mellitus, experimental diabetes mellitus, insulin-dependent diabetes mellitus, ketosis-prone diabetes mellitus, non-insulin-dependent diabetes mellitus, diabetes mellitus type 2, autoimmune diabetes, diabetic cardiomyopathies, diabetic cataract, diarrhea, diastrophic dysplasia, dicarboxylicaminoaciduria, disease exacerbation, distal renal tubular acidosis, down syndrome, partial trisomy 21 down syndrome, drug abuse, drug dependence, drug habituation, drug toxicity, drug use disorders, drug withdrawal symptoms, drug-induced acute liver injury, drug-induced depressive state, drug-induced liver disease, ductal carcinoma, dysarthria, flaccid dysarthria, guttural dysarthria, mixed dysarthria, scanning dysarthria, spastic dysarthria, dysglossia, dyslalia, dysosteosclerosis, dysphoric mood, dysthymic disorder, dystonia, dystonia 18 (disorder), diurnal dystonia, limb dystonia, paroxysmal dystonia, ear diseases, early infantile epileptic encephalopathy with suppression bursts, early myoclonic encephalopathy, infantile eczema, edema, Ehlers-Danlos syndrome, type iv Ehlers-Danlos syndrome, elliptocytosis 4, hereditary elliptocytosis, embryo disintegration, embryo loss, embryonal neoplasm, embryonal rhabdomyosarcoma, encephaloclastic proliferative vasculopathy, encephalopathies, endogenous depression, endometrioma, endometriosis, enterovirus infections, epilepsy, anterior fronto-polar epilepsy, benign psychomotor childhood epilepsy, childhood absence epilepsy, cingulate epilepsy, cryptogenic epilepsy, frontal lobe epilepsy, susceptibility to idiopathic generalized epilepsy 12, susceptibility to idiopathic generalized epilepsy 14, lateral temporal epilepsy, opercular epilepsy, orbito-frontal epilepsy, pyridoxine-dependent epilepsy, supplementary motor epilepsy, temporal lobe epilepsy, epileptic drop attack, epileptic encephalopathy, early infantile epileptic encephalopathy 25, early infantile epileptic encephalopathy 34, early infantile epileptic encephalopathy 41, epileptic seizures, multiple epiphyseal dysplasia 4, type 6 (disorder) episodic ataxia, erythrocyte lactate transporter defect, erythrocytosis, esophageal neoplasms, etat marbre, riboflavin-responsive exercise intolerance, experimental hepatoma, external ophthalmoplegia, extrahepatic cholangiocarcinoma, extrapyramidal disorders, extravascular hemolysis, fahr's syndrome (disorder), familial Alzheimer disease (fad), familial apoceruloplasmin deficiency, familial dementia, familial juvenile parkinsonism, familial ménière disease, familial renal glucosuria, fanconi syndrome, fanconi-bickel syndrome, fatty liver, fazio-londe syndrome, fibrosis, liver fibrosis, flaccid muscle tone, floppy muscles, hereditary folate malabsorption, follicular adenoma, food allergy, foveal hypoplasia 2, foveal hypoplasia and anterior segment dysgenesis, fowler syndrome, fracture, spiral fracture, benign childhood frontal epilepsy, fuchs endothelial dystrophy, gastric cancer, gastrointestinal dysfunction, gastrointestinal neoplasms, gastrointestinal stromal sarcoma, gastrointestinal stromal tumors, generalized absence seizures, generalized pustular psoriasis, generalized seizures, germ cell cancer, germ cell tumor, gianotti-crosti syndrome, gitelman syndrome, glaucoma, glioma, glomerular filtration rate, glomerulosclerosis, glucose-6-phosphate transport defect, glut1 deficiency syndrome, autosomal recessive glut1 deficiency syndrome 1, glycine encephalopathy with normal serum glycine, glycogen storage disease ic, glycogen storage disease type id, renal glycosuria, goiter, gorlin chaudhry moss syndrome, gout, gout susceptibility 2, grand mal status epilepticus, gustatory seizure, harlequin fetus, hartnup disease, head and neck squamous cell carcinoma, hearing impairment, extreme hearing loss, heart decompensation, heart diseases, heart failure, right-sided heart failure, hematological disease, hemochromatosis, type 4 hemochromatosis, hemolysis (disorder), nonalcoholic fatty liver disease hepatic steatosis, hepatitis b, drug-induced hepatitis, toxic hepatitis, morris hepatoma, novikoff hepatoma, hepatomegaly, hereditary clear cell renal cell carcinoma, hereditary clubbing, hereditary diffuse gastric cancer, hereditary hemochromatosis, hereditary hyperexplexia, hereditary motor and sensory neuropathy type i, hereditary motor and sensory neuropathy with optic atrophy (disorder), hereditary motor and sensory-neuropathy type ii, hereditary spherocytosis, herpes encephalitis, herpetic acute necrotizing encephalitis, herpetic meningoencephalitis, hhh syndrome, histiocytosis, histiocytosis with joint contractures and sensorineural deafness, hiv coinfection, hiv infections, Huntington disease, late onset Huntington disease, hydrocephalus, hydrops fetalis, hyperactive behavior, hyperalgesia, primary hyperalgesia, secondary hyperalgesia, thermal hyperalgesia, hyperammonaemia, hyperammonemia, hypercalcemia, idiopathic hypercalcemia of infancy, infantile hypercalcemia, infantile hypercalcemia 1, infantile hypercalcemia 2, hypercalciuria, familial hypercholesterolemia, hyperekplexia 3, hyperglycinuria (disorder), hyperhomocysteinemia, familial hyperinsulinemic hypoglycemia 7, hyperinsulinism due to uncoupling protein 2 deficiency, generalized hyperkinesia, hypermanganesemia with dystonia 2, hypermanganesemia with dystonia polycythemia and cirrhosis, hyperoxaluria, hyperphosphatemia, hyperpigmentation, hypertensive disease, hypertrichosis, hypertrophic cardiomyopathy, primary autosomal dominant hypertrophic osteoarthropathy, primary autosomal recessive hypertrophic osteoarthropathy 2, hyperuricemia, hypogonadism, isolated hypogonadotropic hypogonadism, hypogonadotropic hypogonadism, global cerebral hypomyelination, hypophosphatemia, hereditary hypophosphatemic rickets with hypercalciuria, hypotension, hypothyroidism, hypotonia-cystinuria syndrome, renal hypouricemia 2, ichthyosis congenita ii, ichthyosis prematurity syndrome, congenital autosomal recessive ichthyosis 6, idiopathic autoimmune hemolytic anemia, idiopathic generalized epilepsy, idiopathic hypogonadotropic hypogonadism, iga glomerulonephritis, iieocolitis, iminoglycinuria, immersion related epilepsy, impaired glucose tolerance, impulse-ridden personality, inadequate personality, inborn errors of metabolism, infantile free sialic acid storage disease, infantile neuronal ceroid lipofuscinosis, infantile nystagmus, infantile sialic acid storage disease, inflammation, inflammatory bowel disease, inflammatory bowel diseases, inflammatory responses in cerebral ischemia and traumatic brain injury, influenza, acute brain injuries, insulin resistance, susceptibility to insulin resistance, insulin sensitivity, intellectual developmental disorder with neuropsychiatric features, intellectual disability, internal ophthalmoplegia, intestinal obstruction, intracranial hemorrhages, intracranial hypertension, intrahepatic cholangiocarcinoma, intravascular hemolysis, involutional depression, involutional paraphrenia, iron deficiency anemia, iron overload, irritable heart, jacksonian seizure, juvenile arthritis, juvenile Huntington disease, juvenile neuronal ceroid lipofuscinosis, juvenile psoriatic arthritis, juvenile-onset still disease, ketotic hypoglycemia, kidney calculi, kidney diseases, acute kidney failure, kidney injury, kidney neoplasm, kohlschutter tonz syndrome, kyphosis deformity of spine, lactic acidosis induced pulmonary vein arrhythmogenesis, lactic acidosis induced sepsis, late-infantile neuronal ceroid lipfuscinosis, learning disabilities, learning disorders, learning disturbance, left-sided heart failure, visceral leishmaniasis, lens opacities, lenticulostriate disorders, leprosy, leukemia, acute lymphocytic leukemia 12, leukodystrophy, libman-sacks disease, lichtenstein-knorr syndrome, lipoidosis, liver cancer, liver carcinoma, liver cirrhosis, experimental liver cirrhosis, liver diseases, liver dysfunction, liver neoplasms, experimental liver neoplasms, long sleeper syndrome, low tension glaucoma, lung cancer, lung neoplasms, systemic lupus erythematosus, lysinuric protein intolerance, macular dystrophy with central cone involvement, major depressive disorder, malabsorption syndrome, malaria, malformations of cortical development, malignant glioma, malignant mesothelioma, malignant neoplasm of breast, malignant neoplasm of esophagus, malignant neoplasm of kidney, malignant neoplasm of liver, malignant neoplasm of lung, malignant neoplasm of ovary, malignant neoplasm of pancreas, malignant neoplasm of prostate, malignant neoplasm of salivary gland, malignant neoplasm of stomach, malignant neoplasm of urinary bladder, malignant neoplasms, malignant tumor of colon, animal mammary carcinoma, mammary neoplasms, experimental mammary neoplasms, human mammary neoplasms, manganese poisoning, manic, manic disorder, marfan syndrome, marijuana abuse, mechanical allodynia, susceptibility to meconium ileus in cystic fibrosis, megacolon, melancholia, melanoma, semantic memory disorder, spatial memory disorder, memory disorders, memory impairment, memory loss, meningococcal meningitis of serogroup a, meningococcal meningitis of serogroup b, meningococcal meningitis of serogroup c, meningococcal meningitis of serogroup w-135, meningococcal meningitis of serogroup y, meningococcal meningitis, mental deficiency, mental depression, south african type mental retardation x-linked, autosomal recessive 48 mental retardation, autosomal recessive 7 mental retardation, psychosocial mental retardation, x-linked mental retardation, x-linked syndromic christianson type mental retardation, mesothelioma, metabolic acidosis, metabolic bone disorder, microangiopathic hemolytic anemia, microcalcification, microcephaly, primary autosomal recessive microcephaly 15, amish type (disorder) microcephaly, microlissencephaly, milk-alkali syndrome, minimal brain dysfunction, mitochondrial diseases, autosomal dominant mitochondrial dna depletion syndrome 12a (cardiomyopathic type) autosomal recessive mitochondrial dna depletion syndrome 12b (cardiomyopathic type), mitochondrial phosphate carrier deficiency, mitochondrial pyruvate carrier deficiency, mitral valve prolapse syndrome, mixed gliomas, monocarboxylate transporter 1 deficiency, mood disorders, motor tic disorders, movement disorders, multiple epiphyseal dysplasia, multiple sclerosis, acute fulminating multiple sclerosis, muscle hypotonia, muscle tone atonic, presynaptic congenital myasthenic syndrome 20, presynaptic congenital myasthenic syndrome 21, congenital myasthenic syndromes, congenital slow channel myasthenic syndromes, protection against mycobacterium tuberculosis, susceptibility to (finding) mycobacterium tuberculosis, myocardial failure, myocardial infraction, myocardial ischemia, myocardial reperfusion injury, myocarditis, myoclonic astatic epilepsy, myoclonic seizures, myoclonic-atonic epilepsy, myopathy, autosomal dominant myopia 24, na, n-acetylneuraminic acid storage disease, narcissistic personality disorder, necrosis, neonatal hypotonia, neoplasm invasiveness, neoplasm metastasis, neoplasms, embryonal and mixed neoplasms, germ cell and embryonal neoplasms, neoplastic cell transformation, nephrocalcinosis, calcium oxalate nephrolithiasis, hypophosphatemic nephrolithiasis-osteoporosis 1, nerve degeneration, nervous system disorder, neurocirculatory asthenia, neurodevelopmental disorders, neuronal ceroid-lipofuscinoses, neuropathic pain, distal hereditary motor type viia neuropathy, hereditary motor and sensory type vibneuropathy, nova scotian type niemann-pick disease, type c niemann-pick disease, type c1 niemann-pick disease, type d niemann-pick disease, congenital stationary night blindness, congenital stationary type 1 night blindness, congenital stationary type 1a night blindness, congenital stationary type 1b night blindness, congenital stationary type 2a night blindness, congenital stationary type 2b (disorder) night blindness, non-convulsive status epilepticus, non-epileptic convulsion, nonepileptic seizures, noninfiltrating intraductal carcinoma, nonorganic psychosis, non-small cell lung carcinoma, obesity, obsessive-compulsive disorder, oculocutaneous albinism type 2, oculocutaneous albinism type 6, type iv oculocutaneous albinism, odontome, olfactory seizure, ophthalmoparesis, ophthalmoplegia, optic atrophy, substance-induced organic mental disorders, orthostatic intolerance, primary hypertrophic osteoarthropathy, osteoarthrosis deformans, osteogenesis imperfecta, osteopenia, malaysian-melanesian-filipino type ovalocytosis, ovarian cancer, ovarian neoplasm, oxalosis, pain, burning pain, crushing pain, migratory pain, splitting pain, pancreatic cancer, pancreatic neoplasm, pancytopenia, papillary adenoma, papillary renal cell carcinoma, paranoia, parkinson disease, autosomal recessive juvenile parkinson disease 2, parkinsonian disorders, experimental parkinsonism, juvenile parkinsonism, infantile parkinsonism-dystonia, pediatric autoimmune disease, pendred's syndrome, peripheral diabetic neuropathy, peripheral neuropathy, peritoneal neoplasms, peritoneal surface malignancy, personality disorders, petit mal status, petty laxova wiedemann syndrome, physiological wound healing, pigmented basal cell carcinoma, pitt-rogers-danks syndrome, poisoning, polycystic kidney disease, polycythemia, disseminated superficial actinic type porokeratosis 8, disseminated superficial actinic porokeratosis, posterior column ataxia with retinitis pigmentosa, posterior fossa hemorrhage, post-traumatic tic disorder, precancerous conditions, precursor cell lymphoblastic leukemia lymphoma, prelingual deafness, prenatal injuries, prescription drug abuse, presenile dementia, primary biliary cirrhosis, primary hypogonadism, primary hypothyroidism, primary microcephaly, profound mental retardation, progressive bulbar palsy, autosomal dominant progressive external ophthalmoplegia with mitochondrial dna deletions 1, autosomal dominant progressive external ophthalmoplegia with mitochondrial dna deletions 2, prostate cancer, prostatic neoplasms, pseudoaphakia, pseudohyperkalemia cardiff, drug psychoses, substance-induced psychoses, involutional psychosis, psychotic disorders, pulmonary alveolar microlithiasis, pulmonary hypertension, radiating pain, ramsay hunt paralysis syndrome, recurrent depression, regional enteritis, renal carnitine transport defect, renal cell carcinoma, renal hypouricemia, renal tubular acidosis, distal renal tubular acidosis with hemolytic anemia (disorder), distal renal tubular acidosis with normal red cell morphology, proximal renal tubular acidosis with ocular abnormalities and mental retardation, type ii renal tubular acidosis, reperfusion injury, respiratory depression, adult respiratory distress syndrome, respiratory failure, respiratory hypersensitivity, respiratory insufficiency, retinal degeneration, retinitis pigmentosa, retinitis pigmentosa 68, rh deficiency syndrome, rhabdomyolysis, rhabdomyosarcoma, rhabdomyosarcoma 1, rheumatoid arthritis, rhinolalia, regulator type rh-null, riboflavin deficiency, rotor syndrome, roussy-levy syndrome (disorder), salivary gland neoplasms, sarcoidosis, sarcomatoid renal cell carcinoma, schizophrenia, catatonic schizophrenia, schneckenbecken dysplasia, schwartz-lelek syndrome, seasonal affective disorder, secondary hypothyroidism, seizures, auditory seizures, clonic seizures, focal seizures, sensory seizures, somatosensory seizures, senile paranoid dementia, sensorineural hearing loss (disorder), sensory hearing loss, severe congenital microcephaly, severe depression, severe major depression with psychotic features, hemorrhagic shock, short sleeper syndrome, finnish type (disorder) sialic acid storage disease, sialuria, sideroblastic anemia, simple partial status epilepticus, single seizure, sinus histiocytosis, variation in skin-hair-eye pigmentation 4, sleep disorders, sleep quality, sleep wake disorders, sleep-related neurogenic tachypnea, southeast asian ovalocytosis, autosomal dominant spastic paraplegia 42, autosomal dominant (disorder) spastic paraplegia 6, hereditary spastic paraplegia, thin corpus callosum spastic tetraplegia and progressive microcephaly, speech disorders, type 4 spherocytosis, autosomal recessive 3 spinocerebellar ataxia, Ehlers-Danlos syndrome-like spondylocheirodysplasia, squamous cell carcinoma, squamous cell carcinoma of esophagus, status epilepticus, alcohol withdrawal-induced status epilepticus, subclinical status epilepticus, steatohepatitis, stomach carcinoma, stomach neoplasms, stomatocytosis i, streptozotocin diabetes, stroke, substance abuse problem, substance dependence, substance use disorders, substance withdrawal syndrome, substance-related disorders, subwakefullness syndrome, sudden infant death syndrome, physical suffering, tactile allodynia, testicular microlithiasis, thiamine metabolism dysfunction syndrome 4 (bilateral striatal degeneration and progressive polyneuropathy type), thiamine responsive megaloblastic anemia syndrome, thinness, thyroid agenesis, thyroid carcinoma, thyroid dyshormonogenesis 1, thyroid gland follicular adenoma, thyroid hormone resistance syndrome, thyroid hypoplasia, thyroid neoplasm, tic disorder, vocal tic disorders, tissue fibrosis, susceptibility to (finding) tobacco addiction, tonic - clonic seizures, tonic seizures, tooth abnormalities, transient tic disorder, rubral tremor, meiotic nondisjunction trisomy 21, mitotic nondisjunction trisomy 21, tuberculosis, tumoral calcinosis, ulcerative colitis, uncinate epilepsy, undifferentiated carcinoma, unilateral hypotonia, unipolar depression, serum quantitative trait locus uric acid concentration 2, serum quantitative trait locus uric acid concentration 4, urolithiasis, vascular diseases, vascular hypertrophy, verbal fluency disorders, vertiginous seizure, visual seizure, withdrawal symptoms, wolf-hirschhorn syndrome, xerocytosis, x-linked csnb, epstein-barr virus infection and neoplasia x-linked immunodeficiency with magnesium defect and neonatal zinc deficiency due to low breast milk zinc.

Preferred diseases include abnormal coordination, abnormality of the cornea, absence seizures, ache, achondrogenesis of type ib (disorder), acidosis, acute lung injury, adenocarcinoma of lung (disorder), age related macular degeneration, Allan-Herndon-Dudley syndrome (ahds), alloxan diabetes, Alzheimer's disease, anasarca, hemolytic anemia, hemolytic acquired anemia, hereditary spherocytic hemolytic anemia, microangiopathic anemia, arthrogryposis, ataxia, appendicular ataxia, motor ataxia, sensory ataxia, truncal ataxia, hereditary ataxias, atelosteogenesis type 2, atherosclerosis, atonic absence seizures, attention deficit hyperactivity disorder (ADHD), atypical ductal breast hyperplasia, aura, autism spectrum disorders, autistic disorder, autosomal recessive polycystic kidney disease, awakening epilepsy, nonobstructive azoospermia, Barrett epithelium, Barrett esophagus, bilateral deafness, biliary cirrhosis, secondary biliary cirrhosis, Birk-Landau-Perez syndrome, developmental bone diseases, breast cancer, breast carcinoma, burn induced multiple organ injury, embryonal cancer, embryonal and mixed cancer, cardiac arrhythmia, cardiac ischaemic reperfusion, cardiomegaly, cataract, cataract 47, juvenile cataract with microcornea and glucosuria, celiac disease, cerebral injury, cervical cancer, child development deviations, specific child development disorders, choledochal cyst, choledochal cyst of type i, choledochal cyst of type ii, choledochal cyst of type iii, choledochal cyst of type iv, choledochal cyst of type v, cholestasis, episodic choreoathetosis/spasticity, chromophobe renal cell carcinoma, cognition disorders, cognitive impairment, collecting duct carcinoma of the kidney, colon cancer, colonic neoplasms, colorectal cancer, complete hearing loss, complex partial seizures, congenital anemia, hearing loss and neurodegeneration congenital cataracts, congenital chloride diarrhea, congenital hypothyroidism, sodium type (disorder) congenital secretory diarrhea, congestive heart failure, constipation, constipation-predominant irritable bowel syndrome, conventional (clear cell) renal cell carcinoma, convulsions, convulsive seizures, corneal dystrophy and perceptive deafness, fuchs endothelial corneal dystrophy 4, corneal endothelial dystrophy 2, corneal opacity, craniofacial abnormalities, stomatin-deficient cryohydrocytosis, cystic fibrosis, cystic fibrosis modifier 1, cytopenia, de la chapelle dysplasia, deaf mutism, deafness, acquired deafness, autosomal recessive deafness 4 with enlarged vestibular aqueduct, autosomal recessive 61 deafness, degenerative polyarthritis, dementia, depression, allergic contact dermatitis, developmental disabilities, diabetes mellitus, experimental diabetes mellitus, non-insulin-dependent diabetes mellitus, diabetic cataract, diarrhea, diastrophic dysplasia, distal renal tubular acidosis, ductal carcinoma, dystonia, dystonia 18 (disorder), ear diseases, edema, elliptocytosis 4, hereditary elliptocytosis, embryonal neoplasm, endometrioma, endometriosis, epilepsy, anterior fronto-polar epilepsy, childhood absence epilepsy 1, cingulate epilepsy, cryptogenic epilepsy, frontal lobe epilepsy, susceptibility to idiopathic generalized epilepsy 12, opercular epilepsy, orbito-frontal epilepsy, supplementary motor epilepsy, epileptic drop attack, epileptic encephalopathy, epileptic seizures, multiple epiphyseal dysplasia 4, erythrocyte lactate transporter defect, familial dementia, familial ménière disease, benign childhood frontal epilepsy, fuchs endothelial dystrophy, gastric cancer, gastrointestinal dysfunction, gastrointestinal neoplasms, generalized absence seizures, generalized seizures, germ cell cancer, germ cell tumor, glaucoma, glioma, glomerular filtration rate, glomerulosclerosis, glut1 deficiency syndrome, autosomal recessive glut1 deficiency syndrome 1, goiter, gustatory seizure, head and neck squamous cell carcinoma, hearing impairment, extreme hearing loss, heart decompensation, heart diseases, heart failure, right-sided heart failure, hematological disease, nonalcoholic fatty liver disease hepatic steatosis, hereditary spherocytosis, Huntington disease, hyperammonaemia, familial hyperinsulinemic hypoglycemia 7, hyperoxaluria, hyperphosphatemia, hypertrophic cardiomyopathy, hypotension, inborn errors of metabolism, inflammatory bowel disease, inflammatory responses in cerebral ischemia and traumatic brain injury, intellectual disability, intestinal obstruction, intracranial hypertension, jacksonian seizure, juvenile arthritis, juvenile psoriatic arthritis, juvenile-onset still disease, kidney calculi, kidney injury, kyphosis deformity of spine, lactic acidosis induced pulmonary vein arrhythmogenesis, lactic acidosis induced sepsis, left-sided heart failure, lens opacities, leukemia, leukodystrophy, lichtenstein-knorr syndrome, lipoidosis, liver cancer, liver carcinoma, experimental liver cirrhosis, liver neoplasms, lung cancer, malaria, malignant mesothelioma, malignant neoplasm of breast, malignant neoplasm of liver, malignant neoplasm of ovary, malignant neoplasm of prostate, malignant tumor of colon, mammary neoplasms, human mammary neoplasms, susceptibility to meconium ileus in cystic fibrosis, melanoma, mental deficiency, south african type mental retardation x-linked, psychosocial mental retardation, x-linked syndromic christianson type mental retardation, metabolic acidosis, microangiopathic hemolytic anemia, microcephaly, microlissencephaly, monocarboxylate transporter 1 deficiency, multiple epiphyseal dysplasia, multiple sclerosis, myocardial failure, myocardial infraction, myoclonic seizures, na, neoplasm metastasis, embryonal and mixed neoplasms, germ cell and embryonal neoplasms, neoplastic cell transformation, nephrocalcinosis, calcium oxalate nephrolithiasis, nervous system disorder, neuropathic pain, non-epileptic convulsion, nonepileptic seizures, noninfiltrating intraductal carcinoma, obesity, olfactory seizure, osteoarthrosis deformans, osteogenesis imperfecta, malaysian-melanesian-filipino type ovalocytosis, ovarian cancer, ovarian neoplasm, oxalosis, pain, burning pain, crushing pain, migratory pain, splitting pain, pancreatic cancer, papillary renal cell carcinoma, pediatric autoimmune disease, pendred's syndrome, peripheral diabetic neuropathy, peritoneal neoplasms, peritoneal surface malignancy, physiological wound healing, polycystic kidney disease, prelingual deafness, primary biliary cirrhosis, primary microcephaly, profound mental retardation, prostate cancer, prostatic neoplasms, pseudoaphakia, pseudohyperkalemia cardiff, radiating pain, renal cell carcinoma, renal tubular acidosis, distal renal tubular acidosis with hemolytic anemia, distal renal tubular acidosis with normal red cell morphology, proximal renal tubular acidosis with ocular abnormalities and mental retardation, renal tubular acidosis of type ii, reperfusion injury, sarcomatoid renal cell carcinoma, schizophrenia, seizures, auditory seizures, clonic seizures, focal seizures, sensory seizures, somatosensory seizures, senile paranoid dementia, sensorineural hearing loss (disorder), sensory hearing loss, severe congenital microcephaly, hemorrhagic shock, single seizure, sleep quality, southeast asian ovalocytosis, autosomal dominant spastic paraplegia 42, hereditary spastic paraplegia, type 4 spherocytosis, stomach carcinoma, streptozotocin diabetes, stroke, physical suffering, thyroid agenesis, thyroid hormone resistance syndrome, thyroid hypoplasia, tissue fibrosis, tonic - clonic seizures, tonic seizures, rubral tremor, ulcerative colitis, urolithiasis, vascular hypertrophy, vertiginous seizure, visual seizure and xerocytosis.

Even more preferred diseases include abnormal coordination, absence seizures, ache, acidosis, acute lung injury, adenocarcinoma of lung (disorder), alloxan diabetes, Alzheimer's disease, anasarca, arthrogryposis, ataxia, appendicular ataxia, motor ataxia, sensory ataxia, truncal ataxia, hereditary ataxias, atherosclerosis, atonic absence seizures, attention deficit hyperactivity disorder (ADHD), atypical ductal breast hyperplasia, autistic disorder, Barrett esophagus, Birk-Landau-Perez syndrome, breast cancer, burn induced multiple organ injury, embryonal cancer, embryonal and mixed cancer, cardiac arrhythmia, cardiac ischaemic reperfusion, cardiomegaly, cataract, celiac disease, cerebral injury, cervical cancer, child development deviations, specific child development disorders, chromophobe renal cell carcinoma, cognitive impairment, collecting duct carcinoma of the kidney, colon cancer, colonic neoplasms, complex partial seizures, congenital anemia, congenital cataracts, hearing loss, and neurodegeneration, sodium type (disorder) congenital secretory diarrhea, congestive heart failure, constipation, constipation-predominant irritable bowel syndrome, conventional (clear cell) renal cell carcinoma, convulsions, convulsive seizures, corneal opacity, cryohydrocytosis, stomatin-deficient, with mental retardation, seizures, cataracts, and massive hepatosplenomegaly, cystic fibrosis, cytopenia, degenerative polyarthritis, dementia, depression, allergic contact dermatitis, developmental disabilities, diabetes mellitus, diabetic cataract, diarrhea, distal renal tubular acidosis, ductal carcinoma, dystonia, edema, embryonal neoplasm, epilepsy, epileptic drop attack, epileptic encephalopathy, epileptic seizures, erythrocyte lactate transporter defect, familial dementia, gastric cancer, gastrointestinal dysfunction, generalized absence seizures, generalized seizures, germ cell cancer, germ cell tumor, glaucoma, glioma, glomerular filtration rate, glomerulosclerosis, glut1 deficiency syndrome, gustatory seizure, head and neck squamous cell carcinoma, hearing impairment, heart decompensation, heart failure, right-sided heart failure, nonalcoholic fatty liver disease hepatic steatosis, Huntington disease, hyperammonaemia, hyperinsulinemic hypoglycemia, familial, 7, hyperphosphatemia, hypertrophic cardiomyopathy, hypotension, inborn errors of metabolism, inflammatory bowel disease, inflammatory responses in cerebral ischemia and traumatic brain injury, intellectual disability, intestinal obstruction, jacksonian seizure, juvenile arthritis, juvenile psoriatic arthritis, juvenile-onset still disease, kidney injury, lactic acidosis induced pulmonary vein arrhythmogenesis, lactic acidosis induced sepsis, left-sided heart failure, lens opacities, leukemia, lichtenstein-knorr syndrome, lipoidosis, liver cancer, experimental liver cirrhosis, liver neoplasms, lung cancer, malignant mesothelioma, malignant neoplasm of breast, malignant neoplasm of liver, malignant neoplasm of ovary, malignant tumor of colon, mammary neoplasms, human mammary neoplasms, susceptibility to meconium ileus in cystic fibrosis, melanoma, mental deficiency, south african type mental retardation x-linked, psychosocial mental retardation, mental retardation, x-linked, syndromic, christianson type, metabolic acidosis, microcephaly, microlissencephaly, monocarboxylate transporter 1 deficiency, multiple sclerosis, myocardial failure, myocardial infraction, myoclonic seizures, neoplasm metastasis, embryonal and mixed neoplasms, germ cell and embryonal neoplasms, neoplastic cell transformation, nervous system disorder, neuropathic pain, non-epileptic convulsion, nonepileptic seizures, noninfiltrating intraductal carcinoma, obesity, olfactory seizure, osteoarthrosis deformans, ovarian cancer, ovarian neoplasm, pain, burning pain, crushing pain, migratory pain, splitting pain, pancreatic cancer, papillary renal cell carcinoma, pediatric autoimmune disease, peripheral diabetic neuropathy, peritoneal neoplasms, peritoneal surface malignancy, physiological wound healing, polycystic kidney disease, primary microcephaly, profound mental retardation, prostate cancer, pseudoaphakia, radiating pain, renal cell carcinoma, renal cell carcinoma, renal tubular acidosis, renal tubular acidosis, proximal, with ocular abnormalities and mental retardation, type ii renal tubular acidosis, reperfusion injury, sarcomatoid renal cell carcinoma, schizophrenia, seizures, auditory seizures, clonic seizures, focal seizures, sensory seizures, somatosensory seizures, senile paranoid dementia, severe congenital microcephaly, hemorrhagic shock, single seizure, sleep quality, autosomal dominant spastic paraplegia 42, hereditary spastic paraplegia, stomach carcinoma, streptozotocin diabetes, stroke, physical suffering, tissue fibrosis, tonic - clonic seizures, tonic seizures, rubral tremor, vascular hypertrophy, vertiginous seizure and visual seizure.

Diseases related to SLC9 proteins include in particular attention deficit hyperactivity disorder (ADHD), acute lung injury, Alzheimer's disease, atherosclerosis, autism, Barrett's oesophagus, brain cancer (e.g. glioma), breast cancer, burn induced multiple organ injury, cardiac arrhythmia, cardiac ischaemic reperfusion, cardiomegaly, celiac disease, cerebral injury, cervical cancer, cognitive impairment, colon cancer, constipation, constipation-predominant irritable bowel syndrome, cystic fibrosis, depression, diabetes, diabetic cataract, epilepsy, gastric cancer, gastrointestinal dysfunction, glomerular filtration rate, glomerulosclerosis, head and neck squamous cell carcinoma, nonalcoholic fatty liver disease hepatic steatosis, hyperammonaemia, hyperphosphatemia, inflammatory bowel disease, inflammatory responses in cerebral ischemia and traumatic brain injury, kidney injury, lactic acidosis induced pulmonary vein arrhythmogenesis, lactic acidosis induced sepsis, leukemia, liver cancer, melanoma, multiple sclerosis, myocardial infraction, neoplasm metastasis, neuropathic pain, ovarian cancer, pediatric autoimmune disease, peripheral diabetic neuropathy, physiological wound healing, polycystic kidney disease, prostate cancer, seizures, sleep quality, stomach carcinoma, stroke, tissue fibrosis, vascular hypertrophy, intellectual disability, ataxia, microcephaly, lung cancer, renal cell carcinoma and pancreatic cancer.

Preferred examples of diseases related to SLC9 proteins include Alzheimer's disease, atherosclerosis, autism, brain cancer (e.g. glioma), breast cancer, cardiac arrhythmia, cardiac ischaemic reperfusion, celiac disease, cervical cancer, colon cancer, diabetes, epilepsy, gastric cancer, gastrointestinal dysfunction, head and neck squamous cell carcinoma, nonalcoholic fatty liver disease hepatic steatosis, inflammatory bowel disease, intellectual disability, lactic acidosis, leukemia, liver cancer, lung cancer, melanoma, myocardial infraction, neoplasm metastasis, ovarian cancer, pancreatic cancer, prostate cancer, renal cell carcinoma, seizures, stomach carcinoma, stroke, tissue fibrosis and vascular hypertrophy.

More preferred examples of diseases related to SLC9 proteins include atherosclerosis, breast cancer, cardiac arrhythmia, cardiac ischaemic reperfusion, cervical cancer, colon cancer, gastric cancer, glioma, head and neck squamous cell carcinoma, leukemia, liver cancer, lung cancer, melanoma, myocardial infraction, neoplasm metastasis, ovarian cancer, pancreatic cancer, prostate cancer, renal cell carcinoma, stomach carcinoma, stroke, tissue fibrosis and vascular hypertrophy.

In context of the invention, the composition may comprise a pharmaceutically acceptable diluent, excipient or carrier. The term "pharmaceutically acceptable", as used in connection with compositions of the invention, refers to molecular entities and other ingredients of such compositions that are physiologically tolerable and do not typically produce untoward reactions when administered to a mammal (e.g., human). The term "pharmaceutically acceptable" may also mean approved by a regulatory agency of the Federal or a state government or listed in the U.S. Pharmacopeia or other generally recognized pharmacopeia for use in mammals, and more particularly in humans. A "pharmaceutically acceptable carrier" refers to an ingredient in a pharmaceutical composition or formulation, other than an active ingredient, which is nontoxic to a subject. A pharmaceutically acceptable carrier includes, but is not limited to, a buffer, excipient, stabilizer, or preservative. Such pharmaceutically acceptable carriers may be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by A.R. Gennaro, 20th Edition.

In one embodiment, the composition may be for use in treating or preventing cancer. The term "cancer" as used herein refers to any malignant tumor in the aforementioned tissue and cell type. Examples of the cancer may include a cancer which can be caused by an abnormal adherent cell, or a cancer which can be caused by an abnormal blood cell (e.g., leukemia, lymphoma, multiple myeloma). Specifically, examples of the cancer which can be caused by the abnormal adherent cell may include a lung cancer (e.g. squamous cell carcinoma, non-small cell carcinoma such as adenocarcinoma and large cell carcinoma, and small cell carcinoma), a gastrointestinal cancer (e.g., stomach cancer, small intestine cancer, large intestine cancer, rectal cancer), a pancreatic cancer, a renal cancer, a hepatic cancer, a thymic cancer, a spleen cancer, a thyroid cancer, an adrenal cancer, a prostate cancer, an urinary bladder cancer, an ovarian cancer, an uterus cancer (e.g., endometrial carcinoma, cervical cancer), a bone cancer, a skin cancer, a brain tumor, a sarcoma, a melanoma, a blastoma (e.g., neuroblastoma), an adenocarcinoma, a planocellular cancer, a solid cancer, an epithelial cancer, and a mesothelioma. Particularly, the cancer may be leukemia, particularly acute myeloid leukemia (AML) and B-cell acute lymphoblastic leukemia (B-ALL) a chronic leukemia, such as chronic myeloid leukemia; adenoid cystic carcinoma; osteosarcoma; ovarian cancer; Ewings sarcoma; lung adenocarcinoma and prostate cancer; lymphoma, neuroblastoma, gastrointestinal cancers, endometrial cancers, medulloblastoma, prostate cancers, esophagus cancer, breast cancer, thyroid cancer, meningioma, liver cancer, colorectal cancer, pancreatic cancer, chondrosarcoma, osteosarcoma, kidney cancer, particularly the cancer may be leukemia. In one embodiment, the composition may be for use in treating or preventing cancer, particularly leukemia such as chronic myeloid leukemia. In one embodiment, the present invention further may relate to a method treating cancer comprising administering the chemical compound or agent to a patient having cancer.

A "patient" or "individual" or "subject" is a mammal. Mammals include, but are not limited to, domesticated animals *(e.g.,* cows, sheep, cats, dogs, and horses), primates *(e.g.,* humans and non-human primates such as monkeys), rabbits, and rodents (e.g., mice and rats). In certain aspects, the patient, individual, or subject is a human. In one embodiment, the patient may be a "cancer patient," *i.e.* one who is suffering or at risk for suffering from one or more symptoms of cancer.

It will be understood that the specific dose level for any particular patient will depend upon a variety of factors including the activity of the specific compound employed, the age, body weight, general health, sex, diet, time of administration, route of administration, rate of excretion, drug combination and the causative mechanism and severity of the particular disease undergoing therapy.

The compounds of formula compounds of formulae (I) can be prepared by methods known in the field of synthetic chemistry and as described in the experimental examples of the present application.

The scope of the invention embraces the compounds provided herein, particularly the compounds of formula compounds of formulae (I), in any solvated form, including, e.g., solvates with water (i.e., as a hydrate) or solvates with organic solvents such as, e.g., methanol, ethanol or acetonitrile (i.e., as a methanolate, ethanolate or acetonitrilate), or in any crystalline form (i.e., as any polymorph), or in amorphous form. It is to be understood that such solvates of the compounds provided herein, particularly the compounds of formula (I), also include solvates of pharmaceutically acceptable salts of the corresponding compounds. Moreover, the compounds provided herein, particularly the compounds of formula compounds of formulae (I), may exist in the form of different isomers, in particular stereoisomers (including, e.g., geometric isomers (or cis/trans isomers), enantiomers and diastereomers) or tautomers. All such isomers of the compounds provided herein are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds provided herein.

The scope of the invention also embraces the compounds provided herein, particularly the compounds of formula compounds of formulae (I), in which one or more atoms are replaced by a specific isotope of the corresponding atom. For example, the invention encompasses compounds of formula compounds of formulae (I), in which one or more hydrogen atoms (or, e.g., all hydrogen atoms) are replaced by deuterium atoms (i.e., ²H; also referred to as "D"). Accordingly, the invention also embraces compounds of formula compounds of formulae (I) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (I) can be increased using deuteration techniques known in the art. For example, a compound of formula compounds of formulae (I) or a reactant or precursor to be used in the synthesis of the compound of formula compounds of formulae (I) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; or Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or ¹H hydrogen atoms in the compounds of formula (I) is preferred.

The present invention also embraces the compounds provided herein, particularly the compounds of formula compounds of formula (I), in which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers or imaging probes in positron emission tomography (PET). The invention thus includes (i) compounds of formula compounds of formulae (I), in which one or more fluorine atoms (or, e.g., all fluorine atoms) are replaced by ¹⁸F atoms, (ii) compounds of formula compounds of formulae (I), in which one or more carbon atoms (or, e.g., all carbon atoms) are replaced by ¹¹C atoms, (iii) compounds of formula compounds of formulae (I), in which one or more nitrogen atoms (or, e.g., all nitrogen atoms) are replaced by ¹³N atoms, (iv) compounds of formula compounds of formulae (I), in which one or more oxygen atoms (or, e.g., all oxygen atoms) are replaced by ¹⁵O atoms, (v) compounds of formula (I), in which one or more bromine atoms (or, e.g., all bromine atoms) are replaced by ⁷⁶Br atoms, (vi) compounds of formula compounds of formulae (I), in which one or more bromine atoms (or, e.g., all bromine atoms) are replaced by ⁷⁷Br atoms, (vii) compounds of formula compounds of formulae (I), in which one or more iodine atoms (or, e.g., all iodine atoms) are replaced by ¹²⁰I atoms, and (viii) compounds of formula compounds of formulae (I), in which one or more iodine atoms (or, e.g., all iodine atoms) are replaced by ¹²⁴I atoms. In general, it is preferred that none of the atoms in the compounds of formula compounds of formulae (I) are replaced by specific isotopes.

The term "hydrocarbon group" refers to a group consisting of carbon atoms and hydrogen atoms.

The term "alicyclic" is used in connection with cyclic groups and denotes that the corresponding cyclic group is non-aromatic.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₅ alkyl" denotes an alkyl group having 1 to 5 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₅ alkenyl" denotes an alkenyl group having 2 to 5 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more carbon-to-carbon double bonds. The term "C₂₋₅ alkynyl" denotes an alkynyl group having 2 to 5 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₅ alkylene" denotes an alkylene group having 1 to 5 carbon atoms, and the term "C₀₋₃ alkylene" indicates that a covalent bond (corresponding to the option "C₀ alkylene") or a C₁₋₃ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "alkoxy" refers to an -O-alkyl group, wherein the alkyl moiety comprised in this group is as defined above.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

As used herein, the term "cyclyl" refers to a carbocyclyl or a heterocyclyl, as defined herein above.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, the term "heteroaryl" particularly preferably refers to pyridinyl (e.g., 2-pyridyl, 3-pyridyl, or 4-pyridyl), imidazolyl, thiazolyl, 1H-tetrazolyl, 2H-tetrazolyl, thienyl (i.e., thiophenyl), or pyrimidinyl.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members. Moreover, unless defined otherwise, the term "cycloalkyl" even more preferably refers to cyclohexyl or cyclopropyl, and yet even more preferably refers to cyclohexyl.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, "heterocycloalkyl" even more preferably refers to tetrahydropyranyl, piperidinyl, piperazinyl, morpholinyl, pyrrolidinyl, or tetrahydrofuranyl.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₁ cycloalkenyl, and more preferably refers to a C₃₋₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-Cl), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂. A particularly preferred "haloalkyl" group is -CF₃.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

A skilled person will appreciate that the substituent groups comprised in the compounds of formula (I) may be attached to the remainder of the respective compound via a number of different positions of the corresponding specific substituent group. Unless defined otherwise, the preferred attachment positions for the various specific substituent groups are as illustrated in the examples.

The pharmaceutical compositions may comprise one or more solubility enhancers, such as, e.g., poly(ethylene glycol), including poly(ethylene glycol) having a molecular weight in the range of about 200 to about 5,000 Da (e.g., PEG 200, PEG 300, PEG 400, or PEG 600), ethylene glycol, propylene glycol, glycerol, a non-ionic surfactant, tyloxapol, polysorbate 80, macrogol-15-hydroxystearate (e.g., Kolliphor® HS 15, CAS 70142-34-6), a phospholipid, lecithin, dimyristoyl phosphatidylcholine, dipalmitoyl phosphatidylcholine, distearoyl phosphatidylcholine, a cyclodextrin, α-cyclodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, a carboxyalkyl thioether, hydroxypropyl methylcellulose, hydroxypropylcellulose, polyvinylpyrrolidone, a vinyl acetate copolymer, vinyl pyrrolidone, sodium lauryl sulfate, dioctyl sodium sulfosuccinate, or any combination thereof.

A proposed, yet non-limiting dose of the compounds according to the invention for oral administration to a human (of approximately 70 kg body weight) may be 0.05 to 8000 mg, preferably 0.1 mg to 4000 mg, of the active ingredient per unit dose. The unit dose may be administered, e.g., 1 to 3 times per day. The unit dose may also be administered 1 to 7 times per week, e.g., with not more than one administration per day. A further exemplary dose of the compounds of formula (I) for oral administration to a human is 50 to 200 mg/kg bodyweight/day, particularly 100 mg/kg/day. It will be appreciated that it may be necessary to make routine variations to the dosage depending on the age and weight of the patient/subject as well as the severity of the condition to be treated. The precise dose and also the route of administration will ultimately be at the discretion of the attendant physician or veterinarian.

The compounds provided herein, particularly the compound of formula (I), or a pharmaceutical composition comprising such a compound can be administered in monotherapy (e.g., without concomitantly administering any further therapeutic agents, or without concomitantly administering any further therapeutic agents against the same disease that is to be treated or prevented with the compound of formula (I)). However, the compound of formula (I) or a pharmaceutical composition comprising the compound of formula (I) can also be administered in combination with one or more further therapeutic agents. If the compound of formula (I) is used in combination with a second therapeutic agent active against the same disease or condition, the dose of each compound may differ from that when the corresponding compound is used alone, in particular, a lower dose of each compound may be used. The combination of the compound of formula (I) with one or more further therapeutic agents (such as, e.g., a BRD4 inhibitor, preferably a direct BRD4 inhibitor) may comprise the simultaneous/concomitant administration of the compound of formula (I) and the further therapeutic agent(s) (either in a single pharmaceutical formulation or in separate pharmaceutical formulations), or the sequential/separate administration of the compound of formula (I) and the further therapeutic agent(s). If administration is sequential, either the compound of formula (I) according to the invention or the one or more further therapeutic agents may be administered first. If administration is simultaneous, the one or more further therapeutic agents may be included in the same pharmaceutical formulation as the compound of formula (I), or they may be administered in one or more different (separate) pharmaceutical formulations.

Preferably, the one or more further therapeutic agents to be administered in combination with a compound of the present invention are anticancer drugs. The anticancer drug(s) to be administered in combination with a compound of formula (I) according to the invention may, e.g., be selected from: a tumor angiogenesis inhibitor (e.g., a protease inhibitor, an epidermal growth factor receptor kinase inhibitor, or a vascular endothelial growth factor receptor kinase inhibitor); a cytotoxic drug (e.g., an antimetabolite, such as purine and pyrimidine analog antimetabolites); an antimitotic agent (e.g., a microtubule stabilizing drug or an antimitotic alkaloid); a platinum coordination complex; an anti-tumor antibiotic; an alkylating agent (e.g., a nitrogen mustard or a nitrosourea); an endocrine agent (e.g., an adrenocorticosteroid, an androgen, an anti-androgen, an estrogen, an anti-estrogen, an aromatase inhibitor, a gonadotropin-releasing hormone agonist, or a somatostatin analog); or a compound that targets an enzyme or receptor that is overexpressed and/or otherwise involved in a specific metabolic pathway that is misregulated in the tumor cell (e.g., ATP and GTP phosphodiesterase inhibitors, histone deacetylase inhibitors, protein kinase inhibitors (such as serine, threonine and tyrosine kinase inhibitors, e.g., Abelson protein tyrosine kinase inhibitors) and the various growth factors, their receptors and corresponding kinase inhibitors (such as epidermal growth factor receptor kinase inhibitors, vascular endothelial growth factor receptor kinase inhibitors, fibroblast growth factor inhibitors, insulin-like growth factor receptor inhibitors and platelet-derived growth factor receptor kinase inhibitors)); methionine, aminopeptidase inhibitors, proteasome inhibitors, cyclooxygenase inhibitors (e.g., cyclooxygenase-1 or cyclooxygenase-2 inhibitors), topoisomerase inhibitors (e.g., topoisomerase I inhibitors or topoisomerase II inhibitors), poly ADP ribose polymerase inhibitors (PARP inhibitors), and epidermal growth factor receptor (EGFR) inhibitors/antagonists.

An alkylating agent which can be used as an anticancer drug in combination with a compound of the present invention may be, for example, a nitrogen mustard (such as cyclophosphamide, mechlorethamine (chlormethine), uramustine, melphalan, chlorambucil, ifosfamide, bendamustine, or trofosfamide), a nitrosourea (such as carmustine, streptozocin, fotemustine, lomustine, nimustine, prednimustine, ranimustine, or semustine), an alkyl sulfonate (such as busulfan, mannosulfan, or treosulfan), an aziridine (such as hexamethylmelamine (altretamine), triethylenemelamine, ThioTEPA (N,N'N'-triethylenethiophosphoramide), carboquone, or triaziquone), a hydrazine (such as procarbazine), a triazene (such as dacarbazine), or an imidazotetrazine (such as temozolomide).

A platinum coordination complex which can be used as an anticancer drug in combination with a compound of the present invention may be, for example, cisplatin, carboplatin, nedaplatin, oxaliplatin, satraplatin, or triplatin tetranitrate.

A cytotoxic drug which can be used as an anticancer drug in combination with a compound of the present invention may be, for example, an antimetabolite, including folic acid analogue antimetabolites (such as aminopterin, methotrexate, pemetrexed, or raltitrexed), purine analogue antimetabolites (such as cladribine, clofarabine, fludarabine, 6-mercaptopurine (including its prodrug form azathioprine), pentostatin, or 6-thioguanine), and pyrimidine analogue antimetabolites (such as cytarabine, decitabine, 5-fluorouracil (including its prodrug forms capecitabine and tegafur), floxuridine, gemcitabine, enocitabine, or sapacitabine).

An antimitotic agent which can be used as an anticancer drug in combination with a compound of the present invention may be, for example, a taxane (such as docetaxel, larotaxel, ortataxel, paclitaxel/taxol, tesetaxel, or nab-paclitaxel (e.g., Abraxane®)), a Vinca alkaloid (such as vinblastine, vincristine, vinflunine, vindesine, or vinorelbine), an epothilone (such as epothilone A, epothilone B, epothilone C, epothilone D, epothilone E, or epothilone F) or an epothilone B analogue (such as ixabepilone/azaepothilone B).

An anti-tumor antibiotic which can be used as an anticancer drug in combination with a compound of the present invention may be, for example, an anthracycline (such as aclarubicin, daunorubicin, doxorubicin, epirubicin, idarubicin, amrubicin, pirarubicin, valrubicin, or zorubicin), an anthracenedione (such as mitoxantrone, or pixantrone) or an anti-tumor antibiotic isolated from Streptomyces (such as actinomycin (including actinomycin D), bleomycin, mitomycin (including mitomycin C), or plicamycin).

A tyrosine kinase inhibitor which can be used as an anticancer drug in combination with a compound of the present invention may be, for example, axitinib, bosutinib, cediranib, dasatinib, erlotinib, gefitinib, imatinib, lapatinib, lestaurtinib, nilotinib, semaxanib, sorafenib, sunitinib, axitinib, nintedanib, ponatinib, or vandetanib.

A topoisomerase inhibitor which can be used as an anticancer drug in combination with a compound of the present invention may be, for example, a topoisomerase I inhibitor (such as irinotecan, topotecan, camptothecin, belotecan, rubitecan, or lamellarin D) or a topoisomerase II inhibitor (such as amsacrine, etoposide, etoposide phosphate, teniposide, or doxorubicin).

A PARP inhibitor which can be used as an anticancer drug in combination with a compound of the present invention may be, for example, BMN-673, olaparib, rucaparib, veliparib, CEP 9722, MK 4827, BGB-290, or 3-aminobenzamide.

An EGFR inhibitor/antagonist which can be used as an anticancer drug in combination with a compound of the present invention may be, for example, gefitinib, erlotinib, lapatinib, afatinib, neratinib, ABT-414, dacomitinib, AV-412, PD 153035, vandetanib, PKI-166, pelitinib, canertinib, icotinib, poziotinib, BMS-690514, CUDC-101, AP26113, XL647, cetuximab, panitumumab, zalutumumab, nimotuzumab, or matuzumab.

Further anticancer drugs may also be used in combination with a compound of the present invention. The anticancer drugs may comprise biological or chemical molecules, like TNF-related apoptosis-inducing ligand (TRAIL), tamoxifen, amsacrine, bexarotene, estramustine, irofulven, trabectedin, cetuximab, panitumumab, tositumomab, alemtuzumab, bevacizumab, edrecolomab, gemtuzumab, alvocidib, seliciclib, aminolevulinic acid, methyl aminolevulinate, efaproxiral, porfimer sodium, talaporfin, temoporfin, verteporfin, alitretinoin, tretinoin, anagrelide, arsenic trioxide, atrasentan, bortezomib, carmofur, celecoxib, demecolcine, elesclomol, elsamitrucin, etoglucid, lonidamine, lucanthone, masoprocol, mitobronitol, mitoguazone, mitotane, oblimersen, omacetaxine, sitimagene, ceradenovec, tegafur, testolactone, tiazofurine, tipifarnib, vorinostat, or iniparib.

Also biological drugs, like antibodies, antibody fragments, antibody constructs (for example, single-chain constructs), and/or modified antibodies (like CDR-grafted antibodies, humanized antibodies, "full humanized" antibodies, etc.) directed against cancer or tumor markers/factors/cytokines involved in proliferative diseases can be employed in cotherapy approaches with the compounds of the invention. Examples of such biological molecules are anti-HER2 antibodies (e.g. trastuzumab, Herceptin®), anti-CD20 antibodies (e.g. Rituximab, Rituxan®, MabThera®, Reditux®), anti-CD19/CD3 constructs (see, e.g., EP1071752) and anti-TNF antibodies (see, e.g., Taylor PC. Antibody therapy for rheumatoid arthritis. Curr Opin Pharmacol. 2003. 3(3):323-328). Further antibodies, antibody fragments, antibody constructs and/or modified antibodies to be used in cotherapy approaches with the compounds of the invention can be found, e.g., in: Taylor PC. Curr Opin Pharmacol. 2003. 3(3):323-328; or Roxana A. Maedica. 2006. 1(1):63-65.

An anticancer drug which can be used in combination with a compound of the present invention may, in particular, be an immunooncology therapeutic (such as an antibody (e.g., a monoclonal antibody or a polyclonal antibody), an antibody fragment, an antibody construct (e.g., a single-chain construct), or a modified antibody (e.g., a CDR-grafted antibody, a humanized antibody, or a "full humanized" antibody) targeting any one of CTLA-4, PD-1/PD-L1, TIM3, LAG3, OX4, CSF1R, IDO, or CD40. Such immunooncology therapeutics include, e.g., an anti-CTLA-4 antibody (particularly an antagonistic or pathway-blocking anti-CTLA-4 antibody; e.g., ipilimumab or tremelimumab), an anti-PD-1 antibody (particularly an antagonistic or pathway-blocking anti-PD-1 antibody; e.g., nivolumab (BMS-936558), pembrolizumab (MK-3475), pidilizumab (CT-011), AMP-224, or APE02058), an anti-PD-L1 antibody (particularly a pathway-blocking anti-PD-L1 antibody; e.g., BMS-936559, MEDI4736, MPDL3280A (RG7446), MDX-1105, or MEDI6469), an anti-TIM3 antibody (particularly a pathway-blocking anti-TIM3 antibody), an anti-LAG3 antibody (particularly an antagonistic or pathway-blocking anti-LAG3 antibody; e.g., BMS-986016, IMP701, or IMP731), an anti-OX4 antibody (particularly an agonistic anti-OX4 antibody; e.g., MEDI0562), an anti-CSFIR antibody (particularly a pathway-blocking anti-CSFIR antibody; e.g., IMC-CS4 or RG7155), an anti-IDO antibody (particularly a pathway-blocking anti-IDO antibody), or an anti-CD40 antibody (particularly an agonistic anti-CD40 antibody; e.g., CP-870,893 or Chi Lob 7/4). Further immunooncology therapeutics are known in the art and are described, e.g., in: Kyi C et al., FEBS Lett, 2014, 588(2):368-76; Intlekofer AM et al., J Leukoc Biol, 2013, 94(1):25-39; Callahan MK et al., J Leukoc Biol, 2013, 94(1):41-53; Ngiow SF et al., Cancer Res, 2011, 71(21):6567-71; and Blattman JN et al., Science, 2004, 305(5681):200-5.

A BRD4 inhibitor (preferably a direct BRD4 inhibitor), such as CeMMEC2, may also be used as a further therapeutic agent in combination with the compound of formula (I).

The combinations referred to above may conveniently be presented for use in the form of a pharmaceutical formulation. The individual components of such combinations may be administered either sequentially or simultaneously/concomitantly in separate or combined pharmaceutical formulations by any convenient route. When administration is sequential, either the compound of the present invention (particularly the compound of formula (I) or a pharmaceutically acceptable salt, solvate or prodrug thereof) or the further therapeutic agent(s) may be administered first. When administration is simultaneous, the combination may be administered either in the same pharmaceutical composition or in different pharmaceutical compositions. When combined in the same formulation, it will be appreciated that the two or more compounds must be stable and compatible with each other and the other components of the formulation. When formulated separately, they may be provided in any convenient formulation.

The compounds provided herein, particularly the compounds of formula (I), can also be administered in combination with physical therapy, such as radiotherapy. Radiotherapy may commence before, after, or simultaneously with administration of the compounds of the invention. For example, radiotherapy may commence 1-10 minutes, 1-10 hours or 24-72 hours after administration of the compounds. Yet, these time frames are not to be construed as limiting. The subject is exposed to radiation, preferably gamma radiation, whereby the radiation may be provided in a single dose or in multiple doses that are administered over several hours, days and/or weeks. Gamma radiation may be delivered according to standard radiotherapeutic protocols using standard dosages and regimens.

The present invention thus relates to a compound of formula (I) or a pharmaceutically acceptable salt, solvate, or prodrug thereof, or a pharmaceutical composition comprising any of the aforementioned entities in combination with a pharmaceutically acceptable excipient, for use in the treatment or prevention of cancer, wherein the compound or the pharmaceutical composition is to be administered in combination with one or more anticancer drugs and/or in combination with radiotherapy.

Yet, the compounds of formula (I) can also be used in monotherapy, particularly in the monotherapeutic treatment or prevention of cancer (i.e., without administering any other anticancer agents until the treatment with the compound(s) of formula (I) is terminated). Accordingly, the invention also relates to a compound of formula (I) or a pharmaceutically acceptable salt, solvate, or prodrug thereof, or a pharmaceutical composition comprising any of the aforementioned entities in combination with a pharmaceutically acceptable excipient, for use in the monotherapeutic treatment or prevention of cancer.

The subject or patient to be treated in accordance with the present invention may be an animal (e.g., a non-human animal), a vertebrate animal, a mammal, a rodent (e.g., a guinea pig, a hamster, a rat, or a mouse), a canine (e.g., a dog), a feline (e.g., a cat), a porcine (e.g., a pig), an equine (e.g., a horse), a primate or a simian (e.g., a monkey or an ape, such as a marmoset, a baboon, a gorilla, a chimpanzee, an orangutan, or a gibbon), or a human. In accordance with the present invention, it is envisaged that animals are to be treated which are economically, agronomically or scientifically important. Scientifically important organisms include, but are not limited to, mice, rats, and rabbits. Lower organisms such as, e.g., fruit flies like *Drosophila melagonaster* and nematodes like *Caenorhabditis elegans* may also be used in scientific approaches. Non-limiting examples of agronomically important animals are sheep, cattle and pigs, while, for example, cats and dogs may be considered as economically important animals. Preferably, the subject/patient is a mammal. More preferably, the subject/patient is a human or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig). Most preferably, the subject/patient is a human.

It will be understood that the invention disclosed and defined herein extends to all alternative combinations of two or more of the individual features mentioned or evident from the text or drawings. All of these different combinations constitute various alternative aspects of the invention. All patents and publications referred to herein are incorporated by reference in their entirety.

In this specification, a number of documents including patent applications, scientific literature and manufacturers' manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

As used herein, unless explicitly indicated otherwise or contradicted by context, the terms "a", "an" and "the" are used interchangeably with "one or more" and "at least one". Thus, for example, a composition comprising "a" compound of formula compounds of formulae (I) can be interpreted as referring to a composition comprising "one or more" compounds of formula compounds of formulae (I).

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

Moreover, unless indicated otherwise, any reference to an industry standard, a pharmacopeia, or a manufacturer's manual refers to the corresponding latest version that was available at the priority date (i.e., at the earliest filing date) of the present specification.

The following Examples further illustrate the present invention but should not be construed to limit the scope thereof.

### EXAMPLES

It was assessed whether SLCs, despite their complex membrane-binding topology and heterogenous subcellular localization, were amenable to chemical degradation. First SLC-dTAG fusion proteins were ectopically expressed. Then SLC proteins expressed from their natural chromosomal locus, and therefore exposed to more physiological regulation, were tested whether they could be made degradable through dTAG knock-in. Finally, the development of a new chemical entity able to degrade an untagged endogenous SLC protein was assessed elucidating SLC functions.

### Materials and Methods

### Synthesis of d9A1-1 - d9A1-4

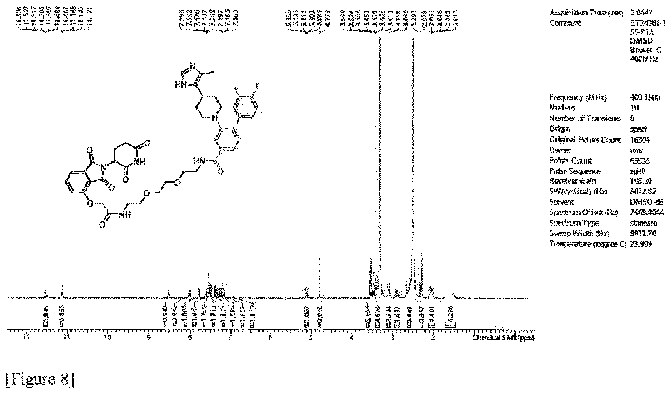

Intermediate 3 was synthesised by the reaction of 1-benzyloxycarbonyl-4-piperidone with 4-methyl-1H-imidazole using 2.5 eq KOtBu as base at 140°C in 4.3% yield (Figure 8). Intermediate 3 was subjected to hydrogenation in EtOH with Pd/C at 15 psi for 12 h, thereby both reducing the tetrahydropyridine ring and deprotecting the CBz group to piperidine 4 in quantitative yield (Figure 8). Compound 4 easily underwent nucleophilic aromatic substitution with ethyl 3-fluoro-4-nitrobenzoate in MeCN at 20°C using iPr₂NEt as base (Figure 8). The obtained nitroarene 7 was reduced to the aromatic amine 7G with iron and acetic acid in 63% yield (Figure 8). This amine was diazotized with 2 eq t-BuONO in MeCN and the diazo intermediate was reacted with CuBr₂ at 20°C for 12 h to obtain 28% of bromoarene 7H (Figure 8). Bromide 7H underwent Suzuki coupling with 4-fluoro-3-methylphenylboronic acid using 10 mol% Pd(dppf)Cl₂ as the catalyst, 2 eq Boc₂O for the *in situ* protection of the substrate, and 2 eq (Figure 8). K₂CO₃ as the base in dioxane with 10% H₂O under MW irradiation at 140°C for 1.5 h (Figure 8). The product of the Suzuki coupling, intermediate 7F, was obtained in 37% yield, the Boc group having undergone deprotection *in situ* (Figure 8). It was then hydrolysed with LiOH in THF / EtOH / H₂O to the carboxylic acid, d9A1 warhead. The compounds d9A1-1 to d9A1-4 were synthesized from the d9A1 warhead and the aminoalkyl substituted Cereblon ligands (synthesized as described in Zhang et al., Eur J Med Chem, 151, 304-314 (2018)) by amide couplings using HATU and iPr₂NEt in DMF (Figure 8).

### Cell lines and viruses

HEK293T, LS180 were obtained from ATCC (Manassas, VA, USA), HAP1 WT, HAP1 -/-SLC38A2 (HZGHC001975c003), HAP1 -/-SLC38A9 (HZGHC000777c011) were obtained from Horizon Genomics. Other cell lines utilized here: CAKI (a gift of Georg Winter's lab), SW620, SW480 (both a gift from Walter Berger's lab, Medical University Vienna), LOVO, HCT15 (both a gift from Chris Gasche), KBM7 (a gift from Thijn Brummelkamp's lab), KBM7 -/- CRBN was described in Mayor-Ruiz et al. Cells were cultured, according to the ATCC recommendation in DMEM, RPMI or IMDM medium (Gibco) supplemented with 10% (v/v) FBS and antibiotics (100 U/ml penicillin and 100 mg/ml streptomycin). Knock-in single cell clones of dTAG-HA SLC38A2 HAP1 cell line were generated as previously described. For starvation, culture media was replaced with media without amino acids or FBS.

### Plasmids and stable cell line generation

SLC1A5, SLC2A1, SLC2A3, SLC16A1, SLC38A1, SLC38A2 were obtained as gateway-compatible pENTR/pDONR vectors from the Harvard PlasmID Repository. SLC39A7 was obtained from Dharmacon, GE Healthcare. The following vectors were synthesized by Genescript: SLC4A4, SLC7A3, SLC38A9, SLC25A1, SLC9A1, SLC9A2, SLC9A3, SLC9A4, SLC9A5, SLC9A6, SLC9A7, SLC9A8, and SLC9B2. SLC30A9, SLC33A1, SLC35B2, SLC25A26, and MTCH2 were a gift from the RESOLUTE consortium. cDNAs were transferred into gateway-compatible lentiviral expression vectors: pLX305 dTAG vectors (Addgene #91797/8) or pRRL Strep-HA (described previously43, EF1a promotor driven expression) using LR recombination (ThermoFisher Scientific). For the generation of lentiviral stable overexpression cells, HEK293T cells were transfected with psPAX2 (Addgene #12260) and pMD2.G (Addgene #12259) and expression vectors using polyethylenimine (PEI). 12 hours post transfection medium was replaced with fresh medium. The medium, containing the virus, was harvested 48 hours later, filtered (0.45 µm), supplemented with 5µg/ml Polybrene (Hexadimethrine bromide, Sigma) and added to target cells. 48 hours after transduction the medium was supplemented with the respective selection antibiotics (puromycin, blasticidin), to derive stably expressing cell populations. dTAG SLC38A2 knock-in cell line was generated using the vector pCRIS-PITCHv2-dTAG-BSD (Addgene #91792) and pX330A, as previously described14,42, using the following primers: 
SLC38A2_5-PITCH (SEQ ID NO. 10):
CGCGTTACATAGCATCGTACGCGTACGTGTTTGGTAGCTTGAAGAAGGCCGAAAC
CATGGCCAAGCCTTTGTCTCAAGAAGAATCC
 
SLC38A2_3-PITCH (SEQ ID NO. 11):

CATCAGCATTCTAGAGCATCGTACGCGTACGTGTTTGGGGGAAATACTGAATCG

TCCCATCTCTGCTTTCTTCATAGATCCGCCGCCACCCGAC
SLC38A2_gRNA_S (SEQ ID NO. 12):
   CACCGAATACTGAATCGTCCCATTT
SLC38A2_gRNA_AS (SEQ ID NO. 13):
   AAACAAATGGGACGATTCAGTATTC

### Antibodies and Immunoblotting

For immunoblotting, whole cell extracts were prepared using RIPA lysis buffer (25mM Tris/HCl pH 7.6, 150mM NaCl, 1% NP-40, 1% sodium deoxycholate, 0.1% SDS, EDTA-free protease inhibitor (Roche) and phosphatase inhibitors 2+3 (Sigma- Aldrich)). Cell extracts were incubated for 15 min on ice in 50 µL RIPA buffer. Lysates were cleared by centrifugation (20,000 g, 10 min, 4°C). Protein extracts were quantified and normalized using the BCA assay (Thermo Scientific). Where indicated, cleared lzsates where treated with the enzyme PNGase (NEB) to deglycosylate the proteins. Lämmli Sample Buffer 4x was added to protein extract samples without boiling. Cell lysates were run on SDS-polyacrylamide gel in tris-glycine running buffer and transferred to nitrocellulose membranes Amersham Protran 0.45 µm (GE Healthcare), according to standard methods. The membranes were incubated with the antibodies indicated below and visualized with horseradish peroxidase-conjugated secondary antibodies (described below) using the ECL Western blotting system (Thermo Scientific). The following antibodies were used: GAPDH (Santa Cruz, sc-365062), Actin (Cyoskeleton Inc., #AAN01), HA (Covance, cat #MMS-101R), HA (Cell Signaling, #3724), HA7-HRP (Sigma, H6533), HA (Roche, #11867423001), SLC9A1 (Santa Cruz, sc-136239), SLC2A1 (Cell Signaling, #12939), SLC38A1 (Cell Signaling, #36057). Secondary antibody for imaging goat anti-Rat IgG Alexa Fluor 488 (ThermoFisher, A11006). The following secondary antibodies were used for immunoblotting: goat anti-mouse HRP (115-035-003, Jackson ImmunoResearch) and goat anti-rabbit HRP (111-035-003, Jackson ImmunoResearch).

### Immunofluorescence staining and imaging

For immunofluorescence detection of the expressed SLCs in HAP1 cells, cells were seeded onto poly-L-lysine hydrobromide (P6282, Sigma-Aldrich)-coated 96-well CellCarrier Ultra plates (PerkinElmer) or onto 13mm No. 1.5H cover glasses (PaulMarienfeld). Cells were incubated and thereafter fixed with 4% formaldehyde in PBS 1x. After fixation, cells were incubated in blocking buffer (0.3% Saponin (47036, Sigma- Aldrich), 10% FCS in PBS 1x) for 1 hour at room temperature (RT), rocking. Primary antibody staining was performed for 2 hours at RT in blocking solution and fluorophore conjugated secondary antibody staining was applied for 1 hour after 3 washes in blocking solution. Final washing was performed three times and counterstaining was done for 15 min at RT with DAPI (1:1000 in PBS 1x) for nuclei and HCS CellMask Deep Red Stain (1:20000 in PBS 1x). Cover glasses were mounted using ProLong Gold antifade reagent (Thermo Scientific) and imaged using a Zeiss LSM-780 confocal microscope. Imaging of CellCarrier Ultra plates was performed on an Opera Phenix High Content Screening System (Perkin Elmer).

### Automated image analysis

For the quantification of cellular HA-tag intensities, CellProfiler v3.1.8 was used. In brief, nuclei were identified from DAPI staining and cells as secondary objects from CellMask images. HA-tag intensities were quantified on a single cell level and consecutive plotting of the data was performed in R 3.4.4.

### Chemicals

MG132, Bortezomib, Pomalidomide were obtained from Selleckchem. MLN4924 was obtained from MedchemExpress. Bafilomycin was obtained from Enzo Life Sciences. dTAG13 was a generous gift from the Nathanael Gray laboratory, Dana Farber Cancer Institute. The d9A1 degrader series (warhead, d9A1-1 to d9A1-4) was synthesized by Wuxi AppTec. The synthesis route and NMR data are provided in Figure 8. All chemicals were dissolved in DMSO (Sigma-Aldrich) and utilized at the concertation described in respective figures.

### Results

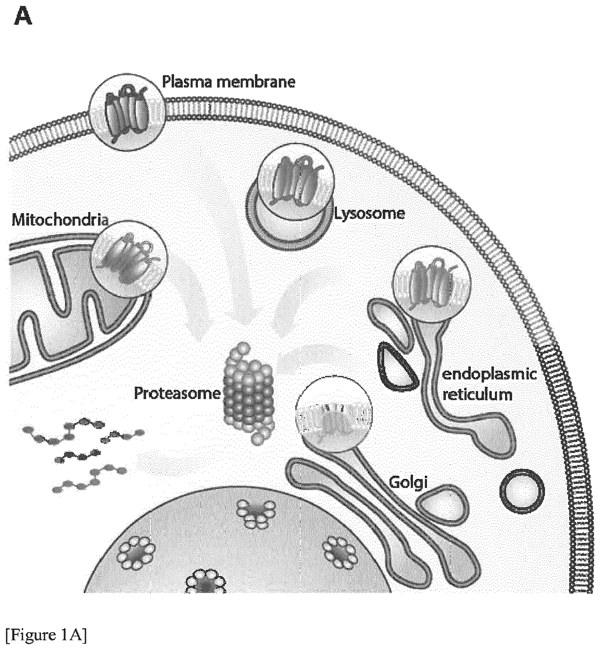

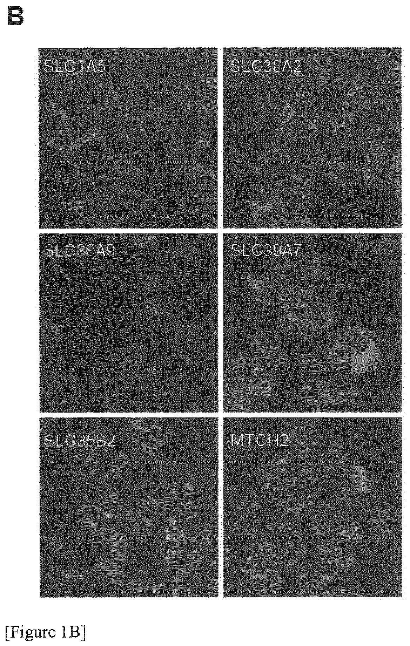

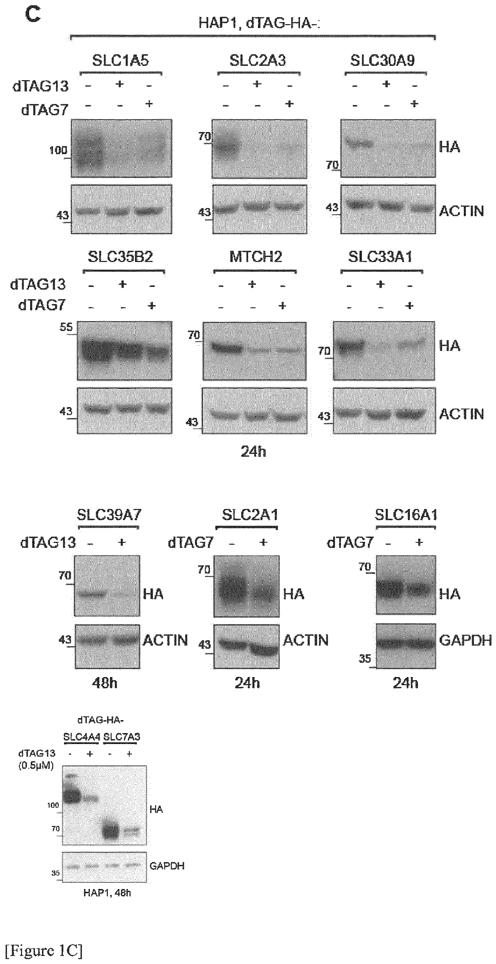

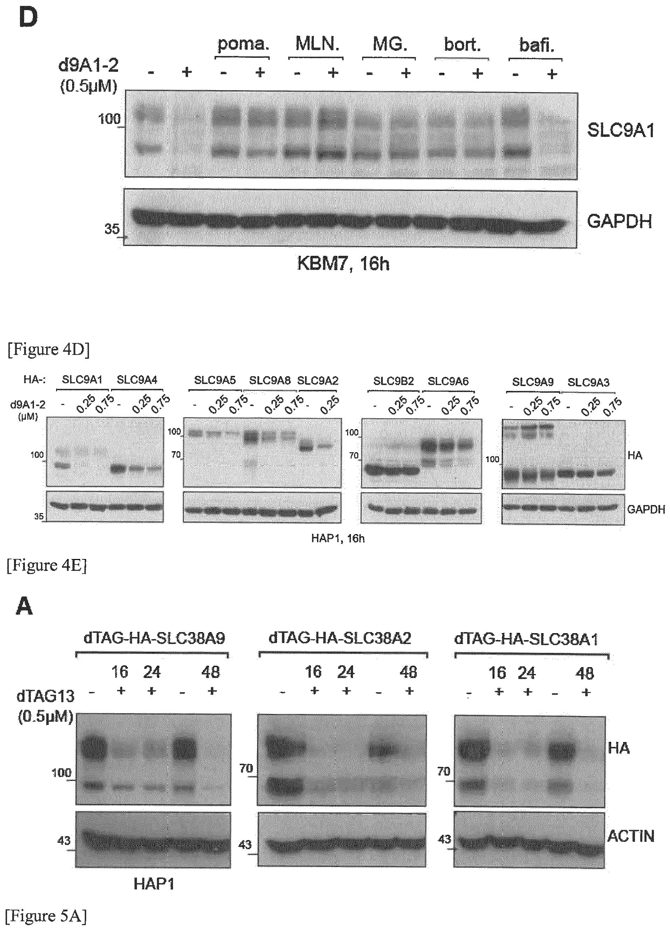

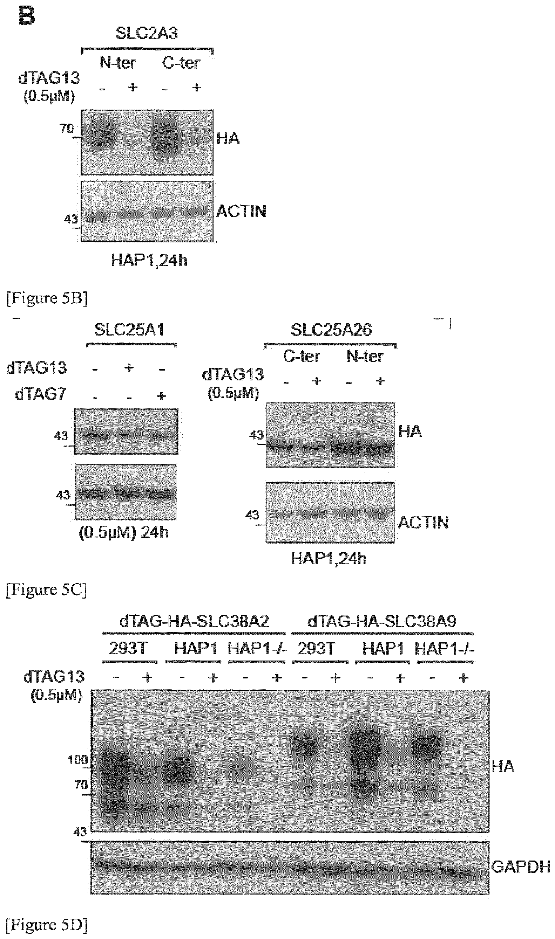

SLCs were ectopically expressed as fusions to a mutated FKBP domain, also known as the dTAG system. In brief, a dTAGed protein is amenable to degradation by specific chimeric degrader molecules (e.g. dTAG7/dTAG13) that simultaneously bind to the dTAG and the CRL4CRBN E3 ligase, inducing molecular proximity and ensuing degradation by the proteasome. Given that SLCs were expressed in all cellular compartments, the amenability of differentially located SLCs to targeted proteolysis was tested (Figure 1A). Each of the tested SLCs was stably expressed in HAP1 cells, and subcellular localization was assessed by immunofluorescence microscopy (Figure 1B). In detail, SLCs located at the cell surface (SLC1A5, SLC38A1, SLC2A1, SLC2A3, SLC16A1, SLC4A4, SLC7A3), cell surface and vesicles (SLC38A2), lysosome (SLC38A9), Golgi (SLC35B2, SLC33A1), mitochondria (SLC25A26, SLC25A1, and MTCH2) and endoplasmic reticulum (SLC39A7, SLC30A9) were assayed. Cellular treatment with a specific degrader molecule (dTAG7 or dTAG13) led to identification of SLCs expressed at cell surface, endoplasmic reticulum and lysosome that were amenable to targeted degradation (Figure 1C, 5A). The dTAG could be placed on either N- or C-terminus of the SLC, if both face the cytoplasm (Figure 5B), as exemplified by SLC2A3. The outer mitochondrial protein MTCH2 (SLC25A50) was amenable to degradation (Figure 1B), while the inner mitochondrial SLCs tested could not be degraded within the assayed timeframe (Figure 5C). While some SLCs, such as SLC38A2 or SLC2A3 could be completely degraded following treatment, other SLCs such as SLC35B2 or SLC39A7 were not degraded to completion (see Table 1).

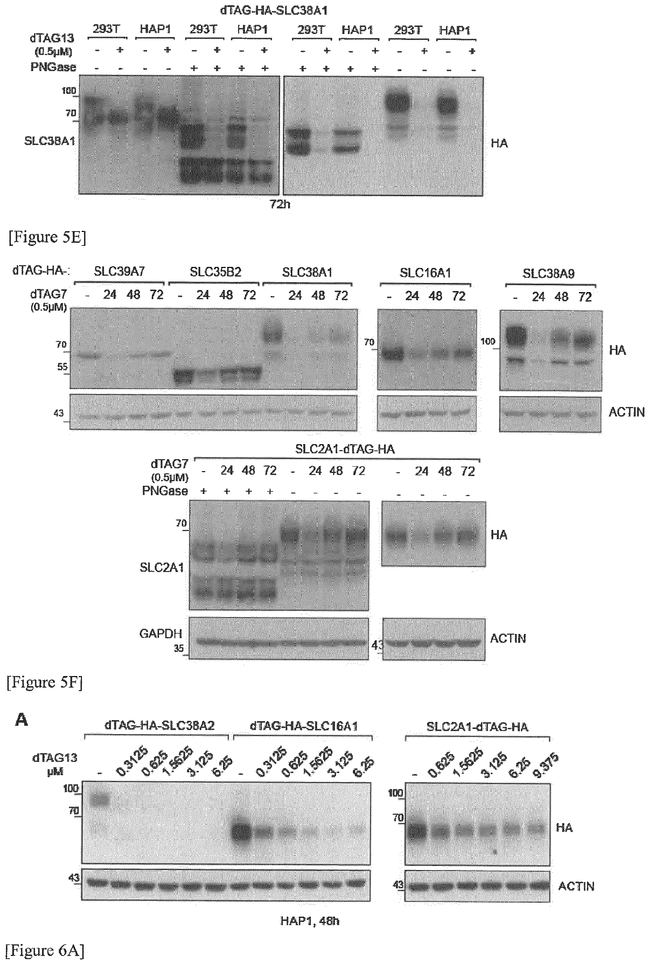

Table 1: summarizing the list of SLC proteins tested as the targets of degradation in Figure 1 and 5, as well as the number of trans-membrane (TM) segments predicted by PROTTER (Omasits U1, Ahrens CH, Müller S, Wollscheid B. Protter: interactive protein feature visualization and integration with experimental proteomic data. Bioinformatics. 2014 Mar 15;30(6):884-6, their subcellular localization and how amendable to degradation these proteins were. PM: plasma membrane; ER: endoplasmic reticulum; in.: inner; out.: outer.

**Table 1**

| **SLC** | **Predicted TMs** | **Cellular localization** | **Amenable to targeted degradation** |
|---|---|---|---|
| SLC1A5 | 8 | PM | +++ |
| SLC2A1 | 13 | PM | ++ |
| SLC2A3 | 12 | PM | +++ |
| SLC16A1 | 12 | PM | ++ |
| SLC38A1 | 11 | PM | +++ |
| SLC38A2 | 11 | PM/vesicles | +++ |
| SLC38A9 | 11 | Lyosome | +++ |
| SLC30A9 | 5 | ER | ++ |
| SLC39A7 | 6 | ER | ++ |
| SLC33A1 | 11 | ER/Golgi | ++ |
| SLC35B2 | 9 | Golgi | + |
| SLC25A1 | 6 | Mitochondria (in.) | - |
| SLC25A26 | 6 | Mitochondria (in.) | - |
| MTCH2 | 3 | Mitochondria (out.) | + |

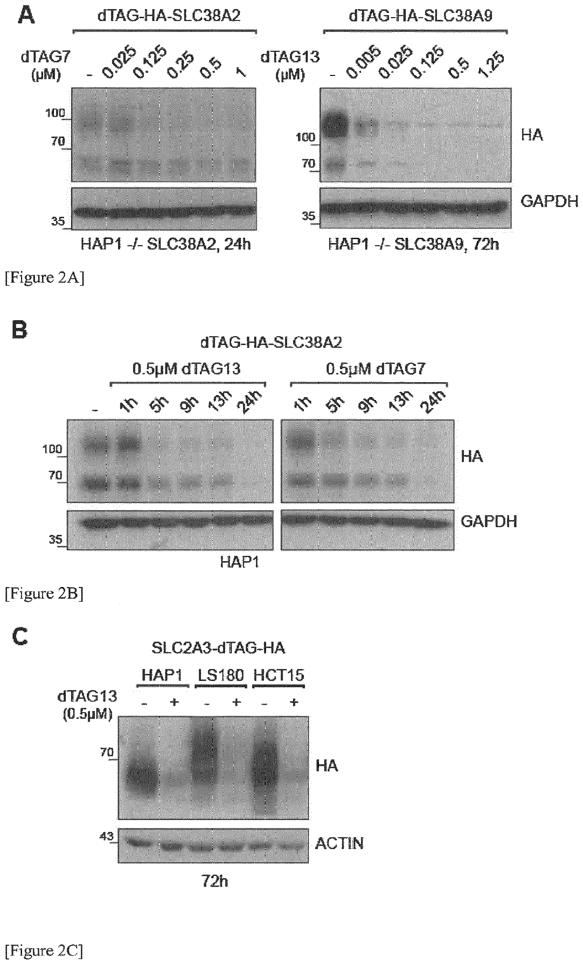

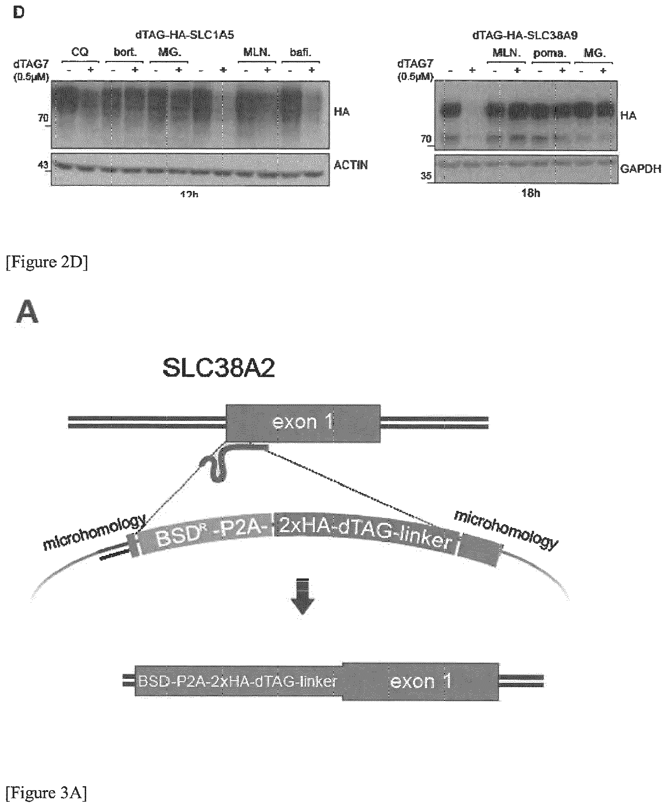

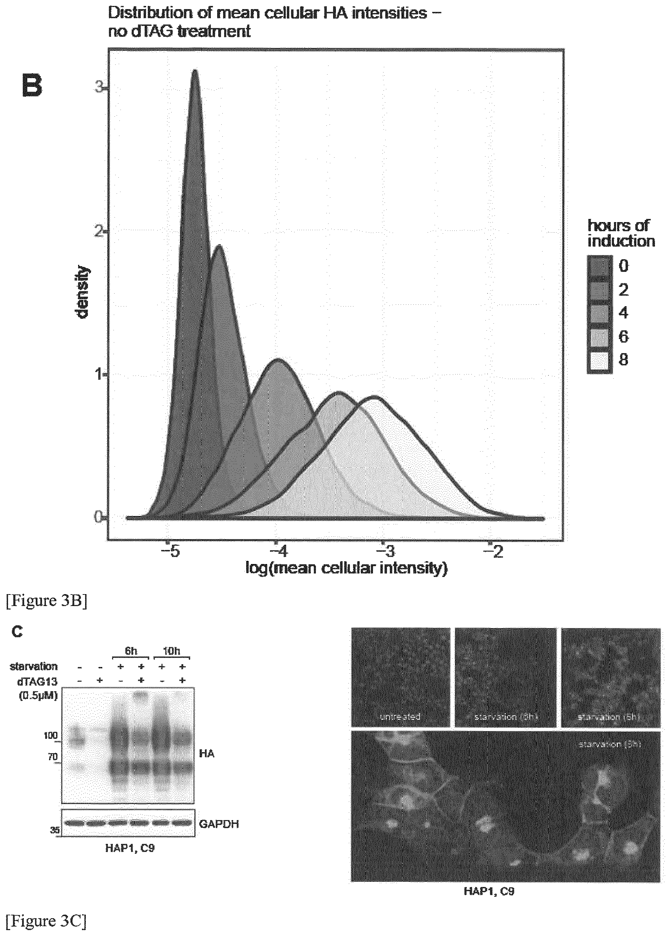

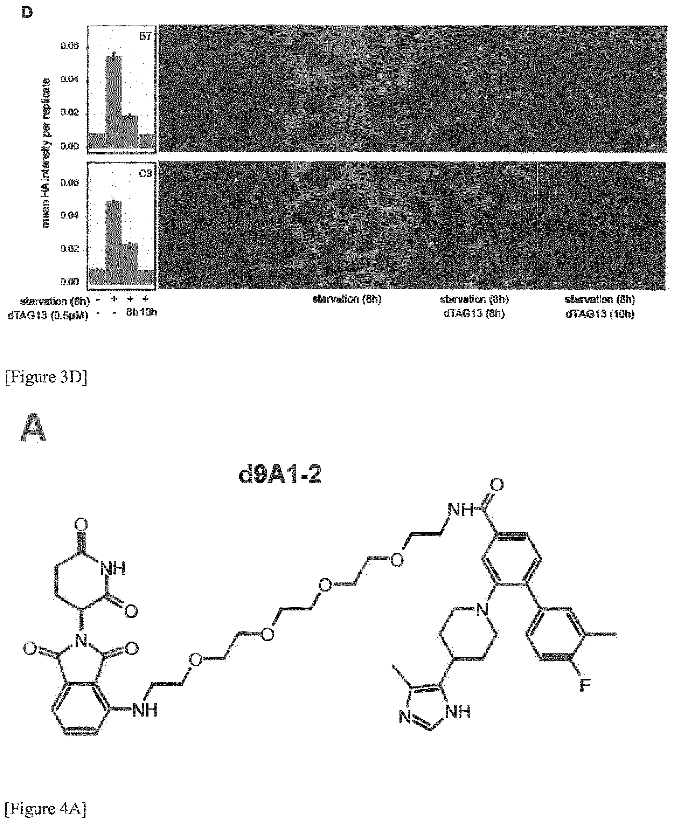

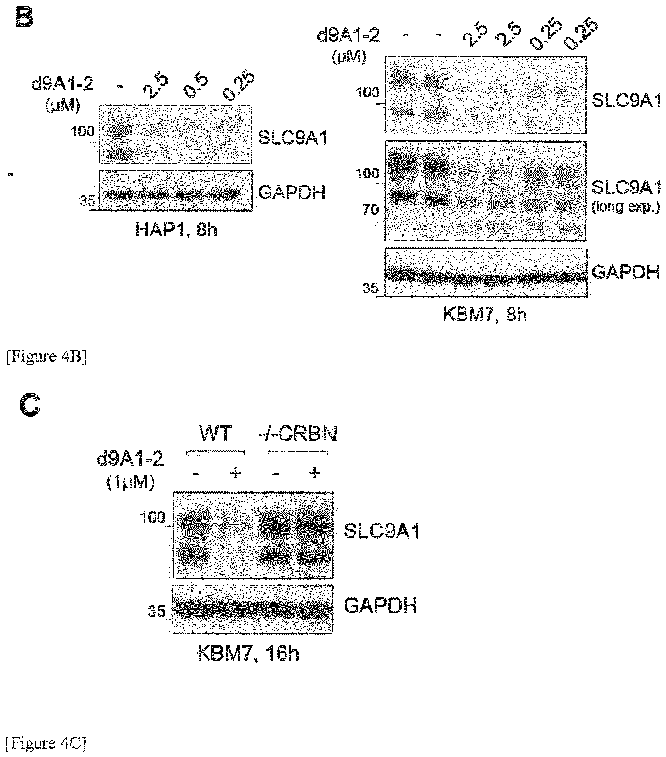

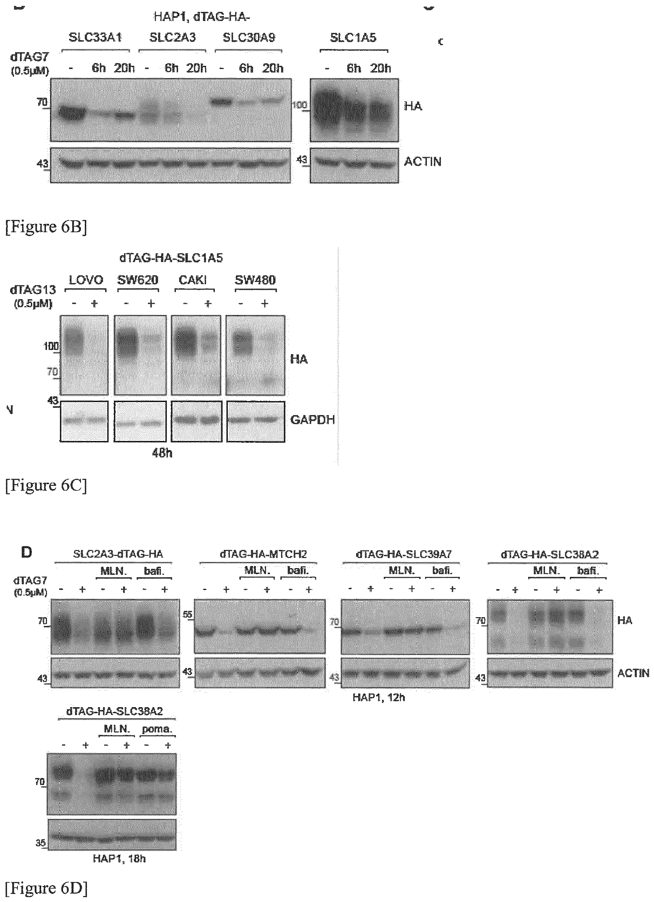

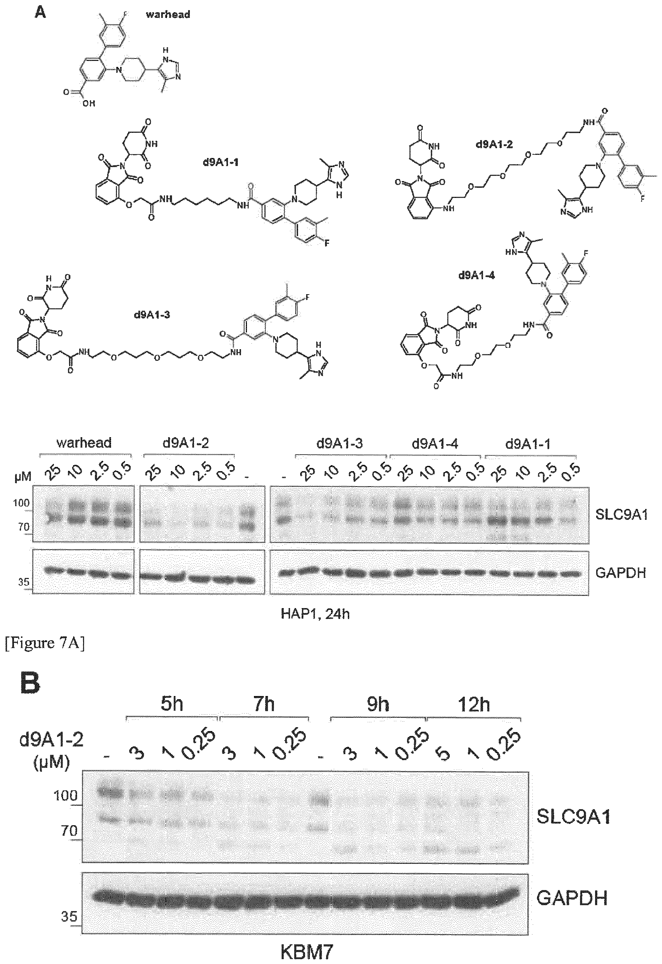

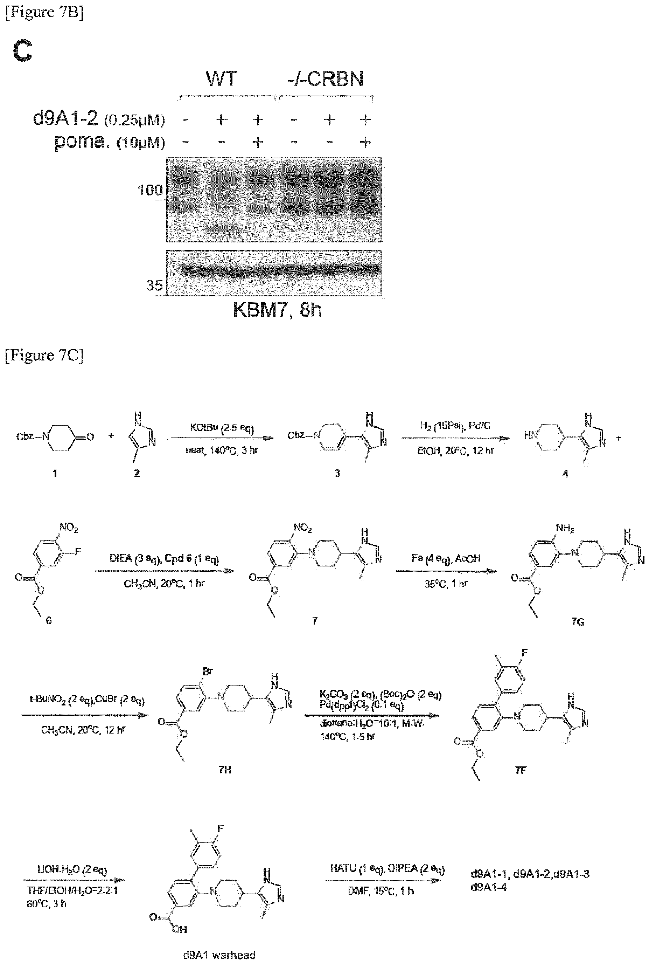

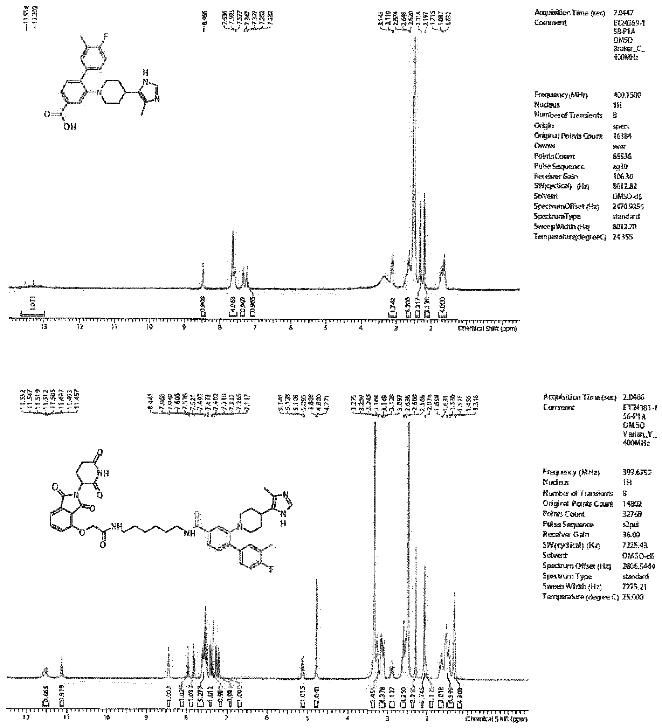

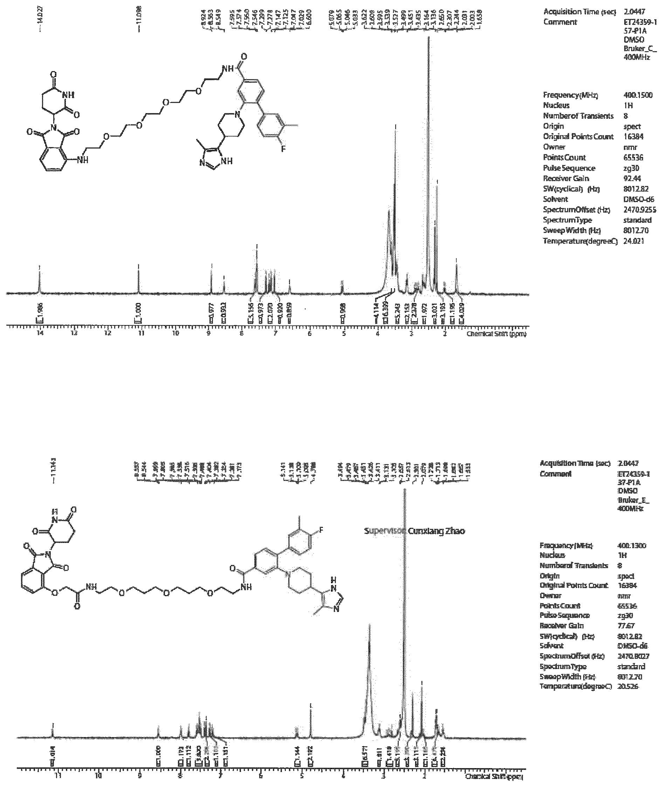

Complete degradation did not appear to correlate with the stable expression level of the dTAG protein nor with the expression of the endogenous protein (Figure 5D). Targeted degradation was also validated via protein-specific antibodies, which generally showed good consistency with detection via the HA epitope of the dTAG (Figure 5E and F). Of note, temporal control of targeted SLC degradation could be influenced by the choice of the respective heterobifunctional molecule: degradation with the PROTAC dTAG7 led to reversible degradation, while dTAG13 maintained the target degradation for at least 72h (Figure 5E and F). Collectively, these data confirm that an intracellular target engagement is sufficient to recruit the CRL^{CRBN} E3 ligase complex and prompt SLC degradation. Near-complete degradation could be achieved in the low nanomolar range for some SLCs, such as SLC38A2, although the dose of degrader required for maximal target degradation varied based on the studied SLC (Figure 2A, 6A). Targeted degradation of SLCs was typically initiated within 3-6 hours at least depending on the SLC (Figure 2B, 6B). Degradation of ectopically expressed dTAG-SLCs was not unique to the HAP1 cell line, but could also be achieved in other cancer cell lines such as HCT15, LS180 and others (Figure 2C, 6C). To validate that the observed SLC destabilization was dependent on an active CRL4^{CRBN} complex, chemical competition experiments were set up. dTAG7 mediated degradation was blocked by inhibiting cullin neddylation, and thus CRL activity, by co-treatment with the NAE1 inhibitor MLN4924 (Figure 2D, 6D). Similarly, saturating the CRBN binding site by treatment with excess concentration of pomalidomide prevented measurable SLC degradation. Finally, treatment with proteasome inhibitors (bortezomib or MG132) rescued SLCs degradation. Noteworthy, degradation appeared unaffected upon cotreatment with the lysosomal modulator bafilomycin A1 (Figure 2D). Thus, targeted degradation of SLCs appears to utilize the proteasome but not the lysosome machinery. Next, knock-in the dTAG domain at a genomic locus to demonstrate feasibility of chemical control of an endogenous SLC protein was performed. SLC38A2 was selected for endogenous tagging at the N-terminus (Figure 3A). SLC38A2 protein levels have been shown to be kept low under normal culture conditions and strongly induced following amino acid starvation (Nardi, F. et al. Proteasomal modulation of cellular SNAT2 (SLC38A2) abundance and function by unsaturated fatty acid availability. J. Biol. Chem. 290, 8173-8184 (2015)). Indeed, in the cell line model used, the endogenously tagged SLC38A2 protein was rapidly expressed within a few hours and could localize to cell membranes upon starvation (Figure 3B). Despite the strong induction of expression, the transporter remained amenable to targeted degradation (Figure 3C). It was found that even under this strong stimulus, SLC38A2 expression was completely ablated by 2 hours of pre-treatment with dTAG13, while co-treatment reduced SLC38A2 expression by more than 50% (Figure 3D). Altogether, the data for exogenous and endogenous expression of dTAGed SLCs indicated that multi-pass transmembrane proteins, such as SLCs, are within reach of the recruited proteolytic machinery and amenable to proteasome mediated targeted degradation. However, the use of genetically encoded degradation systems, such as the dTAG approach, limits its use to established and genetically tractable cell culture systems. To investigate whether SLCs could be degraded by chemically reprogramming the CRL4^{CRBN} E3 ligase complex, a directly-acting first in class, SLC degrader was designed. SLC9A1, also known as NHE1, is an electroneutral and reversible ion transporter that exchanges one Na+ ion for one H+ ion. It contributes to both cytoplasmic alkalization (pHi), and acidification of the microenvironment (Parks, S. K., Chiche, J. & Pouysségur, J. Disrupting proton dynamics and energy metabolism for cancer therapy. Nature Reviews Cancer 13, 611-623 (2013)). It has been previously shown to be involved in cancer and therefore is an attractive model target for our purpose (Stock, C. & Pedersen, S. F. Roles of pH and the Na+/H+ exchanger NHE1 in cancer: From cell biology and animal models to an emerging translational perspective, Seminars in Cancer Biology 43, 5-16 (2017)). A focused library of putative SLC9A1 degraders was synthesized by systematically varying linker length, composition and attachment chemistry to the pomalidomide-based CRBN binder (Figure 4A, 7A). Degradation of SLC9A1 was assessed in two cell lines, HAP1 and KBM7. The most potent degrader, denoted here as d9A1-2, led to degradation of endogenous SLC9A1 at sub-micromolar concentration within eight hours post treatment (Figure 4B, 7B). One additional degrader of the same series, denoted as d9A1-3, may also lead to degradation of the target, albeit at lower potency (Figure 7A), stressing the importance of linker design in generating an efficient degrader. As expected, genetic depletion of the E3 ligase adaptor CRBN ablated the degradation of SLC9A1 (Figure 4C, 7C). Again in line with a mechanism of controlled proteolysis, SLC9A1 destabilization was abrogated by blocking CRL activity via pharmacologic inhibition of NAE1 via MLN4924. Similarly, competition with excess amounts of pomalidomide blocked d9A1-2-induced degradation of SLC9A1 (Figure 4D, 7C). Treatment with proteasome inhibitors (bortezomib or MG132), but not bafilomycin A1, rescued SLCs degradation (Figure 4D). Next, the selectivity for the degradation of SLC9A1 over other SLC9 family members was assessed. In order to render this survey independent of endogenous transporter expression levels in HAP1 cells, tagged proteins were ectopically overexpressed, corresponding to nine members of the SLC9 family (A1,A2,A3,A4,A5,A6,A8,A9,B2). At 16 hours post treatment with d9A1-2 at 250 or 750 nM, SLC9A1 demonstrated the most efficient degradation. In addition, d9A1-2 also prompted degradation of the closely-related SLC9A2 and SLC9A4 (Figure 4E). Comparatively, abundance of the other assayed SLCs was not significantly affected at these experimental conditions. Thus, while not exclusively selective for SLC9A1, d9A1-2 features some level of intra-family selectivity. These experiments further attest to the degradability of SLCs by heterobifunctional degraders. Collectively, the data demonstrates the amenability of multi-pass transmembrane proteins to chemicallyinduced degradation and introduce the first SLC-directed PROTAC.

The Figures show
**Figure 1****: amenability of SLCs to targeted degradation**
   Exogenous expression of solute carriers (SLCs) tagged by FKBP12F36V ("dTAG") demonstrates amenability to targeted degradation in different cellular compartments:
   (A) Illustration representing SLC proteins from different subcellular locations (plasma membrane, Lysosome, mitochondria, Golgi, endoplasmic reticulum) were selected for testing their amenability to targeted degradation
   (B) Immunofluorescence (αHA) imaging of the indicated dTAG-HA SLCs was carried out to confirm subcellular localization
   (C)HAP1 cells expressing dTAG-HA SLC1A5, 2A3, 30A9, 35B2, MTCH2, and 33A1 were treated for 24 hours with 0.5 µM of dTAG7 or dTAG13, as indicated. HAP1 cells expressing dTAG-HA SLC2A1 and 16A1 were treated for 24 hrs with 0.5 µM of dTAG7. HAP1 cell lines expressing dTAG-HA SLC39A7, dTAG-HA SLC4A4, and SLC7A3-dTAG-HA were treated for 48 hrs with 0.5 µM of dTAG13.
**Figure 2****: characteristics of targeted degradation of SLCs by dTAG system**
   (A) A range of the degrader molecule concentrations was tested in a cell line reconstituted to express only dTAG-HA SLC38A2 or 38A9. Close to complete degradation can be achieved at low nanomolar range for these example SLCs.
   (B) A time course of dTAG driven SLC degradation. A HAP1 cell line expressing dTAGHA SLC38A2 was treated with 0.5 µM of dTAG7 or dTAG13, and samples were harvested at several time points. The glycosylated form (upper band) appeared to be degraded faster, within five hours, than the unglycosylated form.
   (C)dTAG-HA SLC2A3 was stably expressed in HAP1, LS180 and HCT15 cells. Following 72 hours of treatment with 0.5 µM dTAG13 SLC2A3 was completely degraded.
   (D)Chemical "rescue" of dTAG-driven degradation of SLCs. HAP1 cell lines expressing dTAG-HA SLC1A5 or dTAG-HA SLC38A9 were treated with 0.5 µM dTAG7 for 12 or 18 hours, respectively. These cells were also treated with chloroquine (CQ, 50µM), bortezomib (bort., 1µM), MG-132 (MG, 1µM), MLN4924 (MLN, 1µM), pomalidomide (poma., 10µM), or bafilomycin A1 (bafi., 2.5µM). SLC degradation was rescued by inhibiting CRL activity or the proteasome, but not by inhibiting the lysosome machinery.
**Figure 3****: dTAG knock-in are equally amenable to targeted degradation**
   (A) dTAG knock-in: SLC38A2 was tagged at N-terminus with dTAG-HA. Blasticidn was used as the selection marker. Single cell clones were derived and characterized as previously described. Two representative clones, B7 and C9 are presented here.
   (B) The expression of dTAG-HA SLC38A2 was induced by replacing the normal culture media with media deprived of amino acids and FBS. Expression of the endogenous SLC is induced rapidly and was monitored by immunofluorescence imaging and quantified by automated image analysis. Representative images are shown.
   (C)The effect of amino acid starvation on dTAG-HA SLC38A2 expression in HAP1 cells. Cells were treated with 0.5 µM dTAG13 and/or media lacking amino acids/FBS for either 6 or 10 hours, after which expression and degradation were monitored via western blot. Near complete degradation can be achieved by cotreatment.
   (D)Immunofluorescence imaging of two different HAP1 clones expressing dTAG-HA SLC38A2. Cells were incubated in media lacking amino acids/FBS for 8 hours and treated with 0.5 µM dTAG13 for either 8 or 10 hours. Expression of the endogenous SLC is induced rapidly and was monitored by immunofluorescence and quantified by automated image analysis. Representative images are presented. The two-hour pre-treatment with dTAG13 is sufficient to completely abrogate any SLC expression.
**Figure 4****: new SLC9A degrader series**
   (A) The chemical structure of the SLC degrader d9A1-2
   (B) HAP1 and KBM7 cells were treated with different concentrations of d9A1-2. Within 8 hours degradation of SLC9A1 was observed in both cell lines.
   (C)Both WT and CRBN knockout KBM7 cell lines were treated with 1µM d9A1-2 for 16 hours. SLC9A1 degradation is observed in WT but not CRBN knockout.
   (D) Chemical "rescue" of d9A1-2 driven degradation. WT KBM7 cell lines were treated with 0.5 µM d9A1-2 for 16 hours. These cells were also treated with bortezomib (bort. 0.25 µM), MG-132 (MG, 1 µM), MLN4924 (MLN,1 µM), pomalidomide (poma., 1 µM), or bafilomycin A1 (bafi., 10 µM). All molecules, apart from bafilomycin, could rescue SLC9A1 from degradation.
   (E) Selectivity of d9Al-2 was tested across HAP1 cell lines expressing Strep/HASLC9A1, 9A4, 9A5, 9A8, 9A2, 9B2, 9A6, 9A9, and 9A3. Cells were treated with varying concentrations of d9A1-2 for 16 hours, after which degradation of the exogenous SLCs was monitored.
**Figure 5****:**
   (A) HAP1 cells expressing dTAG-HA SLC38A9, 38A2, or 38A1 were treated with 0.5 µM dTAG13 for 16, 24 or 48 hours. Near-complete degradation of the target SLC was observed and maintained.
   (B) SLC2A3 was tagged on the N- or C-terminus with dTAG-HA. The two HAP1 cell lines were treated for 24 hours with dTAG13 and SLC2A3 was degraded in both.
   (C) HAP1 cell lines expressing dTAG-HA SLC25A1 and 25A26 did not demonstrate significant degradation after 24 hours of treatment with 0.5 µM dTAG.
   (D) Expression of dTAG-HA SLC38A2 or 38A9 in either 293T or HAP1 WT or a HAP1 clone in which the respective SLC was genetically ablated. The variance in expression levels indicated that degradation is achieved irrespective of the initial stable expression level, nor dependent on the presence of the endogenous SLC.
   (E) dTAG-HA SLC38A1, expressed in HAP1 or 293T, was degraded by treatment with dTAG13. Indicated protein extracts were de-glycosylated by treatment with PNGase. Western blotting with antibodies against the HA-tag and the total protein confirmed that the tagged protein as a whole is completely degraded.
   (F) HAP1 cells expressing dTAG-HA SLC39A7, 35B2, 16A1, 38A9 or 2A1 were treated with 0.5 µM of dTAG7 for 24, 48 or 72 hours. dTAG7 leads to reversible degradation of the target SLC, allowing accurate temporal control over protein levels. Western blotting with antibodies against the HA-tag and the total protein mirrored the observed pattern.
**Figure 6****:**
   (A) A range of the degrader molecule dTAG13 concentrations was tested in HAP1 cell lines expressing dTAG-HA SLC38A2, 16A1 and 2A1. Cells were treated for 48 hours. The concentration required to achieve complete or near to complete degradation depends on the SLC.
   (B) A time course of dTAG driven SLC degradation demonstrates initiation of degradation within 6 hours. HAP1 cell lines expressing dTAG-HA SLC33A1, 2A3, 30A9 or 1A5 were treated with 0.5 µM of dTAG7.
   (C) dTAG-HA SLC1A5 was expressed in LOVO, SW620, CAKI and SW480 cell lines. Cells were treated with 0.5 µM dTAG13 and degradation was observed after 48 hours.
   (D) Chemical "rescue" of dTAG driven degradation. HAP1 cell lines expressing dTAGHA SLC2A3, 39A7, 38A2, and MTCH2 were treated with 0.5 µM dTAG7 for 12 hours. A cell line expressing dTAG-HA SLC38A2 was treated with 0.5 µM dTAG7 for 18 hours. These cells were treated with MLN4924 (MLN, 1µM), pomalidomide (poma., 10µM), or bafilomycin A1 (bafi., 2.5µM) to monitor the inhibition of degradation.
**Figure 7****:**
   (A) Chemical structures of warhead and derived compunds of the d9A1 series.
   (B) HAP1 cells were treated with a dose curve of the warhead, d9A1-1, d9A1-2, d9A1-3, d9A1-4, for 24 hours. SLC9A1 degradation was most effective with d9A1-2 but could also be achieved with d9A1-3.
   (C) KBM7 cells were treated with varying concentrations of d9A1-2. Reduction of SLC9A1 expression was observed at 5, 7, 9 or 12 hours.
**Figure 8****:**
   Workflow for the synthesis of PROTAC d9A1 warhead and ensuing degraders, including the HNMR data.

## Claims

1. A compound having the following formula (I): 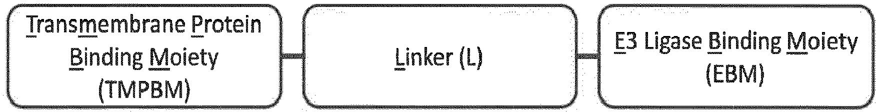 wherein
TMPBM is a moiety binding to a transmembrane protein,
L is a linker moiety; and
EBM is a moiety modifying the function of the E3 ligase and/or binding to at least one member of the E3 ligase complex.

2. The compound according to claim 1, wherein TMPBM is a moiety having the following partial formula (II) or a stereoisomer, tautomer, pharmaceutically acceptable salt, solvate or prodrug: 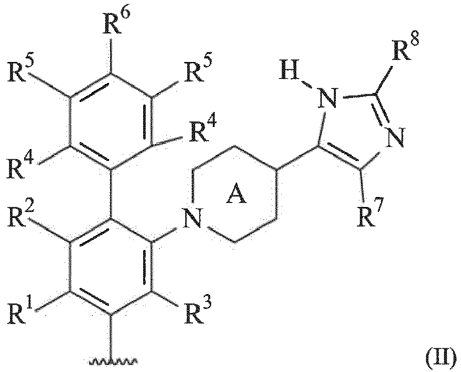 wherein
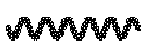 indicates the position of attachment of the partial formula (II) to the linker (L), R¹, R², R³, R⁴, R⁵, R⁶, R⁷ and R⁸ are each independently selected from -H, -halogen, - NO₂, -CN, -C₁₋₃ alkyl, -OH, -O-C₁₋₂ alkyl, NH₂, -NH(C₁₋₂ alkyl), -N(C₁₋₂ alkyl)₂, - CHO, -CO(C₁₋₂ alkyl), COOH, COO(C₁₋₂ alkyl), CONH₂, CONH(C₁₋₂ alkyl), and CON(-C₁₋₂ alkyl)₂, wherein each C₁₋₂ alkyl is optionally substituted with one or more F, and Ring A is optionally substituted with one or more F.

3. The compound according to claim 2, wherein in the moiety of partial formula (II) the following definitions apply:
R¹, R², R³ and each R⁴ are each hydrogen,
R⁷ is methyl,
R⁸ is H or NH₂,
and Ring A is not substituted with any F.

4. The compound according to claim 2 or 3, wherein in the moiety of partial formula (II) the following definitions apply:
one R⁵ is selected from H, F, Cl, Me, CF₃, CF₂H and CH₂F,
the other R⁵ is H, and
R⁶ is F or Cl.

5. The compound according to any one of claims 2 to 4, wherein the moiety of partial formula (II) has the following formula (IIa):

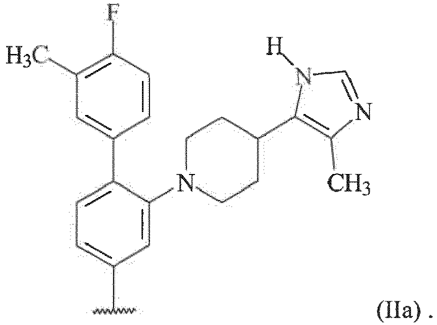

6. The compound according to any one of claims 1 to 5, wherein L is selected from C₁₄₋₂₄ alkylene, C₁₄₋₂₄ alkenylene, and C₁₄₋₂₄ alkynylene, wherein said alkylene, said alkenylene and said alkynylene are each optionally substituted with one or more groups independently selected from halogen, C₁₋₅ haloalkyl, -O(C₁₋₅ haloalkyl), -CN, -OR²¹, -NR²¹R²¹, -NR²¹OR²¹, -COR²¹, -COOR²¹, -OCOR²¹, -CONR²¹R²¹, -NR²¹COR²¹, -NR²¹COOR²¹, -OCONR²¹R²¹, -SR²¹, -SOR²¹, -SO₂R²¹, -SO₂NR²¹R²¹, -NR²¹SO₂R²¹, -SO₃R²¹, and -NO₂, and further wherein one or more -CH₂- units comprised in said alkylene, said alkenylene or said alkynylene are each optionally replaced by a group independently selected from -O-, -NR²¹-, -CO-, -S-, -SO-, and -SO₂-;
each R²¹ is independently selected from hydrogen, C₁₋₅ alkyl, C₂₋₅ alkenyl, C₂₋₅ alkynyl, carbocyclyl, and heterocyclyl, wherein said alkyl, said alkenyl and said alkynyl are each optionally substituted with one or more groups R^{Alk}, and further wherein said carbocyclyl and said heterocyclyl are each optionally substituted with one or more groups R^{Cyc};
any two R²¹ are optionally linked to form a ring;
each R^{Alk} is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl);
each R^{Cyc} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl).

7. The compound of formula (I) according to any one of the preceding claims, wherein EBM is a moiety binding to at least one member of the E3 ligase complex with a Kd of 10 µM or less.

8. The compound of formula (I) according to any one of the preceding claims, wherein EBM is a moiety of partial formula (III): 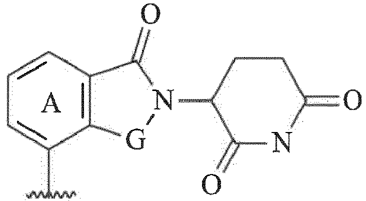 wherein
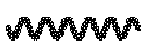 indicates the position of attachment of the partial formula (III) to the linker (L),
G is selected from -C(=O)- and CH₂,
and Ring A is optionally substituted with one or more F,
or a stereoisomer, tautomer, pharmaceutically acceptable salt, solvate or prodrug.

9. The compound of any of claims 1 to 8, wherein the TMPBM is selected from a moiety binding to membrane transport proteins, which are preferably carriers and/or channels, more preferably carriers.

10. The compound of any of claims 1 to 9, wherein the TMPBM is a moiety binding to a solute carrier (SLC) protein,
preferably selected from SLC1 (high affinity glutamate and neutral amino acid transporter), SLC2 (facilitative GLUT transporter), SLC3 (heavy subunits of the heteromeric amino acid transporters), SLC4 (bicarbonate transporter), SLC5 (sodium glucose cotransporter), SLC6 (sodium- and chloride-dependent neurotransmitter transporter), SLC7 (cationic amino acid transporter/glycoprotein-associated amino-acid transporter), SLC8 (Na+/Ca2+ exchanger), SLC9 (Na+/ H+ exchanger), SLC10 (sodium bile salt cotransport), SLC11 (proton coupled metal ion transporter), SLC12 (electroneutral cation-Cl cotransporter), SLC13 (human Na+-sulfate/carboxylate cotransporter), SLC14 (urea transporter), SLC15 (proton oligopeptide cotransporter), SLC16 (monocarboxylate transporter), SLC17 (vesicular glutamate transporter), SLC18 (vesicular amine transporter), SLC19 (folate/thiamine transporter), SLC20 (type III Na+-phosphate cotransporter), SLC21/SLCO (organic anion transporting), SLC22 (organic cation/anion/zwitterion transporter), SLC23 (Na+-dependent ascorbic acid transporter), SLC24 (Na+/(Ca2+-K+) exchanger), SLC25 (mitochondrial carrier), SLC26 (multifunctional anion exchanger), SLC27 (fatty acid transport protein), SLC28 (Na+-coupled nucleoside transport), SLC29 (facilitative nucleoside transporter), SLC30 (zinc efflux), SLC31 (copper transporter), SLC32 (vesicular inhibitory amino acid transporter), SLC33 (Acetyl-CoA transporter), SLC34 (type II Na+-phosphate cotransporter), SLC35 (nucleoside-sugar transporter), SLC36 (proton-coupled amino acid transporter), SLC37 (sugar-phosphate/phosphate exchanger), SLC38 (System A & N, sodium-coupled neutral amino acid transporter), SLC39 (metal ion transporter), SLC40 (basolateral iron transporter), SLC41 (MgtE-like magnesium transporter), SLC42 (Rh ammonium transporter family (pending)), SLC43 (Na+-independent, system-L like amino acid transporter), SLC44 (Choline-like transporter), SLC45 (Putative sugar transporter), SLC46 (Folate transporter), SLC47 (Multidrug and Toxin Extrusion (MATE)), SLC48 (Heme transporter), SLC49 (FLVCR-related transporter), SLC50 (Sugar efflux transporters), SLC51 (Transporters of steroid-derived molecules), and SLC52 (Riboflavin transporter), SLC53 (Phosphate carriers), SLC54 (Mitochondrial pyruvate carriers), SLC 55 (Mitochondrial cation/proton exchangers), SLC56 (Sideroflexins), SLC57 (NiPA-like magnesium transporter family), SLC58 (MagT-like magnesium transporter family), SLC59 (Sodium-dependent lysophosphatidylcholine symporter family), SLC60 (Glucose transporters), SLC61 (Molybdate transporter family), SLC 62 (Pyrophosphate transporters), SLC63 (Sphingosine-phosphate transporters), SLC64 (Golgi Ca2+/H+ exchangers), SLC65 (NPC-type cholesterol transporters) proteins,
more preferably selected from SLC9 (Na+/ H+ exchanger) family proteins,
even more preferably selected from SLC9A subfamily proteins.

11. The compound of any of claims 1 to 10, wherein the TMPBM is a moiety binding to SLC9A1.

12. The compound of any of claims 1 to 11, wherein the TMPBM is a moiety binding to a SLC having between 3 and 17 transmembrane domains, preferably between 3 and 14 transmembrane domains, preferably between 3 and 13 transmembrane domains, preferably between 6 and 14 transmembrane domains, preferably between 8 and 14 transmembrane domains, and even more preferably between 8 and 12 transmembrane domains.

13. A composition comprising a compound of any of claims 1 to 12.

14. The compound of any of claims 1 to 12 or the composition of claim 13 for use as a medicament.

15. The compound of any of claims 1 to 12 or 14, or the composition of claim 13 or 14 for use in treating or preventing cancer, particularly leukemia such as chronic myeloid leukemia.
